# EUROPEAN PATENT APPLICATION

(11) **EP 1 614 351 A1**
(43) Date of publication of application: **11.01.2006**
(21) Application number: 04725508.8
(22) Date of filing: 02.04.2004
(51) Int. Cl.: A01N 31/02, A61K 9/00, A23L 3/349

(54) **SKIN PREPARATION COMPOSITION FOR EXTERNAL USE**

(30) Priority: 04.04.2003 JP 2003102326; 07.04.2003 JP 2003102456; 30.10.2003 JP 2003371240; 30.10.2003 JP 2003371241; 19.12.2003 JP 2003422035; 25.12.2003 JP 2003428532; 03.02.2004 JP 2004026349
(71) Applicant: SHISEIDO COMPANY, LTD., Chuo-ku, Tokyo 104-8010 (JP)
(72) Inventor: KAMINUMA, Mikiko, c/o SHISEIDO RESEARCH CENTER, Yokohama-shi, Kanagawa 224-8558 (JP); SUETSUGU, Masaru, c/o SHISEIDO RESEARCH CENTER, Yokohama-shi, Kanagawa 224-8558 (JP); OGAWA, Shigeyuki, c/o SHISEIDO RESEARCH CENTER, Yokohama-shi, Kanagawa 224-8588 (JP); YOKOTA, Eriko, c/o SHISEIDO RESEARCH CENTER, Yokohama-shi, Kanagawa 224-8558 (JP); HARA, Eijiro, c/o SHISEIDO RESEARCH CENTER, Yokohama-shi, Kanagawa 224-8558 (JP)
(74) Representative: Kurtz, Laurent Charles Edmond
(86) International application number: PCT/JP2004/004826
(87) International publication number: WO 2004/089087

(57) **Abstract**

An external skin treatment composition containing, based upon the total weight of the composition, 0.001% by mass or more of a diol compound of formula (I), (II), (III), or (IV) or the derivative thereof and a balance of a carrier: 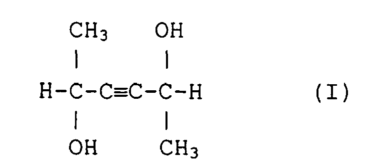 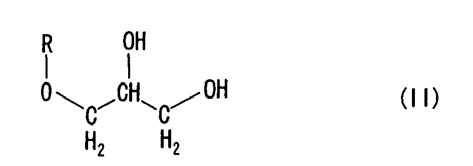 wherein R indicates a benzyl group or a phenyl group.

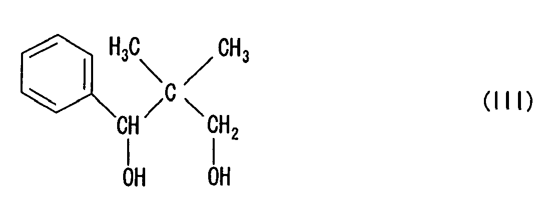

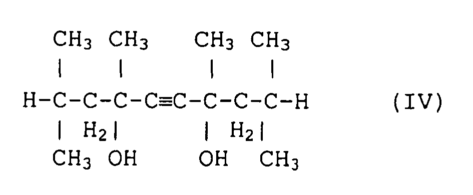

## Description

### TECHNICAL FIELD

The present invention relates to an external skin treatment composition and an antimicrobial agent. More specifically, it relates to an external skin treatment composition and antimicrobial agent capable of being preferably used for external skin treatment compositions, cleansers, food, daily necessities, etc., including a diol compound or its derivative having superior antimicrobial activity to a broad range of microorganisms and safe to the human body, maintaining sufficient preservability, while remarkably improved in usability and safety.

As fields of use, the present invention may be utilized for various types of external skin treatment compositions in the fields of pharmaceuticals, quasi-drugs and cosmetics (including various preparations used for human and other animal use), specifically application to lotion, milk lotions, creams (including ointments), sun screens, foundations, oils, packs, soaps (including medicated soaps), body soaps, lipstick, manicure preparations, eye cosmetics, perfumes, facial cleansers, oral hygiene products (toothpastes, mouthwashes, etc.), deodorants (underarm odor, foot odor, etc.), bath agents, shampoos, rinses, hair tonics, hair sprays, hair dyes, etc. may be mentioned. Further, application to medical use cleansers meant for disinfecting and cleaning medical equipment or diseased parts, household cleansers for sterilizing and washing teblewares, cleansers for the food industry, etc. may be mentioned. Further, use for antimicrobial treatment of textile products (sheets, apparel, etc.), food packaging film, plastic, wood, daily necessities, etc., various types of oral medications, nonwoven fabrics such as sanitary napkins, wet tissue or paper towels, sterilizing cloth, or oral compositions (gum, candies, etc.) or fishpaste products such as Japanese *kamaboko, chikuwa,* or animal products such as sausages, ham, western confectioneries, Japanese confectioneries, noodles such as raw noodles, boiled noodles, Chinese noodles, Japanese *udon* noodles, Japanese *soba* noodles, spaghetti, seasoning such as soysauce, sauces, gravies, foods, juices, soups, and other general foods and beverages can be mentioned.

### BACKGROUND ART

Up to now, as antimicrobial agents and preservatives for food, pharmaceuticals, cosmetics, etc. for suppressing the proliferation of microorganisms, sorbic acid, dehydroacetic acid, and their salts, paraoxybenzoic acid derivatives, etc. have been often used, but these have problems in terms of safety, and therefor the amounts of addition and foods to be added are restricted (for example, the upper limit of use of sorbic acid and its salts in food is 0.2%, dehydroacetic acid and its salts can only be used for cheese, butter and margarine and the upper limit of use of sodium methyl paraoxybenzoate in cosmetics is 1.0%).

Further, to suppress the proliferation of microorganisms, invasion by microorganisms has been suppressed by the physical method of the skin or mucuous membrane in the body or by the chemical method such as the distribution of bactericidal substances or mucous. Further, resistance has been given against microorganisms invading the body through inhibition of adhesion by immunogloblin, the phage action by phagocytes, the lysis action of lysozymes, innate aspecific resistance to infection and immunoresponse to specific pathogenic bacteria, etc.

However, if the infective power of the microorganism becomes higher than the resistance in the organism, there is the risk that the organism will show symptoms of infection, for example, pyoderma and other infectious dermititis due to *Staphylococcus* aureus, food poisoning, blood poisoning, conjunctivitis and iritis due to *Bacillus subtilis,* urinal tract infection, etc. diarrhea due to *E. coli,* periodontitis due to *Actinobacillus* etc., cavities due to cavity-causing bacteria, and further acne, dandruff, ichiness accompanying dandruff, and various other symptoms due to *Propioni bacterium acnes* and *Pityrosporum ovale,* etc.

Therefore, various chemicals have been used up to now for the prevention or treatment of infection internally and externally. The antibiotics or antimicrobial agents and preservatives accounting for the majority of these are indeed strong in effect, but have problems in safety. Serious care is required in their use. More useful substances, in terms of efficacy, safety, etc. have been sought.

In recent years, safe external skin treatment compositions soft on the skin have been sought. On the other hand, external skin treatment compositions have to stress shelf life. Consideration of preservative action is required. Many external skin treatment compositions in fact secure preservability by use of a preservative constituted by a paraoxybenzoic acid ester, also known as "parabens".

However, when using a paraben as a preservative, sometimes some sensitive users will exhibit skin irritations such as stinging at the time of use, and therefore, the usability was also unsatisfactory. Using no paraben to prepare the external skin preparation composition is of course possible, but in that case securing preservability requires the use of small single-use containers, the provision of a backless mechanism in a tube container, or other complicated means. This lacked general usability.

### DISCLOSURE OF THE INVENTION

Accordingly, an object of the present invention is to provide an external skin treatment composition satisfying both usability and safety, while securing sufficient preservability.

Another object of the present invention is to provide an antimicrobial agent having an antimicrobial and preservative effect against infection and diseased parts microorganisms such as due to bacteria, yeast, mold, that is, an effect of killing or preventing proliferation with time and safe even if used in various different fields.

In accordance with the present invention, there is provided an external skin treatment composition comprising, based upon the total weight of the composition, 0.001% by mass or more of a diol compound having the formula (I), (II), (III) or (IV) or the derivatives thereof and a balance of a base component. 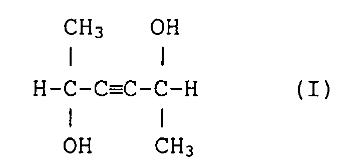 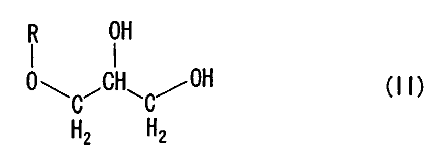
wherein R indicates a benzyl group or a phenyl group.

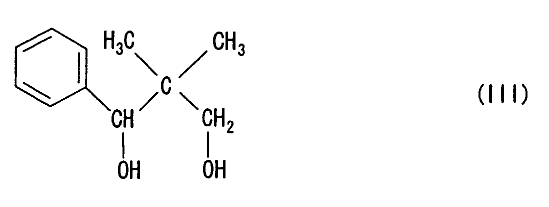

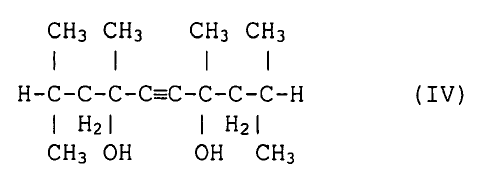

In accordance with the present invention, there is also provided an antimicrobial agent comprising a diol compound having the formula (I), (II), (III) or (IV) or the derivative thereof. 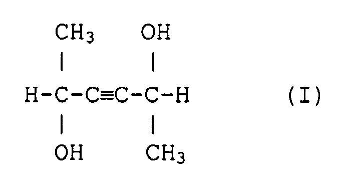 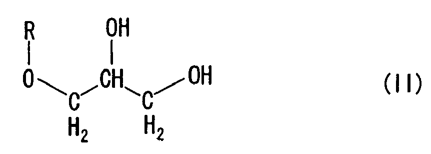
where R indicates a benzyl group or a phenyl group.

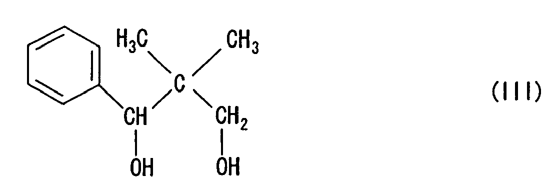

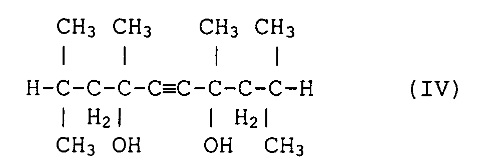

### BEST MODE FOR CARRYING OUT THE INVENTION

The present inventors, in view of the above problems, confirmed that the diol compounds having the formulae (I) to (IV) and their derivatives have strong antimicrobial and preservative effects to various types of microorganisms and are safe for human or animal use, and found that applying them as novel antimicrobial agents in various fields was extremely effective, whereby the present invention has been completed.

The inventors further engaged in intensive research on substances having a antimicrobial and preservative effect against infections and diseases arising due to microorganisms such as bacteria, yeast, mold, that is, an effect of killing bacteria or prevention of proliferation with the elapse of time and safe even if utilized in various fields and, as a result, confirmed that diol compounds having the formulae (I) to (IV) and their derivatives have strong antimicrobial and preservative effect against various types of microorganisms and are safe for human and animal use, found that use as novel antimicrobial agents in various fields is extremely effective, whereby the present invention has been completed.

The constitution of the present invention will now be explained in detail.

The 3-hexine-2,5-diol having the formula (1) used in the present invention is a known substance, but the use thereof as an antimicrobial agent is novel.

The 3-hexine-2,5-diol used in the present invention can be easily produced from acetylene and acetoaldehyde. Further, for example, it is commercially available from Tokyo Kasei Kogyo, Kanto Kasei Kogyo, Aldrich, etc. and can be easily available. The 3-hexine-2,5-diol used in the present invention may also be an optical isomer, racemie, mesomer or mixtures thereof.

The glycerin derivative having the formula (II) used in the present invention is a known substance, but the use thereof as a microbial agent is novel. This glycerin derivative can be produced by a general synthetic method. For example, it can be easily produced from glycidol, glycerin, chlorohydrin or bromohydrin or another halohydrin and phenol or benzyl alcohol. Further, it may also be obtained by hydrolysis of a phenyl glycidyl ether or benzyl glycidyl ether. 3-phenoxy-1,2-propanediol is commercially available from, for example, Tokyo Kasei Kogyo, while 3-benzyloxy-1,2-propanediol is commercially available from, for example, Sigma etc., so these can be easily available. The glycerin derivative used in the present invention may be an optical isomer, racemie or a mixture thereof. Further, the 3-phenoxy-1,2-propanediol and 3-benzyloxy-1,2-propanediol may be used alone, but may also be used in any combination ratio.

The 2,2-dimethyl-1-phenyl-1,3-propanediol having the formula (III) used in the present invention is a known substance, but the use thereof as an antimicrobial agent is novel. This 2,2-dimethyl-1-phenyl-1,3-propanediol can be produced by a general synthesis method. For example, it can be easily produced from benzaldehyde and isobutyl aldehyde. (e.g., it can be obtained by the method of Wolter ten Hoeve et al. (*J. Org. Chem.* 1985, 50, 4508-4514)). Further, 2,2-dimethyl-1-phenyl-1,3-propanediol is, for example, commercially available from Aldrich etc. and can be easily available. The 2,2-dimethyl-1-phenyl-1,3-propanediol used in the present invention may be any of an optical isomer, racemie or a mixture thereof.

The 2,4,7,9-tetramethyl-5-decine-4,7-diol having the formula (IV) used in the present invention is a known substance, but the use thereof as an antimicrobial agent is novel.

The 2,4,7,9-tetramethyl-5-decine-4,7-diol used in the present invention can be produced by a general synthesis method. For example, it can be synthesized from acetylene and 4-methyl-2-pentanone. Further, 2,4,7,9-tetramethyl-5-decine-4,7-diol is, for example, commercially available from Tokyo Kasei, etc. and can be easily obtained. The 2,4,7,9-tetramethyl-5-decine-4,7-diol used in the present invention may be any of an optical isomer, racemie or any mixture thereof.

The antimicrobial agent of the present invention may be formulated into any external skin treatment composition. Further, the antimicrobial agent of the present invention can be introduced as an anti-microbial agent for a medical use cleanser meant for disinfecting and cleaning medical equipment or diseased parts, a household cleanser for sterilizing and washing eating utensils, a cleanser for the food industry, food packaging film, plastic, wood, daily necessities, etc., an antimicrobial paint, and an antimicrobial agent for an oral composition (gum, candy, etc.) or fishpaste products such as Japanese *kamaboko, chikuwa,* animal products such as sausages, ham and confectioneries, noodles, seasoning such as sauces, soysauce, food dishes, beverages, and other food.

The method of using the antimicrobial agent of the present invention is not particularly limited and can be applied to various objects. For example, the method of adding it to an object, the method of spraying it, the method of coating it, the method of dipping in it, the method of impregnating it, the method of mixing it in at the time of shaping and other generally employed methods may be used as they are.

The antimicrobial agent of the present invention can be utilized as it is, but when used as an antimicrobial agent, the incorporation amount differs according to the type of product and the degree of action expected. It is not particularly limited, but usually is at least 100 ppm, preferably 1000 ppm or more, more preferably 1000 to 5000 ppm.

Further, when formulating the antimicrobial agent of the present invention into various compositions, it may be used, together with, to an extent not impairing the effect of the present invention, various ingredients generally used for food, cosmetics, pharmaceuticals, quasi-drugs, etc., for example, sugar, condensed milk, flour, shortening, table salt, glucose, eggs, butter, margarine, malt syrup, calcium, iron, seasoning, spices, or oils (animal and vegetable oils, mineral oils, ester oils, wax oils, silicone oils, higher alcohols, phospholipids, fatty acids, etc.), surfactants (anionic, cationic, amphoteric or non-ionic surfactants), vitamins (the vitamin A group, the vitamin B group, folic acid, nicotinic acid, pantothenic acid, biotin, the vitamin C group, the vitamin D group, the vitamin E group, other ferulic acids, γ-orizanole, etc.), UV absorbants (paraminobenzoic acid, anthranile, salicylic acid, coumarine, benzotriazole, tetrazole, imidazoline, pyrimidine, dioxane, furane, pyrane, camphor, nucleic acid, allantoin, and their derivatives, amino acid-based compounds, shikonin, baicalin, baicalein, berberine, etc.), antioxidants (stearic acid esters, nordihydroguairetic acid, dibutylhydroxytoluene, butylhydroxyanisole, parahydroxyanisole, propyl gallate, sesamol, sesamolin, gossypol, etc.), thickeners (hydroxyethylcellulose, ethylcellulose, carboxyethylcellulose, methylcellulose, carboxymethylcellulose, sodium carboxymethylcellulose, hydroxypropylcellulose, nitrocellulose, polyvinyl alcohol, polyvinylmethyl ether, polyvinylpyrrolidone, polyvinyl methacrylate, polyacrylates, carboxyvinyl polymers, gum arabic, tragacanth gum, agar, casein, dextrin, gelatin, pectin, starch, alginic acid, and the salts thereof, etc.), humectants (propyleneglycol, 1,3-butyleneglycol, polyethyleneglycol, glycerin, 1,2-pentanediol, hexyleneglycol, chondroitin sulfuric acid and the salts thereof, hyaluronic acid and the salts thereof, sodium lactate, etc.), and lower alcohols, polyhydric alcohols, water-soluble polymers, pH adjusters, preservative and anti-mold agents, coloring agents, fragrances, refrigerants, stabilizers, animal and plant extracts, animal and plant proteins and their decomposed products, animal and plant polysaccharides and their decomposed products, animal and plant glycoproteins and their decomposed products, microorganism-cultured metabolized ingredients, blood circulation promoting agents, antipologistics, antiinflammatories, antiallergenics, cell activants, amino acids and their salts, peeling agents, astringents, wound treatment agents, foam boosters, oral preparations, deodorants, emulsifiers, etc.

Further, the antimicrobial agent of the present invention may be formulated into external skin treatment composition in any form. It is formulated by ordinary methods. For example, lotion, creams, ointments, milk lotions, foundations, oils, packs, soaps (including medicated soaps), body soaps, lipstick, manicure preparations, eye cosmetics, perfumes, facial cleansers, oral hygiene products (toothpastes, mouthwashes, etc.), deodorants (underarm odor, foot odor, etc.), bath agents, shampoos, rinses, hair tonics, hair sprays, hair dyes, etc. may be mentioned.

Further, the antimicrobial agent of the present invention may be formulated into other compositions in any form. They are blended by ordinary methods. For example, oral medications, textile products (sheets, apparel, etc.), nonwoven fabrics such as sanitary napkins, wet tissue, paper towels, sterilizing cloth, or oral compositions (gum, candies, etc.) or fishpaste products such as Japanese *kamaboko, chikuwa,* animal products such as sausages, ham, and western confectioneries, Japanese confectioneries, raw noodles, boiled noodles, noodles such as Chinese noodles, Japanese *udon* noodles, Japanese *soba* noodles, spaghetti, seasoning such as soysauce, sauces, gravies, foods, juices, soups, and other general foods and beverages can be mentioned.

Further, the antimicrobial agent of the present invention may be formulated in various compositions in any form. Solutions, creams, pastes, gels, jellies, foam, solids or powders can be used.

The antimicrobial agent comprised of the diol compound having the formulae (I) to (IV) of the present invention or the derivatives thereof may be formulated into any external skin treatment composition. The formulation amount differs depending on the form of the product and is not particularly limited, but is considering the spoilability of an ordinary external skin preparation composition, at least 0.001% by mass is necessary, preferably at least 0.1% by mass. More preferably, with incorporation in an amount of at least 1.0% by mass, the remarkable effect of the present invention is manifested. The upper limit of incorporation is particularly difficult to define due to the properties of the external skin preparation composition, but if formulated in excess, stickiness or other deterioration of usage characteristics occur, so incorporation of not more than 10.0% by mass is preferable.

As the substrate of the external skin preparation composition containing the antimicrobial agent having the formulae (I) to (IV) of the present invention, any base components of ordinary external skin treatment compositions may be used. That is, a liquid, gel, paste, milk lotion, cream, etc. may be used. Further, ingredients usually formulated into external skin treatment compositions, for example, powder ingredients, liquid oils and fats, solid oils and fats, waxes, hydrocarbon oils, higher fatty acids, higher alcohols, ester oils, silicone oils, anionic surfactants, cationic surfactants, amphoteric surfactants, non-ionic surfactants, humectants, water soluble polymers, thickeners, film forming agents, UV absorbants, sequestering agents, lower alcohols, polyhydric alcohols, sugars, amino acids, organic amines, polymer emulsions, pH adjusters, skin nourishing creams, vitamins, antioxidants, antioxidation aids, other medicaments, dyes, fragrances, water, etc. may be used as well.

Further, it is possible to increase the antimicrobial action by formulating a general dihydric alcohol etc.

The glycerin derivative having the formula (II) of the present invention is both hydro- and lyo-philic from the structure thereof and is easily dissolved in or formulated well with water, an alcohol, oil, etc. Further, there is the advantage of good feeling in use.

The ingredients capable of formulating into the external skin treatment composition containing the antimicrobial agent of the present invention will be explained in detail below. It is possible to formulate one or two or more of the following ingredients to prepare the external skin treatment composition.

As a powder ingredient, for example, an inorganic powder (e.g., talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, a tungstic acid metal salt, magnesium, silica, zeolite, barium sulfate, calcined calcium sulfate (calcined gypsum), calcium phosphate, fluorine apatite, hydroxyapatite, ceramic powder, metal soaps (e.g., zinc myristate, calcium palmitate, aluminum stearate), boronitride, etc.); organic powder (e.g., polyamide resin powder (nylon powder), polyethylene powder, polymethyl methacrylate powder, benzoguanamine resin powder, polyethylenetetrafluoride powder, cellulose powder, etc.); inorganic white pigments (e.g., titanium dioxide, zinc oxide, etc.); inorganic red pigments (e.g., iron oxide (bengara), iron titanate, etc.); inorganic brown pigments (e.g., γ-iron oxide etc.); inorganic yellow pigments (e.g., yellow iron oxide, loess, etc.); inorganic black pigments (e.g., black iron oxide, lower titanium oxide, etc.); inorganic violet pigments (e.g., Mango Violet, Cobalt Violet, etc.); inorganic green pigments (for example, chromium oxide, chromium hydroxide, cobalt titanate, etc.); inorganic blue pigments (e.g., ultramarine blue, Prussian blue, etc.); pearl pigments (e.g., titanium oxide coated mica, titanium oxide coated bismuth oxychloride, titanium oxide coated talc, colored titanium oxide-coated mica, bismuth oxychloride, fish scales, etc.); metal powder pigments (e.g., aluminum powder, copper powder, etc.); zirconium, barium, or aluminum lakes and other organic pigments (e.g., Red 201, Red 202, Red 204, Red 205, Red 220, Red 226, Red 228, Red 405, Orange 203, Orange 204, Yellow 205, Yellow 401, and Green 404 and other organic pigments, Red 3, Red 104, Red 106, Red 227, Red 230, Red 401, Red 505, Orange 205, Yellow 4, Yellow 5, Yellow 202, Yellow 203, Green 3, Green 1, etc.); natural dyes (e.g., chlorophyll, β-carotene, etc.) etc. may be mentioned.

As liquid fats and oils, avocado oil, tsubaki oil, macademia nut oil, corn oil, olive oil, rapeseed oil, sesame oil, wheat germ oil, sasanqua oil, castor oil, linseed oil, safflower oil, cotton seed oil, soybean oil, peanut oil, tea seed oil, rice bran oil, jojoba oil, germ oil, triglycerin, etc. may be mentioned.

As solid fats and oils, for example, cacao butter, coconut oil, hydrogenated coconut oil, palm oil, palm kernel oil, Japanese core wax nucleus oil, hydrogenated oil, Japan wax, hydrogenated castor oil, etc. may be mentioned.

As a wax, for example, beeswax, candelilla wax, carnauba wax, lanolin, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin ethyl alcohol ether, etc. may be mentioned.

As the hydrocarbon oil, for example, liquid paraffin, ozokerite, squalane, pristane, paraffin, ceresine, squalene, vaseline, microcrystalline wax, Fisher-Trope wax, etc. may be mentioned.

As a higher fatty acid, for example, lauric acid, myristic acid, palmitic acid, stearic acid, behenic acid, oleic acid, undecyleic acid, isostearic acid, linolic acid, linoleic acid, eicosapentanoic acid (EPA), docosahexanoic acid (DHA), etc. may be mentioned.

As a higher alcohol, for example, linear alcohol (e.g., lauryl alcohol, cetyl alcohol, stearyl alcohol, behenyl alcohol, myristyl alcohol, oleyl alcohol, cetostearyl alcohol, etc.); branched chain alcohol (e.g., monostearyl glycerin ether (batyl alcohol), 2-decyltetradecinol, lanolin alcohol, cholesterol, phitosterol, hexyldodecanol, isostearyl alcohol, octyl dodecanol, etc.), etc. may be mentioned.

As the ester oil, isopropyl myristate, cetyl octanate, octyldodecyl myristate, isopropyl palmitate, butyl stearate, hexyl laurate, myristyl myristate, decyl oleinate, hexyldecyldimethyl octanate, cetyl lactate, myristyl lactate, lanolin acetate, isocetyl stearate, isocetyl isostearate, 12-hydroxystearate cholestearyl, ethylene glycol di-2-ethylhexanate, dipentaerythritol fatty acid ester, monoisostearate N-alkyl glycol, neopentyl glycol dicaprylate, diisostearyl malate, glyceryl di-2-heptylundecanate, trimethylolpropane tri-2-ethylhexanate, trimethylolpropane triisostearate, pentaerythritol tetra-2-ethylhexanate, glyceryl tri-2-ethylhexanate, glyceryl trioctanate, glyceryl triisopalmitate, trimethylolpropane triisostearate, cetyl 2-ethyl hexanoate, 2-ethylhexyl palmitate, glyceryl trimyristate, glyceride tri-2-heptylundecanate, castor oil fatty acid methyl ester, oleyl oleanate, acetoglyceride, palmitate 2-heptylundecyl, isobutyl adipate, N-lauroyl-L-glutamate-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, etc. may be mentioned.

As a silicone oil, for example, a straight chain polysiloxane (e.g., dimethylpolysiloxane, methylphenylpolysiloxane, diphenylpolysiloxane, etc.); a cyclic polysiloxane (e.g., octamethyl cyclotetrasiloxane, decamethyl cyclopentasiloxane, dodecamethyl cyclohexasiloxane, etc.), methyl trimeticon, silicone resin forming a 3D net structure, silicone rubber, various modified polysiloxanes (aminomodified polysiloxane, polyether modified polysiloxane, alkyl modified polysiloxane, fluorine modified polysiloxane, etc.), etc. may be mentioned.

As an anionic surfactant, for example, a fatty acid soap (e.g., sodium laurate, sodium palmitate, etc.); a higher alkyl sulfuric acid ester salt (e.g., sodium laurilsulfate, potassium laurilsulfate, etc.); an alkyl ether sulfuric acid ester salt (e.g., triethanolamine POE-laurilsulfate, sodium POE-laurilsulfate, etc.); N-acylsarcosinic acid (e.g., sodium lauroylsarcocinate etc.); a higher fatty acid amide sulfonate (e.g., sodium N-myristoyl-N-methyltaurin, sodium cocoyl methyltaurin, sodium cocyl methyltaurin taurin, sodium laurylmethyltaurin, sodium laurylmethyltaurinmethyltaurin, etc.); a sulfosuccinate (for example, sodium di-2-ethylhexylsulfosuccinate, sodium monolauroylmonoethanolamide polyoxyethylene sulfosuccinate, sodium laurylpolypropyleneglycol sulfosuccinate, etc.); an alkyl benzene sulfonate (e.g., sodium linear dodecylbenzene sulfonate, linear dodecylbenzene sulfonate triethanolamine, linear dodecylbenzene sulfonic acid, etc.); a higher fatty acid ester sulfuric acid ester salt (e.g., sodium hydrogenated glyceryl cocoate sulfate, etc.); N-acyl glutamate (e.g., monosodium N-lauroyl glutamate, disodium N-stearoyl glutamate, monosodium N-myristoyl-L-glutamate, etc.); POE-alkyl ether carboxylic acid; POE-alkyl allyl ether carboxylate; α-olefin sulfonate, sodium lauroyl monoethanolamide succinate; ditriethanolamine N-palmitoyl aspartate, etc. may be mentioned.

As a cationic surfactant, for example, an alkyl trimethyl ammonium salt (e.g., stearyl trimethyl ammonium salt, lauryl trimethyl ammonium chloride, etc.); alkyl pyridinium salt (e.g., cetylpyridinium chloride etc.); distearyldimethyl ammonium chloride dialkyl dimethyl ammonium salt; poly(N,N'-dimethyl-3,5-methylenepiperidinium)chloride; alkyl tetraammonium salt; alkyl dimethylbenzyl ammonium salt; alkyl isoquinolinium salt; dialkyl morphonium salt; benzalkonium chloride; benzetonium chloride. etc. may be mentioned.

As an amphoteric surfactant, for example, imidazoline-based amphoteric surfactant (e.g., sodium 2-undecyl-N,N,N-(hydroxyethylcarboxymethyl)-2-imidazoline, 2-cocyl-2-imidazolinium hydroxide-1-carboxyethyloxyl 2 sodium salt, etc.); betaine-based surfactant (e.g., 2-heptadecyl-N-carboxymethyl-N-hydroxyethylimidazolinium betaine, amide amine type amphoteric surfactants, lauryldimethylaminoacetic acid betaine, alkyl betaine, amide betaine, sulfobetaine, etc.) etc. may be mentioned.

As a lyophilic anionic surfactant, for example, a sorbitan fatty acid ester (e.g., sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, diglycerol sorbitan tetra-2-ethylhexylate, etc.); a glyceryl polyglyceryl fatty acid (e.g., glyceryl monocotton seed oil fatty acid, glyceryl monoerucate, glyceryl sesquioleate, glyceryl monostearate, glyceryl α,α'-oleate pyroglutamate, glyceryl monostearate malate, etc.); a propyleneglycol fatty acid ester (e.g., monostearate propyleneglycol etc.); a hydrogenated castor oil derivative; a glycerinalkyl ether may be mentioned.

As a hydrophilic anionic surfactant, for example, a POE-sorbitan fatty acid ester (e.g., POE-sorbitan monooleate, POE-sorbitan monostearate, POE-sorbitan monooleate, POE-sorbitan tetraoleate etc.); a POE sorbitol fatty acid ester (e.g., POE-sorbitol monolaurate, POE-sorbitol monooleate, POE-sorbitol pentaoleate, POE-sorbitol monostearate, etc.); a POE-glycerin fatty acid ester (e.g., POE-glycerin monostearate, POE-glycerin monoisostearate, POE-glycerin triisostearate, and other POE-monooleates); a POE-fatty acid ester (e.g., POE-distearate, POE-monodioleate, distearate ethylene glycol, etc.); a POE-alkyl ether (e.g., POE-lauryl ether, POE-oleyl ether, POE-stearyl ether, POE-behenyl ether, POE-2-octyldodecyl ether, POE-cholestanol ether, etc.);a pluaronic (e.g., pluaronic); a POE-POP-alkyl ether (e.g., POE-POP-cetyl ether, POE-POP-2-decyltetradecyl ether, POE-POP-monobutyl ether, POE-POP-hydrated lanolin, POE-POP-glycerin ether); a tetraPOE-tetraPOP-ethylene diamine condensate (e.g., tetraonic etc.); POE-castor oil, hydrogenated castor oil derivative (e.g., POE-castor oil, POE-hydrogenated castor oil, POE-hydrogenated castor oil monoisostearate, POE-hydrogenated castor oil triisostearate, POE-hydrogenated castor oil monopyroglutamic acid monoisostearate diester, POE-hydrogenated castor oil maleic acid etc.); alkanolamide (e.g., cocyl diethanolamide, monoethanolamide laurate, fatty acid isopropanol amide, etc.); sucrose fatty acid ester; alkyl ethoxydimethylamine oxide; trioleyl phosphoric acid, etc. may be mentioned.

As the humectant, for example, polyethylene glycol, propylene glycol, glycerin, 1,3-butylene glycol, xylitol, sorbitol, maltitol, chondroitin sulfuric acid, hyaluronic acid, acetylated hyaluronic acid, mucoitin sulfuric acid or other mucosaccharide and their salts, cholestearyl-12-hydroxystearate, lactic acid, sodium lactate, dl-pyrrolidone carboxylate, trimethylglycine, diglycerin (EO) PO adducts, chestnut rose extract, yarrow extract, sweet clover extract, etc. may be mentioned.

As a natural water-soluble polymer, for example, a plant polymer (e.g., gum arabic, gum tragacanth, galactan, guar gum, carrageenan, pectin, agar, locust bean gum, quince seed (*Cydonia oblonga*), algae colloids (brown algae extract), starches (rice, corn, potato, and wheat), microorganism-type polymers (e.g., xanthan gum, dextran, succinoglucan, gellan gum, and pullulan); etc., may be mentioned.

As a semisynthetic water-soluble polymer, for example, a starch-type polymer (e.g., carboxymethyl starch and methylhydroxypropyl starch); a cellulosic polymer (e.g., methyl cellulose, ethyl cellulose, methylhydroxypropyl cellulose, hydroxyethyl cellulose, cellulose sodium sulfate, hydroxypropyl cellulose, carboxymetyl-cellulose, sodium carboxymethyl cellulose, crystal cellulose, and cellulose powder); and an alginic acid-type polymer (e.g., sodium alginate and propyleneglycol alginate, etc.) may be mentioned.

As a synthetic water-soluble polymer, for example, a vinyl polymer (e.g., polyvinyl alcohol, polyvinylmethyl ether, polyvinyl pyrrolidone, carboxyvinyl polymer, alkyl modified carboxyvinyl polymer, etc.); polyoxyethylene-based polymer (e.g., polyethylene glycol 20,000, 40,000, 60,000 polyoxyethylene polyoxypropylene copolymer, etc.); acryl-based polymer (e.g., sodium polyacrylate, polyethyl acrylate, polyacryl amide, etc.); polyethylene imine; cationic polymer, etc. may be mentioned.

As a thickener, in addition to the above water-soluble polymers, for example, aluminum magnesium silicate, bentonite, organic modified bentonite, hectorite, AlMg silicate (veegum), laponite, anhydrous silicate, etc. may be mentioned.

As a UV absorbant, for example, a benzoic acid-based UV absorbant (e.g., p-aminobenzoic acid (hereinafter referred to as "PABA"), PABA monoglycerinester, N,N-dipropoxyl PABA ethyl ester, N,N-diethoxy PABA ethyl ester, N,N-dimethyl PABA ethyl ester, N,N-dimethyl PABA butyl ester, N,N-dimethyl PABA ethyl ester, etc.); salicylic acid-based UV absorbants (e.g., amyl salicylate, mentyl salicylate, homomentyl salicylate, octyl salicylate, phenyl salicylate, benzyl salicylate, p-isopropanol phenyl salicylate, etc.); cinnamic acid-based UV absorbants (e.g., octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate(2-ethylhexyl-p-methoxycinnamate), 2-ethoxyethyl-p-methoxycinnamate, cyclohexyl-p-methoxycinnamate, glyceryl mono-2-ethylhexanoyl-diparamethoxy cinnamate, etc.); 3-(4'-methylbenzylidene)-d,l-camphor, 3-benzylidene-d,1-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzaladine; dianisoylmethane; 4-methoxy-4'-t-butyldibenzoylmethane; 5-(3,3-dimethyl-2-norbonylidene)-3-pentan-2-one; bis-ethylhexyloxyphenol-methoxyphenyl-triazine; 2,4,6-tris[4-(2-ethylhexyloxycarbonyl)anilino]1,3,5-triazine may be mentioned.

As a metal ion sequestering agent, for example, 1-hydroxyethane-1,1-diphosphonic acid, 1-hydroxyethane-1,1-disphosponic acid tetrasodium salt, disodium edetate, trisodium edetate, tetrasodium edetate, sodium hexametaphosphate, sodium citrate, sodium polyphosphate, sodium metaphosphate, gluconic acid, phosphoric acid, citric acid, ascorbic acid, succinic acid, edetic acid, trisodium ethylenediaminehydroxyethyl triacetate, etc. may be mentioned.

As a lower alcohol, for example, ethanol, isopropanol, isobutyl alcohol, t-butyl alcohol, etc. may be mentioned.

As a polyhydric alcohol, for example, dihydric alcohols (e.g., ethylene glycol, propylene glycol, trimethylene glycol, 1,2-butylene glycol, 1,3-butylene glycol, tetramethylene glycol, 2,3-butyleneglycol, pentamethylene glycol, 2-butene-1,4-diol, hexylene glycol, octylene glycol, etc.); trivalent alcohol (for example, glycerin, trimethylolpropane, etc.); tetravalent alcohol (e.g., 1,2,6-hexanetriol or other pentaerythritol etc.); pentavalent alcohol (e.g., xylitol etc.); hexavalent alcohol (e.g., sorbitol, mannitol, etc.); polyhydric alcohol polymers (e.g., diethylene glycol, dipropyleneglycol, triethylene glycol, polypropyleneglycol, tetraethylene glycol, diglycerin, polyethylene glycol, triglycerin, tetraglycerin, polyglycerin, etc.); bivalent alcohol alkyl ethers (e.g., ethylene glycolmonomethyl ether, ethylene glycolmonoethyl ether, ethylene glycolmonobutyl ether, ethylene glycolmonophenyl ether, ethylene glycolmonohexyl ether, ethylene glycolmono2-methylhexyl ether, ethylene glycolisoamyl ether, ethylene glycolbenzyl ether, ethylene glycolisopropyl ether, ethylene glycoldimethyl ether, ethylene glycoldiethyl ether, ethylene glycoldibutyl ether, etc.); dihydric alcohol alkyl ethers (e.g., diethylene glycol monomethyl ether, diethylene glycol monoethyl ether, diethylene glycol monobutyl ether, diethylene glycol dimethyl ether, diethylene glycoldiethyl ether, diethylene glycolbutyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc.); dihydric alcohol ether ester (e.g., ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc.); glycerin monoalkyl ether (e.g., ichthammol, selachyl alcohol, batyl alcohol, etc.); sugar alcohols (e.g., sorbitol, maltitol, maltotriose, mannitol, sucrose, erythritol, glucose, fructose, starch amylolysis sugar, maltose, xylitose, alcohol prepared by the reduction of starch amylolysis sugar, etc.); glysolid; tetrahydrofurfuryl alcohol; POE-tetrahydrofurfuryl alcohol; POP-butyl ether; POP-POE-butyl ether; tripolyoxypropyleneglycerin ether; POP-glycerin ether; POP-glycerin ether phosphate; POP-POE-pentane erithritol ether, polyglycerin, etc. may be mentioned.

As a monosaccharide, for example, a triose (e.g., D-glyceryl aldehyde, dihydroxyacetone, etc.); a tetrose (e.g., D-erythrose, D-erythrulose, D-threose, erythritol, etc.); a pentose (e.g., L-arabinose, D-xylose, L-xylose, D-arabinose, D-ribose, D-ribulose, D-xylulose, L-xylulose, etc.); a hexose (e.g., D-glucose, D-talose, D-psicose, D-galactose, D-fructose, L-galactose, L-mannose, D-tagatose, etc.); a heptose (e.g., aldoheptose, heprose, etc.); an octose (e.g., octurose etc.); deoxysugar (for example, 2-deoxy-D-ribose, 6-deoxy-L-galactose, 6-deoxy-L-mannose, etc.); amino sugar (e.g., D-glucosamine, D-galactosamine, sialic acid, amino uronic acid, muramic acid, etc.); a uronic acid (e.g., D-glucuronic acid, D-mannuronic acid, L-guluronic acid, D-galacturonicacid, L-iduronic acid, etc.) may be mentioned.

As an oligosaccharide, for example, sucrose, gentianose, unbelliferose, lactose, planteose, isolignoses, α,α-trehalose, raffinose, etc. may be mentioned.

As an amino acid, for example, a neutral amino acid (e.g., threonine, cysteine, etc.); a basic amino acid (e.g., hydroxylysine etc.) etc. may be mentioned. Further, as an amino acid derivative, for example, trimethylglycine, glutathione, pyrrolidone carboxylic acid, etc. may be mentioned.

As an organic amine, for example, monoethanolamine, diethanolamine, triethanolamine, morpholine, triisopropanol amine, 2-amino-2-methyl-1,3-propanediol, 2-amino-2-methyl-1-propanol, etc. may be mentioned.

As a polymer emulsion, for example, an acryl resin emulsion, ethyl polyacrylate emulsion, acryl resin solution, polyacryl alkyl ester emulsion, polyvinyl acetate resin emulsion, natural rubber latex, etc. may be mentioned.

As a pH adjuster, for example, lactic acid-sodium lactate, citric acid-sodium citrate, succinic acid-sodium succinate, or another buffer etc. may be mentioned.

As a vitamin, for example, vitamin A, B1, B2, B6, C, E, and their derivatives, pantothenic acid and its derivatives, biotin, etc. may be mentioned.

As an antioxidant, for example, tocopherols, dibutyl hydroxytoluene, butyl hydroxyanisole, gallic acid esters, etc. may be mentioned.

As an antioxidation aid, for example, phosphoric acid, citric acid, ascorbic acid, maleic acid, malonic acid, succinic acid, fumaric acid, cephalin, hexametaphosphite, phytic acid, ethylene diamine tetraacetic acid, etc. may be mentioned.

As other usable ingredients, for example, antiinflammatory agents (e.g., glycyrrhizic acid derivatives, glycyrrhetinic derivatives, salicyclic acid derivatives, hinokitiol, zinc oxide, allatoin, etc.); whitening agents (e.g., placenta extract, creeping saxifrage extract, albutin, tranexamic acid, potassium 4-methoxysalicylate, etc.; various kinds of extracts (for example, phellodendron bark, goldthread, lithosperum root, Paeonia lactiflora, Swertia japonica, birch, sage, loquat, ginseng, aloe, Malva sylvestris, iris, grape, coix, sponge gourd, lily, saffron, Cnidium offinale, ginger, Hypericum erectum, Ononis, garlic, Guinea pepper, citrus unshium peel, Ligusticum acutilobum, seawee, etc.), activators (e.g., royal jelly, photosensitive substances, cholesterol derivatives, etc.); blood circulation promoting agents (e.g., nonylic acid vanillylamide, nicotinic acid benzyl esters, nicotinic acid β-butoxyethylesters, capsaicin, zingerone, cantharis tincture, ichthammol, tannic acid, α-borneol, tocopherol nicotinate, inositol hexanicotinate, clandelate, cinnarizine, tolazoline, acetylcholine, verapamil, cepharanthine, γ-orizanol, etc.); anti-seborrhea agents (e.g., sulfur, thiantol, etc.); antiinflammatory agents (e.g., thiotaurine, hypotaurine, etc.); and stabilizers (4,5-dimorpholino-3-hydroxypyridazine), etc. may be mentioned.

### EXAMPLES

The present invention will now be explained in further detail by the following Examples. The present invention is not limited to these Examples. The formulation amounts are based upon % by mass.

### Example I-1: Antimicrobial Effect

The minimum inhibiting concentrations (MIC) for various types of microbials were found.

Using the agar plate method, for bacteria, the following various bacteria were inoculated in SCD agar media (made by Eiken) containing 3-hexine-2,5-diol in different concentrations and cultured at 30°C for 24 hours. The concentrations of 3-hexine-2,5-diol not forming colonies (minimum inhibiting concentration: MIC) were found. Further, for fungi, the following various bacteria were inoculated in a potato dextrose agar media containing 3-hexine-2,5-diol in different concentrations and cultured at 25°C for 48 hours. The concentrations of 3-hexine-2,5-diol not forming colonies (minimum inhibiting concentration: MIC) were found. The same was conducted for methyl paraoxybenzoate methyl. The results of the judgment are shown in Table 1 based on the following evaluation criteria:

### (Test Bacteria)

- Ps:: *Pseudomonas aeuginosa* (ATCC1542)
- E:: *Escherichia coli* (ATCC8739)
- S:: *Staphylococcus aureus* (ATCC6538)
- Can:: *Candida albicans* (ATCC10231)
- Asp:: *Aspergillus niger* (ATCC16404)

### (Evaluation Criteria)

A: Minimum inhibiting concentration of less than 1000 ppm
B: Minimum inhibiting concentration of 1000 ppm to less than 5000 ppm
C: Minimum inhibiting concentration of 5000 ppm to less than 10000 ppm
D: Minimum inhibiting concentration of 10000 ppm to less than 30000 ppm
E: Minimum inhibiting concentration or 30000 ppm or more

**Table I-1**

| Test bacteria | Antimicrobial effect | |
|---|---|---|
| | 3-hexine-2,5-diol | Methyl paraoxybenzoate |
| *Pseudomonas aeuginosa* (ATCC15442) | A | C |
| *Escherichia coli* (ATCC8739) | A | B |
| *Staphylococcus* aureus (ATCC6538) | A | B |
| *Candida albicans* (ATCC10231) | A | B |
| *Aspergillus niger* (ATCC16404) | A | B |

### Example I-2: Safety Test

The 3-hexine-2,5-diol of the present invention was tested for safety. A single administration toxicity test was conducted, as a result, of which the toxicity was judged to be extremely weak. Further, a primary skin irritation test and a continuous skin irritation test were conducted, as a result, of which the skin irritability was judged to be extremely weak. Further, a skin sensitization test and a genetic toxicity test were conducted, as a result, of which negative results were obtained.

As explained above, the safety of the 3-hexine-2,5-diol of the present invention was good.

The methods for utilizing the present invention will now be described in detail, but the present invention is by no means limited to the following Examples. The present invention is able to be formulated in, for example, various drugs, quasi-drugs, external skin treatment compositions such as cosmetics, detergents, foods, daily products. The following Examples were produced according to a conventional manner for the production of each product and the formulation amounts are shown below,

### Example I-3: Lotion

| | mass% |
|---|---|
| Ethanol | 5.0 |
| 1,3-Butylene glycol | 6.0 |
| Glycerol | 4.0 |
| Oleyl alcohol | 0.1 |
| POE (20) Sorbitan monolaurate | 0.5 |
| POE (15) Lauryl ether | 0.5 |
| 3-Hexyne-2,5-diol | 1.0 |
| Fragrance | q.s. |
| Purified water | balance |

### Example I-4: Emulsion

| | mass% |
|---|---|
| 3-Hexyne-2,5-diol | 3.0 |
| Glycerol | 3.0 |
| Cetanol | 1.5 |
| Stearyl alcohol | 1.8 |
| Dimethylpolysiloxane (20 cs) | 1.5 |
| Squalane | 2.0 |
| Vaseline | 2.0 |
| Isopropyl myristate | 2.5 |
| Glyceryl monostearate | 1.8 |
| Polyoxyethylene (POE = 5) glyceryl monostearate | 1.8 |
| Polyoxyethylene (POE = 20) Cetyl ether | 1.5 |
| Carboxyvinyl polymer | 0.25 |
| Potassium hydroxide | 0.05 |
| L-Arginine | 0.2 |
| Dipropylene glycol | 5.0 |
| 1,3-Butylene glycol | 3.0 |
| Trisodium edetate | 0.2 |
| Methyl paraben | 0.01 |
| Purified water | balance |

### Example I-5: Cream

| | mass% |
|---|---|
| 3-Hexyne-2,5-diol | 10.0 |
| Stearyl alcohol | 3.5 |
| Stearic acid | 2.0 |
| Squalane | 10.5 |
| Isopropyl myristate | 7.5 |
| Polyoxyethylene (POE = 25) Cetyl alcohol ether | 3.0 |
| Glycerol monostearate | 2.0 |
| Tocopherol acetate | 0.2 |
| Monoammonium glycyrrhizinate | 0.05 |
| Glycerol | 3.0 |
| Dipropylene glycol | 5.0 |
| 1,3-Butylene glycol | 3.0 |
| Phenoxyethanol | 0.2 |
| Trisodium edetate | 0.01 |
| Ethyl paraben | 0.1 |
| Purified water | balance |

### Example I-6: Cleansing

| | mass% |
|---|---|
| 3-Hexyne-2,5-diol | 0.5 |
| Stearic acid | 8.0 |
| Palmitic acid | 6.0 |
| Myristic acid | 6.0 |
| Lauric acid | 4.0 |
| Potassium hydroxide | 5.2 |
| Glyceryl monostearate | 2.0 |
| Propylene glycol | 1.0 |
| Bees wax | 1.5 |
| Polyethylene glycol 1500 | 5.0 |
| Glycerol | 10.0 |
| Purified water | balance |

### Example I-7: Shampoo

| | mass% |
|---|---|
| Lauryl polyoxyethylene (3) sulfate ester Sodium salt (30% aqueous solution) | 25.0 |
| Sodium lauryl sulfate ester (30% aqueous solution) | 8.0 |
| Coconut oil fatty acid diethanolamide | 4.0 |
| Isoprene glycol | 4.0 |
| Dipropylene glycol | 1.0 |
| 1,3-Butylene glycol | 1.0 |
| Trisodium edetate | 0.01 |
| 3-Hexyne-2,5-diol | 0.1 |
| Dye | q.s. |
| Fragrance | q.s. |
| Purified water | balance |

### Example I-8: Gelly pack

| | mass% |
|---|---|
| 3-Hexyne-2,5-diol | 0.1 |
| Polyoxyethylene oleyl alcohol ether | 0.5 |
| Monoammonium glycyrrhizinate | 0.05 |
| Carboxymethyl cellulose | 5.0 |
| Ethanol | 12.0 |
| Polyvinyl alcohol | 12.0 |
| 1,3-Butylene glycol | 5.0 |
| Trisodium edetate | 0.01 |
| Purified water | balance |

### Example I-9: Eyeliner

| | mass% |
|---|---|
| 3-Hexyne-2,5-diol | 3.0 |
| Iron oxide (black) | 14.0 |
| Isopropyl myristate | 1.5 |
| Polyoxyethylene sorbitan monooleic ester | 1.0 |
| Vinyl acetate resin emulsion | 45.0 |
| Monoammonium glycyrrhizinate | 0.05 |
| Carboxyvinyl polymer | 1.5 |
| Acetyltributyl citrate | 1.0 |
| Dipropylene glycol | 5.0 |
| 1,2-Pentance diol | 3.0 |
| Trisodium edetate | 0.01 |
| Purified water | balance |

### Example I-10: Hair tonic

| | mass% |
|---|---|
| Hydrogenated castor oil ethyleneoxide (40 mol) addition product | 2.0 |
| Ethanol | 60.0 |
| Fragrance | q.s. |
| 3-Hexyne-2,5-diol | 0.01 |
| Purified water | balance |

### Example I-11: Bath agent

| | mass% |
|---|---|
| Sodium bicarbonate | 60.0 |
| Anhydrous sodium sulfate | 35.0 |
| 3-Hexyne-2,5-diol | 5.0 |

### Example I-12: Chinese noodle

| | mass% |
|---|---|
| Wheat flour | 98.0 |
| Table salt | 1.0 |
| Sweetner | 0.5 |
| 3-Hexyne-2,5-diol | 0.5 |

### Example I-13: Noodle soup

| | mass% |
|---|---|
| Soysauce | 80.0 |
| Vinegar | 1.0 |
| Glucose | 15.0 |
| Sodium glutamate | 2.0 |
| Sugar | 1.0 |
| 3-Hexyne-2,5-diol | 1.0 |

### Example I-14: Japanese noodle ("Soba")

| | mass% |
|---|---|
| Soba flour | 96.0 |
| Table salt | 0.9 |
| Water | 3.0 |
| 3-Hexyne-2,5-diol | 0.1 |

### Example I-15: Bread

| | mass% |
|---|---|
| Wheat flour | 90.0 |
| Table salt | 1.2 |
| Sugar | 2.0 |
| Water | 6.0 |
| 3-Hexyne-2,5-diol | 0.8 |

### Example I-16: Ham

| | mass% |
|---|---|
| Minced meat | 95.0 |
| Chicken egg | 4.0 |
| Table salt | 0.5 |
| Spice | 0.4 |
| 3-Hexyne-2,5-diol | 0.1 |

### Example I-17: Fruitjuice beverage

| | mass% |
|---|---|
| Glucose liquid sugar | 13.0 |
| Orange fruit juice | 85.0 |
| Fragrance | 1.0 |
| 3-Hexyne-2,5-diol | 1.0 |

### Usability Test and Preservability Test

The lotion having the formulation shown in Table II-1 was prepared according to the following preparation method and subjected to the usability and preservability (antisepsis) tests.

### Preparation of Lotion

In the purified water, 3-hexyne-2,5-diol, citric acid, trisodium citrate, trisodium edetate are dissolved (i.e., an aqueous phase). Ethanol, glycerol, 1,3-butylene glycol, POE (60) hydrogenated castor oil, methyl paraben are dissolved (i.e., an alcohol phase). The aqueous phase and the alcohol phase were mixed.

### Test Method for Usability

A panel consisting of 10 women having a sensitive skin, who had an irritation feeling when an external skin treatment composition containing paraben was used. The test was carried out in such a manner that each paneller used the above external skin treatment composition twice a day for 1 week and, based upon the satisfactory degree and the presence or absence of the skin irritation of each paneller, the usability is evaluated based upon the following 4-rank standards. The number of the persons having irritation feeling was also counted.
A: Number of paneller having good feeling is 8 or more
B: Number of paneller having good feeling is 5 to less than 8
C: Number of paneller having good feeling is 3 to less than 5
D: Number of paneller having good feeling is less than 3

### Test Method for Judging Preservability

To 30 ml of the samples of the Examples and Comparative Examples, microbials in a liquid were inoculated and then the change in the number of the microbial was checked after 2 weeks from the inoculation by a smear culture method. The inoculated microbials were as follows.
Mould: Aspergillus niger ATCC16404. Inoculation amount 10⁴ cfu (colony forming unit)/g
Yeast: Candida albicans ATCC10231. Inoculation amount 10⁵ cfu/g
Bacteria: Escherichia coli ATCC8739. Inoculation amount 10⁶ cfu/g; Staphylococcus aureus ATCC6538. Inoculation amount 10⁶ cfu/g; Pseudomonas aeruginosa ATCC15442. Inoculation amount 10⁶ cfu/g

The preservability (or antisepsis) was evaluated used upon the changes in the number of the microbials and the results were classified to the fallowing 4 ranks.
A: All of the mould, yeast and bacteria are decreased to 100 cfu/g or less within 1 week.
B: All of the mould, yeast and bacteria are decreased to 100 cfu/g or less within 2 weeks.
C: Either of the mould, yeast or bacteria remain in an amount of 100 cfu/g or more even after 2 weeks.
D: All of the mould, yeast and bacteria remain in an amount of 100 cfu/g or more even after 2 weeks.

As the preservability of an external skin treatment composition, those judged as "A" or "B" is acceptable. The results of the usability and preservability tests are shown in Table II-1.

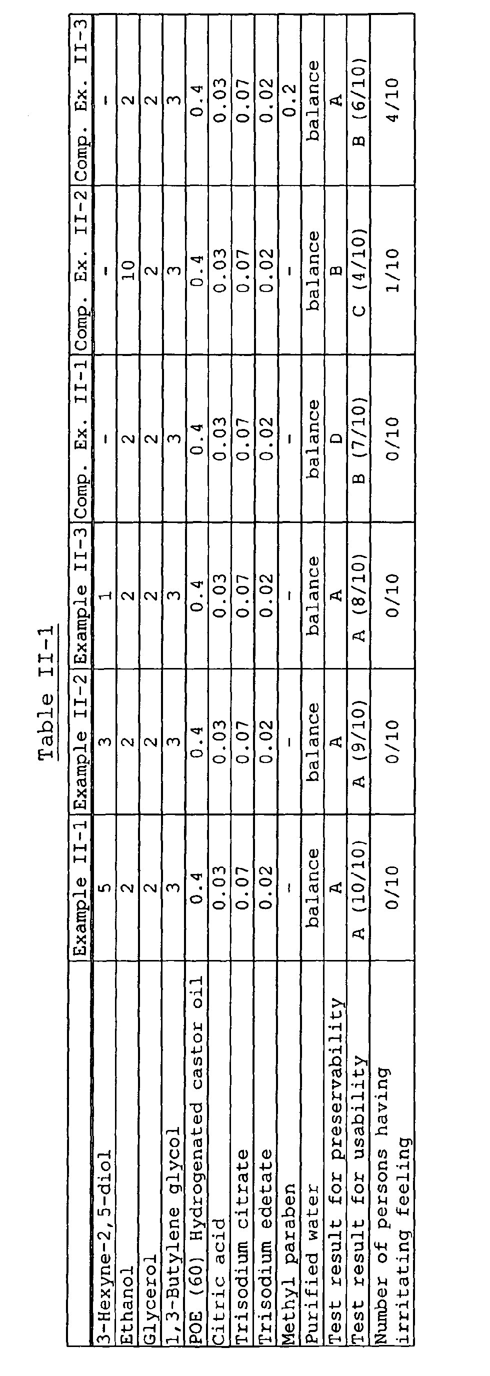

There were no persons having irritating feeling for Examples II-1, II-2 and II-3 and the ratio of the persons having satisfactory usability are large and thus the preservability was confirmed. This is the effect of the present invention. Also in Comparative Example II-1, a large number of the panellers are satisfactory in the usability and the skin irritation is small, but the preservability is poor. In Comparative Example II-2, there are no problems in the preservability, the satisfaction in the usability is low although the preservability is not problem, one paneller felt a skin irritation. Comparative Example II-3 was problem in the skin irritation because many panellers had skin irritation although the preservability is excellent.

Next, the emulsion having the formulation shown in Table II-2 was prepared according to the following preparation method and the usability and preservability tests were carried out in the same manner as mentioned above. The results of the usability and preservability tests are shown in Table II-2.

### Preparation of Emulsion

In the purified water, 3-hexyne-2,5-diol, 1,3-butylene glycol, polyethylene glycol 1500, trisodium edetate, triethanol amine were added, followed by heating at 70°C to prepare an aqueous phase. Stearic acid, cetyl alcohol, vaseline, squalane were dissolved and sorbitan monooleic acid ester and methyl paraben were added thereto, followed by heating at 70°C to prepare an oil phase. The oil phase was added to the aqueous phase to the pre-emulsified and then uniformly emulsified in a homomixer followed by cooling.

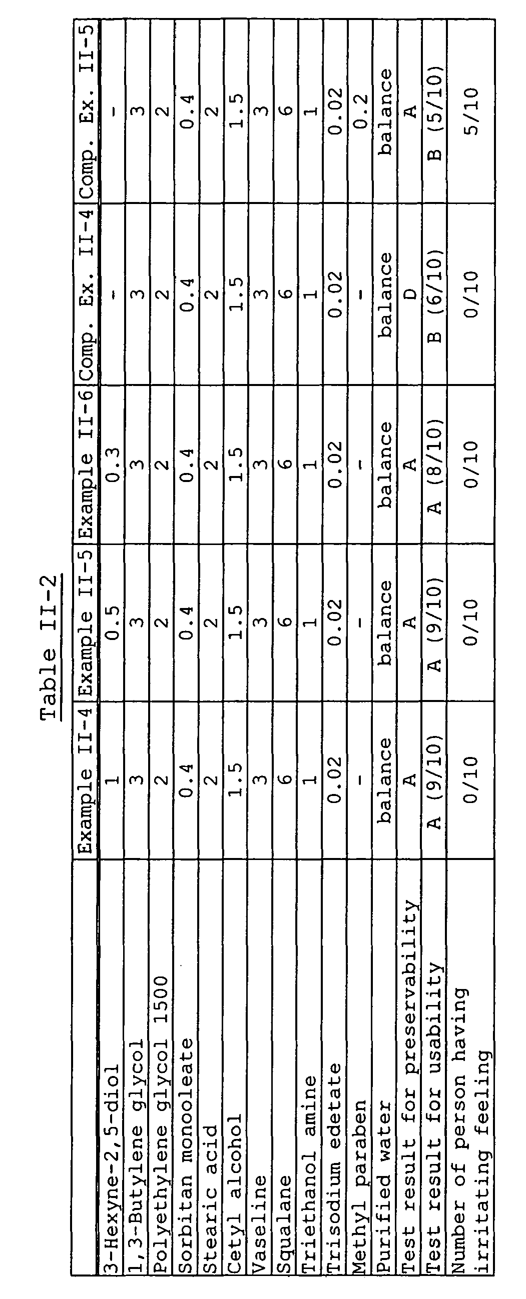

There were no persons having irritating feeling for Examples II-4, II-5 and II-6 and the ratio of the persons having satisfactory usability are large and thus the preservability was confirmed. This is the effect of the present invention. Also in Comparative Example II-4, a large number of the panellers are satisfactory in the usability and the skin irritation is small, but the preservability is poor. Comparative Example II-5 was problem in the skin irritation because many panellers had skin irritation although the usability and preservability are excellent.

Next, the emulsion having the formulation shown in Table II-3 was prepared according to the following preparation method and the usability and preservability tests were carried out in the same manner as mentioned above. The results of the usability and preservability tests are shown in Table II-3.

### Preparation of Cream

In the purified water, 3-hexyne-2,5-diol, 1,3-butylene glycol, propylene glycol were added, followed by heating at 70°C to prepare an aqueous phase. Stearyl alcohol, stearyl alcohol, hydrogenated lanolin, squalane, octyl dodecanol were dissolved and POE (25) cetyl alcohol ether, glycerol monostearate and methyl paraben were added thereto, followed by heating at 70°C to prepare an oil phase. The oil phase was added to the aqueous phase and uniformly emulsified in a homomixer followed by cooling.

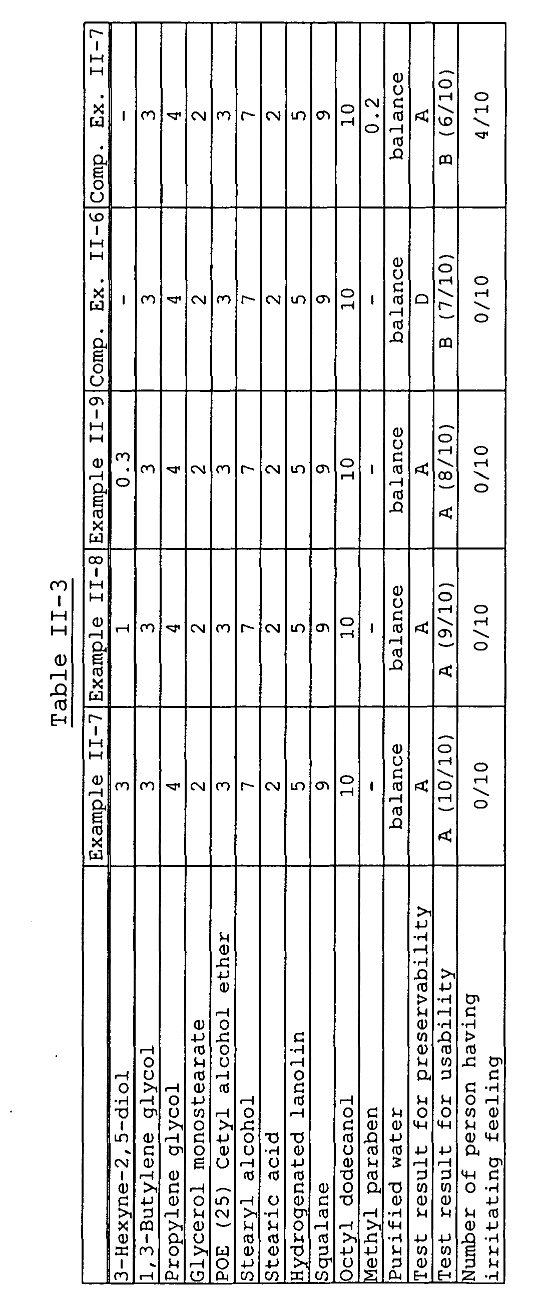

There were no persons having irritating feeling for Examples II-7, II-8 and II-9 and the ratio of the persons having satisfactory usability are large and thus the preservability was confirmed. This is the effect of the present invention. Also in Comparative Example II-6, a large number of the panellers are satisfactory in the usability and the skin irritation is small, but the preservability is poor. Comparative Example II-7 was problem in the skin irritation because many panellers had skin irritation although the usability and preservability are excellent.

Various external skin treatment composition will now be explained and all Examples have no skin irritation and good usability, while maintaining the preservability.

### Example II-10: Lotion

| (Alcohol phase) | mass% |
|---|---|
| Ethanol | 5.0 |
| Oleyl alcohol | 0.2 |
| POE (20) Sorbitan monolaurate | 0.5 |
| POE (15) Lauryl ether | 0.5 |
| 4,5-Dimorpholino-3-hydroxypyridazine | 0.1 |
| Phenoxyethanol | 0.3 |
| Fragrance | q.s. |

| (Aqueous phase) | mass% |
|---|---|
| 1,3-Butylene glycol | 6.0 |
| 1,2-Pentanediol | 2.0 |
| 3-Hexyne-2,5-diol | 0.5 |
| Glycerol | 5.0 |
| Purified water | balance |

### (Preparation method)

The aqueous phase and the alcohol phase were mixed after the preparation of the aqueous phase and the alcohol phase.

### Example II-11: Lotion

| (Alcohol phase) | mass% |
|---|---|
| Ethanol | 5.0 |
| POE (20) Oleyl ether | 0.5 |
| 3-Hexyne-2,5-diol | 0.1 |
| Fragrance | q.s. |

| (Aqueous phase) | mass% |
|---|---|
| Dipropylene glycol | 6.0 |
| Sorbitol | 4.0 |
| PEG1500 | 5.0 |
| Methyl cellulose | 0.2 |
| Quince Seed | 0.1 |
| Purified water | balance |

### (Preparation method)

To a portion of the purified water, methyl cellulose and Quince Seed were mixed, followed by stirring to prepare a viscous liquid. The remainder of the purified water and the other components of the aqueous phase were mixed and dissolved, and the above viscous liquid were added to obtain a uniform aqueous phase. The alcohol phase was prepared and then added to the aqueous phase, followed by mixing.

### Example II-12: Cream

| | mass% |
|---|---|
| Stearic acid | 5.0 |
| Stearyl alcohol | 4.0 |
| Isopropyl myristate | 18.0 |
| Glycerol monostearate | 3.0 |
| Propylene glycol | 10.0 |
| 1,2-Hexane diol | 3.0 |
| 3-Hexyne-2,5-diol | 3.0 |
| Potassium hydroxide | 0.2 |
| Phenoxyethanol | 0.3 |
| Sodium bisulfite | 0.01 |
| Fragrance | q.s. |
| Purified water | balance |

### (Preparation method)

In the purified water, propylene glycol and potassium hydroxide were added to be dissolved, followed by heating and maintained at 70°C (i.e., an aqueous phase). The other components were mixed, followed by heating and melting and maintained at 70°C (i.e., an oil phase). The oil phase was gradually added to the aqueous phase to be pre-emulsified and uniformly emulsified in a homomixer, followed by cooling to 30°C, with mixing.

### Example II-13: Cream

| | mass% |
|---|---|
| Stearic acid | 6.0 |
| Sorbitan monostearate | 2.0 |
| POE (20) Sorbitan monostearate | 1.5 |
| Propylene glycol | 10.0 |
| 3-Hexyne-2,5-diol | 10.0 |
| Glycerol trioctanoate | 10.0 |
| Squalane | 5.0 |
| Sodium bisulfite | 0.01 |
| Fragrance | q.s. |
| Purified water | balance |

### (Preparation method)

In the purified water, propylene glycol was added to be dissolved, followed by heating and maintained at 70°C (i.e., an aqueous phase). The other components were mixed, followed by heating and melting and maintained at 70°C (i.e., an oil phase). The oil phase was gradually added to the aqueous phase to be pre-emulsified and uniformly emulsified in a homomixer, followed by cooling to 30°C, with mixing.

### Example II-14: Emulsion

| | mass% |
|---|---|
| Stearic acid | 2.5 |
| Cetyl alcohol | 1.5 |
| Vaseline | 5.0 |
| Liquid paraffin | 10.0 |
| POE (10) Monooleate | 2.0 |
| PEG1500 | 3.0 |
| Triethanolamine | 1.0 |
| 3-Hexyne-2,5-diol | 5.0 |
| Sodium bisulfite | 0.01 |
| Carboxyvinyl polymer | 0.05 |
| Fragrance | q.s. |
| Purified water | balance |

### (Preparation method)

In a small amount of the purified water, carboxyvinyl polymer was dissolved (i.e., phase A). To the remainder of the purified water, PEG 1500 and triethanolamine were added and dissolved upon heating at 70°C (i.e., an aqueous phase). The other components were mixed, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example II-15: Gel

| | mass% |
|---|---|
| 95% Ethanol | 5.0 |
| Dipropylene glycol | 15.0 |
| 1,2-Octane diol | 3.0 |
| POE (50) Oleyl ether | 2.0 |
| Carboxyvinyl polymer | 1.0 |
| Sodium hydroxide | 0.15 |
| 3-Hexyne-2,5-diol | 0.1 |
| Fragrance | q.s. |
| Purified water | balance |

### (Preparation method)

To the purified water, the carboxyvinyl polymer was uniformly dissolved (i.e., phase A). The POE (50) oleyl ether was dissolved in the 95% ethanol and then added to the phase A. After the components other than the sodium hydroxide was added, the sodium hydroxide was added thereto to be neutralized and thickened.

### Example II-16: Beauty liquid

| | mass% |
|---|---|
| 95% Ethanol | 5.0 |
| POE (20) Octyldodecanol | 1.0 |
| Pantonyl ethyl ether | 0.1 |
| Potassium hydroxide | 0.1 |
| Glycerol | 5.0 |
| Dipropylene glycol | 10.0 |
| Sodium bisulfite | 0.03 |
| Carboxyvinyl polymer | 0.2 |
| 3-Hexyne-2,5-diol | 0.3 |
| Phenoxyethanol | 0.3 |
| Purified water | balance |

### (Preparation method)

In a portion of the purified water, the carboxyvinyl polymer was dissolved (i.e., phase A). Similarly, a portion of the purified water, the potassium hydroxide was dissolved (i.e., phase B). In the remainder of the purified water, the water-soluble components were dissolved (i.e., phase C). To the ethanol, POE (20) octyl dodecanol and the pantotenyl ethyl ether were dissolved and then the above phase C was added thereto and mixed with stirring, then the above phase A was mixed therewith with mixing and thereafter the above phase B was added thereto and mixed with stirring in a homomixer.

### Example II-17: Pack

| (A phase) | mass% |
|---|---|
| Dipropylene glycol | 5.0 |
| POE (60) Hydrogenated castor oil | 5.0 |

| (B phase) | mass% |
|---|---|
| Olive oil | 5.0 |
| Tocopherol acetate | 0.2 |
| Fragrance | 0.2 |

| (C phase) | mass% |
|---|---|
| Sodium bisulfite | 0.03 |
| Polyvinyl alcohol (Degree of saponification 90, Degree of polymerization 2000) | 13.0 |
| Ethanol | 5.0 |
| 3-Hexyne-2,5-diol | 0.5 |
| Purified water | balance |

### (Preparation method)

The phase A, phase B and phase C were uniformly mixed respectively and then the phase B was added to the phase A to be solubilized. Thereafter, the phase C was added thereto, followed by mixing.

### Example II-18: Solid powdery foundation

| | mass% |
|---|---|
| (1) Talc | 15.0 |
| (2) Cericite | 10.0 |
| (3) Spherical nylon powder | 10.0 |
| (4) Porous anhydrous silicic acid powder | 15.0 |
| (5) Boron nitride | 5.0 |
| (6) Titanium dioxide | 5.0 |
| (7) Iron oxide | 3.0 |
| (8) Zinc stearate | 5.0 |
| (9) Liquid paraffin | balance |
| (10) Glycerol triisooctanoate | 15.0 |
| (11) Sorbitan sesquioleate | 1.5 |
| (12) 3-Hexyne-2,5-diol | 1.0 |
| (13) Fragrance | q.s. |

### (Preparation method)

The components (1)-(8) were mixed and ground and a mixture of the components (9)-(13) was added thereto, followed by mixing with stirring, and the resultant mixture was molded in a container to obtain a solid foundation.

### Example II-19: Oil-in-water type emulsified foundation

| | mass% |
|---|---|
| (1) Spherical nylon | 10.0 |
| (2) Porous anhydrous silicic acid powder | 8.0 |
| (3) Mica titanium | 2.0 |
| (4) Silicone-treated cericite | 2.0 |
| (5) Silicone-treated mica | 12.0 |
| (6) Silicone-treated titanium dioxide | 5.0 |
| (7) Silicone-treated iron oxide | 2.0 |
| (8) Purified water | balance |
| (9) 3-Hexyne-2,5-diol | 3.0 |
| (10) Decamethylcyclopentane siloxane | 18.0 |
| (11) Dimethyl polysiloxane | 5.0 |
| (12) Squalane | 1.0 |
| (13) Polyoxyethylene modified dimethylpolysiloxane | 2.0 |
| (14) Fragrance | q.s. |

### (Preparation method)

The components (9)-(14) were uniformly mixed and dissolved and, then, the mixed and ground components (1)-(7) were added and dispersed therein. To the dispersion thus obtained, the component (8) was added and emulsified, followed by filling in a container to obtain the oil-in-water type emulsified foundation.

### Example II-20: Face powder

| | mass% |
|---|---|
| (1) Talc | balance |
| (2) Cericite | 10.0 |
| (3) Spherical nylon powder | 10.0 |
| (4) Boron nitride | 5.0 |
| (5) Iron oxide | 3.0 |
| (6) Magnesium carbonate | 5.0 |
| (7) Squalane | 3.0 |
| (8) Glycerol triisooctanoate | 2.0 |
| (9) Sorbitan sesquioleate | 2.0 |
| (10) 3-Hexyne-2,5-diol | 3.0 |
| (11) Fragrance | q.s. |

### (Preparation method)

The components (1)-(6) were mixed and ground and the previously mixed components (7)-(11) were added thereto, followed by mixing with stirring, to obtain the face powder.

### Example II-21: Eye shadow

| | mass% |
|---|---|
| (1) Talc | balance |
| (2) Mica | 15.0 |
| (3) Spherical nylon powder | 10.0 |
| (4) Boron nitride | 5.0 |
| (5) Iron oxide | 3.0 |
| (6) Titanium oxide-coated mica | 5.0 |
| (7) Squalane | 3.0 |
| (8) Glycerol triisooctanoate | 2.0 |
| (9) Sorbitan sesquioleate | 2.0 |
| (10) 3-Hexyne-2,5-diol | 0.5 |
| (11) Fragrance | q.s. |

### (Preparation method)

The components (1)-(6) were mixed and ground and the components (7)-(11) were added thereto, followed by mixing with stirring, to obtain the eyeshadow.

### Example II-22: Lipstick

| | mass% |
|---|---|
| (1) Carnauba wax | 0.5 |
| (2) Candelilla wax | 5.0 |
| (3) Ceresine | 10.0 |
| (4) Squalane | balance |
| (5) Glycerol triisostearate | 10.0 |
| (6) Glycerol diisostearate | 20.0 |
| (7) 3-Hexyne-2,5-diol | 0.2 |
| (8) Cholesteryl Macademia nut oil fatty acid | 4.0 |

| | |
|---|---|
| (9) Synthetic sodium-magnesium silicate | 0.5 |
| (10) Hydrophobic silica | 0.5 |
| (11) Purified water | 2.0 |
| (12) Coloring agent | q.s. |
| (13) Fragrance | q.s. |

### (Preparation method)

In the component (8) heated at 60°C, the components (9) and (10) were dispersed, and the component (11) was added thereto, followed by sufficiently stirring. The resultant mixture was added to a mixture of the components (1)-(7), which were separately dissolved by heating at 70°C, followed by sufficiently mixing, and then, the components (12) and (13) were added thereto, followed by dispersing with stirring. Thereafter, the dispersion was flown into a container, followed by cooling and molding to obtain the lipstick.

### Example II-23: Hair foam

| (Stock solution formulation) | mass% |
|---|---|
| (1) Acrylic resin alkanol amine solution (50%) | 8.0 |
| (2) Polyoxyethylene hydrogenated castor oil | 1.0 |
| (3) Liquid paraffin | 5.0 |
| (4) Glycerol | 3.0 |
| (5) Fragrance | q.s. |
| (6) 3-Hexyne-2,5-diol | 0.01 |
| (7) Ethanol | 5.0 |
| (8) Purified water | balance |

| (Filling formulation) | mass% |
|---|---|
| (1) Stock solution | 90.0 |
| (2) Liquefied petroleum gas | 10.0 |

### (Preparation method)

The liquid paraffin was added to a dissolved mixture of the glycerol and the polyoxyethylene hydrogenated castor oil and uniformly emulsified in a homomixer. The emulsified product was added to a solution of the other components. The stock solution was filled in a can and, after a valve is attached, a gas is filled.

### Example II-24: Shampoo

| | mass% |
|---|---|
| Sodium lauryl polyoxyethylene (3) sulfate ester (30% aqueous solution) | 30.0 |
| Sodium lauryl sulfate ester (30% aqueous solution) | 10.0 |
| Coconut oil fatty acid diethanol amide | 4.0 |
| Glycerol | 1.0 |
| 3-Hexyne-2,5-diol | 3.0 |
| Sodium benzoate | 0.5 |
| Dye | q.s. |
| Fragrance | q.s. |
| Metal sequestering agent | q.s. |
| Purified water | balance |

### (Preparation method)

The purified water was heated to 70°C and the other components were added thereto, followed by uniformly dissolving and then cooling.

### Example II-25: Rinse

| | mass% |
|---|---|
| Silicone oil | 3.0 |
| Liquid paraffin | 1.0 |
| Cetyl alcohol | 1.5 |
| Stearyl alcohol | 1.0 |
| Stearyl trimethylammonium chloride | 0.7 |
| 3-Hexyne-2,5-diol | 5.0 |
| Glycerol | 3.0 |
| Dye | q.s. |
| Fragrance | q.s. |
| Purified water | balance |

### (Preparation method)

To the purified water, the stearyl trimethylammonium chloride, glycerol and dye were added and maintained at 70°C (i.e., an aqueous phase). The other components were mixed, followed by heating and dissolving and maintained at 70°C (i.e., an oil phase). The oil phase was added to the aqueous phase and emulsified in a homomixer, followed by cooling, with mixing.

### Example II-26: Cream

| | mass% |
|---|---|
| Liquid paraffin | 10.0 |
| Dimethyl polysiloxane | 2.0 |
| Glycerol | 10.0 |
| 1,3-Butylene glycol | 2.0 |
| Erythritol | 1.0 |
| Polyethylene glycol 1500 | 5.0 |
| Squalane | 15.0 |
| Tetra 2-ethyl hexanoic acid pentaerythritol | 5.0 |
| Potassium hydroxide | 0.1 |
| Sodium hexametaphosphate | 0.05 |
| Tocopherol acetate | 0.05 |
| 3-Hexyne-2,5-diol | 3.0 |
| Hydroxydipropyl methylcellulose | 0.3 |
| Polyvinyl alcohol | 0.1 |
| Carboxydivinyl polymer | 0.2 |
| Acrylic acid·methacrylic acid alkyl copolymer (Pemulene TR-2) | 0.1 |
| Purified water | balance |

### (Preparation method)

In a small amount of the purified water, carboxyvinyl polymer and acrylic acid alkyl methacrylate copolymer were dissolved (i.e., phase A). To the remainder of the purified water, PEG 1500 and triethanolamine were added and dissolved upon heating at 70°C (i.e., an aqueous phase). The other components were mixed, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example II-27: Cream

| | mass% |
|---|---|
| Vaseline | 2.0 |
| Dimethylpolysiloxane (6 mPa.s) | 2.0 |
| Ethanol | 5.0 |
| Behenyl alcohol | 0.5 |
| Batyl alcohol | 0.2 |
| Glycerol | 7.0 |
| 1,3-Butylene glycol | 5.0 |
| Polyethylene glycol 20000 | 0.5 |
| Jojoba oil | 3.0 |
| Squalane | 2.0 |
| Phytosteryl hydroxystearate | 0.5 |
| Pentaerythitol Tetra 2-ethyl hexanoate | 1.0 |
| Polyoxyethylene (60) hydrogenated castor oil | 1.0 |
| Potassium hydroxide | 0.1 |
| Sodium pyrosulfite | 0.01 |
| Sodium hexametaphosphate | 0.05 |
| Stearyl glycyrrhetiate | 0.1 |
| Pantothenyl ethyl ether | 0.1 |
| Albutin | 7.0 |
| Tranexamic acid | 1.0 |
| Tocopherol acetate | 0.1 |
| Sodium hyaluronate | 0.05 |
| 3-Hexyne-2,5-diol | 3.0 |
| Trisodium edetate | 0.05 |
| 4-t-Butyl-4'-methoxydibenzoyl methane | 0.1 |
| Diparamethoxycinnamic acid mono-2-ethylhexane glyceryl | 0.1 |
| Yellow iron oxide | q.s. |
| Xanthan gum | 0.1 |
| Carboxyvinyl polymer | 0.2 |
| Purified water | balance |

### (Preparation method)

In a small amount of the purified water, carboxyvinyl polymer and xanthan gum were dissolved to form a solution (i.e., phase A). In the remainder of the purified water, the water-soluble components were dissolved upon heating at 70°C (i.e., an aqueous phase). In the liquid oil, the oily components were mixed followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example II-28: Cream

| | mass% |
|---|---|
| Decamethylcyclopenta siloxane | 30.0 |
| Polyoxyethylene·methylpolysiloxane copolymer (M.W. 6000) | 1.5 |
| Trimethylsiloxy silicic acid | 0.5 |
| Glycerol | 2.0 |
| Dipropylene glycol | 5.0 |
| 3-Hexyne-2,5-diol | 2.0 |
| Talc | 5.0 |
| Spherical anhydrous silicic acid | 0.5 |
| Dextrin palmitate-coaded titanium oxide fine powder (30 nm) | 7.0 |
| Spheric polyethylene powder | 2.0 |
| Poly(oxyethylene·oxypropylene)·methyl polysiloxane copolymer (M.W. 55000) | 1.0 |
| Phenoxyethanol | 0.2 |
| Trisodium edetate | 0.02 |
| Dimethyl distearyl ammonium hectolite | 0.5 |
| Purified water | balance |

### (Preparation method)

After dissolving the oily components upon heating, the polyoxyethylene·methyl polysiloxane copolymer, poly(oxyethylene·oxypropylene)·methyl polysiloxane copolymer, dimethyl distearyl ammonium hectrite and the other oily components were added thereto. The temperature of the mixture was adjusted to 70°C and uniformly dispersed and dissolved to obtain an oily gel. To the purified water, the glycerol, dipropylene glycol and 3-hexyne-2,5-diol were added and the temperature was adjusted to 70°C. The resultant mixture was gradually added to the oily gel with stirring and, after uniformly mixing in a homomixer, the mixture was cooled to 30°C.

### Example II-29: Emulsion

| | mass% |
|---|---|
| Liquid paraffin | 7.0 |
| Vaseline | 3.0 |
| Decamethylcyclopentane siloxane | 2.0 |
| Behenyl alcohol | 1.0 |
| Glycerol | 5.0 |
| Dipropylene glycol | 7.0 |
| Polyethylene glycol 1500 | 2.0 |
| 3-Hexyne-2,5-diol | 2.0 |
| Jojoba oil | 1.0 |
| Isostearic acid | 0.5 |
| Stearic acid | 0.5 |
| Behenic acid | 0.5 |
| Tetra 2-ethyl hexanoic acid pentaerythitol | 3.0 |
| Cetyl 2-ethylhexanoate | 3.0 |
| Glyceryl monostearate | 1.0 |
| Polyoxyethylene glyceryl monostearate | 1.0 |
| Potassium hydroxide | 0.1 |
| Sodium hexametaphosphate | 0.05 |
| Stearyl glycyrrhetinate | 0.05 |
| L-Arginine | 0.1 |
| Royal gelly extract | 0.1 |
| Tocopherol acetate | 0.1 |
| Sodium acetylated hyaluronate | 0.1 |
| Trisodium edetate | 0.05 |
| 4-t-Butyl-4'-methoxydibenzoyl methane | 0.1 |
| P-methoxy cinnamic acid 2-ethylhexyl | 0.1 |
| Carboxyvinyl polymer | 0.15 |
| Purified water | balance |

### (Preparation method)

In a small amount of the purified water, carboxyvinyl polymer was dissolved (i.e., phase A). To the remainder of the purified water, the water-soluble components was dissolved upon heating and maintained at 70°C (i.e., an aqueous phase). The oily components were mixed to the liquid oil, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example III-1: Antimicrobial Effect

The minimum inhibiting concentrations (MIC) for various types of microbials were carried out for 3-phenoxy-1,2-propane diol, 3-benzyloxy-1,2-propane diol, methyl p-oxybenzoate.

Using the agar plate method, for bacteria, the following various bacteria were inoculated in SCD agar media (made by Eiken) containing 3-hexine-2,5-diol in different concentrations and cultured at 30°C for 24 hours. The concentrations of 3-hexine-2,5-diol not forming colonies (minimum inhibiting concentration: MIC) were found. Further, for fungi, the following various bacteria were inoculated in a potato dextrose agar media containing 3-hexine-2,5-diol in different concentrations and cultured at 25°C for 48 hours. The concentrations of 3-hexine-2,5-diol not forming colonies (minimum inhibiting concentration: MIC) were found. The same was conducted for methyl paraoxybenzoate methyl. The results of the judgment are shown in Table III-1 based on the following evaluation criteria:

### (Test Bacteria)

- Ps:: *Pseudomonas aeuginosa* (ATCC1542)
- E:: *Escherichia coli* (ATCC8739)
- S:: *Staphylococcus* aureus (ATCC6538)
- Can:: *Candida albicans* (ATCC10231)
- Asp:: *Aspergillus niger* (ATCC16404)

### (Evaluation Criteria)

A: Minimum inhibiting concentration of less than 1000 ppm
B: Minimum inhibiting concentration of 1000 ppm to less than 5000 ppm
C: Minimum inhibiting concentration of 5000 ppm to less than 10000 ppm
D: Minimum inhibiting concentration of 10000 ppm to less than 30000 ppm
E: Minimum inhibiting concentration or 30000 ppm or more

**Table III-1**

| Test bacteria | Antimicrobial effect | | |
|---|---|---|---|
| | 3-Phenoxy-1,2-propane diol | 3-Benzyloxy-1,2-propane diol | Methyl p-oxybenzoate |
| Pseudomonas aeuginosa (ATCC15442) | A | A | C |
| Escherichia coli (ATCC8739) | A | A | B |
| Staphylococcus aureus (ATCC6538) | A | A | B |
| Candida albicans (ATCC10231) | A | A | B |
| Aspergillus niger (ATCC16404) | A | A | B |

### Example III-2: Safety Test

The glycerol derivatives of the present invention i.e., 3-phenoxy-1,2-propane diol and 3-benzyloxy-1,2-propane diol, were tested for safety. A single administration toxicity test was conducted, as a result, of which the toxicity was judged to be extremely weak. Further, a primary skin irritation test and a continuous skin irritation test were conducted, as a result, of which the skin irritability was judged to be extremely weak. Further, a skin sensitization test and a genetic toxicity test were conducted, as a result, of which negative results were obtained.

As explained above, the safety of the glycerol derivatives of the present invention i.e., 3-phenoxy-1,2-propane diol and 3-benzyloxy-1,2-propane diol, were good.

### Example III-1: Lotion

| | mass% |
|---|---|
| Ethanol | 5.0 |
| 1,3-Butylene glycol | 6.0 |
| Glycerol | 4.0 |
| Oleyl alcohol | 0.1 |
| POE (20) sorbitan monolaurate | 0.5 |
| POE (15) lauryl ether | 0.5 |
| 3-Phenoxy-1,2-propanediol | 1.0 |
| Fragrance | q.s. |
| Purified water | balance |

### Example III-2: Lotion

| | mass% |
|---|---|
| Ethanol | 5.0 |
| 1,3-Butylene glycol | 6.0 |
| Glycerol | 4.0 |
| Oleyl alcohol | 0.1 |
| POE (20) sorbitan monolaurate | 0.5 |
| POE (15) lauryl ether | 0.5 |
| 3-Benzyloxy-1,2-propanediol | 1.0 |
| Fragrance | q.s. |
| Purified water | balance |

### Example III-3: Lotion

| | mass% |
|---|---|
| Ethanol | 5.0 |
| 1,3-Butylene glycol | 6.0 |
| Glycerol | 4.0 |
| Ethylhexanediol | 0.2 |
| 2,2-Diethyl-1,3-propanediol | 0.3 |
| 2,2,4-Trimethyl-1,3-pentanediol | 0.5 |
| Oleyl alcohol | 0.1 |
| POE (20) sorbitan monolaurate | 0.5 |
| POE (15) lauryl ether | 0.5 |
| 3-Phenoxy-1,2-propanediol | 0.7 |
| 3-Benzyloxy-1,2-propanediol | 0.4 |
| Fragrance | q.s. |
| Purified water | balance |

### Example III-4: Lotion

| | mass% |
|---|---|
| Ethanol | 5.0 |
| 1,3-Butylene glycol | 6.0 |
| Glycerol | 5.0 |
| Oleyl alcohol | 0.1 |
| Ethylhexanediol | 0.3 |
| 2,2-Dimethylol pentane | 0.2 |
| POE (20) sorbitan monolaurate | 0.5 |
| POE (15) lauryl ether | 0.5 |
| 3-Phenoxy-1,2-propanediol | 0.7 |
| Fragrance | q.s. |
| Purified water | balance |

### Example III-5: Lotion

| | mass% |
|---|---|
| Ethanol | 5.0 |
| 1,3-Butylene glycol | 6.0 |
| Glycerol | 5.0 |
| Oleyl alcohol | 0.1 |
| Ethylhexanediol | 0.2 |
| POE (20) sorbitan monolaurate | 0.5 |
| POE (15) lauryl ether | 0.5 |
| 3-Phenoxy-1,2-propanediol | 0.7 |
| Phenoxyethanol | 0.2 |
| Fragrance | q.s. |
| Purified water | balance |

### Example III-6: Lotion

| | mass% |
|---|---|
| Ethanol | 4.0 |
| 1,3-Butylene glycol | 6.0 |
| Glycerol | 4.0 |
| Oleyl alcohol | 0.1 |
| 2,2-Diethyl-1,3-propanediol | 0.1 |
| Ethylhexanediol | 0.3 |
| POE (20) sorbitan monolaurate | 0.5 |
| POE (15) lauryl ether | 0.5 |
| 3-Benzyloxy-1,2-propanediol | 0.8 |
| Phenoxyethanol | 0.2 |
| Fragrance | q.s. |
| Purified water | balance |

### Example III-7: Lotion

| | mass% |
|---|---|
| Sorbitol | 4.0 |
| 1,3-Butylene glycol | 6.0 |
| Glycerol | 2.0 |
| POE (20) oleyl alcohol ether | 0.5 |
| Methyl cellulose | 0.2 |
| Quince Seed | 0.1 |
| 2,2-Diethyl-1,3-propane diol | 0.1 |
| Ethylhexanediol | 0.3 |
| 3-Benzyloxy-1,2-propanediol | 0.8 |
| Methyl paraben | 0.2 |
| Fragrance | q.s. |
| Purified water | balance |

### Example III-8: Emulsion

| | mass% |
|---|---|
| 3-Phenoxy-1,2-propanediol | 3.0 |
| Glycerol | 3.0 |
| Ethylhexanediol | 1.0 |
| Cetanol | 1.5 |
| Stearyl alcohol | 1.8 |
| Dimethyl polysiloxane (20 cs) | 1.5 |
| Squalane | 2.0 |
| Vaseline | 2.0 |
| Isopropyl myristate | 2.5 |
| Glyceryl monostearate | 1.8 |
| Polyoxyethylene (POE = 5) glyceryl monostearate | 1.8 |
| Polyoxyethylene (POE = 20) cetyl ether | 1.5 |
| Carboxyvinyl polymer | 0.25 |
| Potassium hydroxide | 0.05 |
| L-Arginine | 0.2 |
| Dipropylene glycol | 5.0 |
| 1,3-Butylene glycol | 3.0 |
| Trisodium edetate | 0.2 |
| Methyl paraben | 0.01 |
| Purified water | balance |

### Example III-9: Emulsion

| | mass% |
|---|---|
| 3-Phenoxy-1,2-propanediol | 1.0 |
| Glycerol | 5.0 |
| Ethylhexanediol | 2.0 |
| Cetanol | 1.5 |
| Stearyl alcohol | 1.8 |
| Dimethyl polysiloxane (20 cs) | 1.5 |
| Squalane | 2.0 |
| Vaseline | 2.0 |
| Isopropyl myristate | 2.4 |
| Glyceryl monostearate | 1.8 |
| Polyoxyethylene (POE = 5) glyceryl monostearate | 1.8 |
| Polyoxyethylene (POE = 20) Cetylether | 1.5 |
| Carboxyvinyl polymer | 0.25 |
| Potassium hydroxide | 0.05 |
| L-Arginine | 0.2 |
| Dipropylene glycol | 5.0 |
| 1,3-Butylene glycol | 3.0 |
| Trisodium edetate | 0.2 |
| Phenoxyethanol | 0.02 |
| Purified water | balance |

### Example III-10: Emulsion

| | mass% |
|---|---|
| 3-Benzyloxy-1,2-propanediol | 3.0 |
| Glycerol | 3.0 |
| Cetanol | 1.5 |
| Ethylhexanediol | 1.0 |
| Stearyl alcohol | 1.8 |
| Dimethyl polysiloxane (20 cs) | 1.5 |
| Squalane | 2.0 |
| Vaseline | 2.0 |
| Isopropyl myristate | 2.5 |
| Glyceryl monostearate | 1.8 |
| Polyoxyethylene (POE = 5) Glyceryl monostearate | 1.8 |
| Polyoxyethylene (POE = 20) cetyl ether | 1.5 |
| Carboxyvinyl polymer | 0.25 |
| Potassium hydroxide | 0.05 |
| L-Arginine | 0.2 |
| Dipropylene glycol | 5.0 |
| 1,3-Butylene glycol | 3.0 |
| Trisodium edetate | 0.2 |
| Methyl paraben | 0.01 |
| Purified water | balance |

### Example III-11: Emulsion

| | mass% |
|---|---|
| 3-Phenoxy-1,2-propanediol | 1.0 |
| 3-Benzyloxy-1,2-propanediol | 2.0 |
| Glycerol | 3.0 |
| Cetanol | 1.5 |
| Stearyl alcohol | 1.8 |
| Ethylhexane diol | 1.0 |
| 2,2,4-Trimethyl-1,3-pentanediol | 0.5 |
| Dimethyl polysiloxane (20 cs) | 1.5 |
| Squalane | 2.0 |
| Vaseline | 2.0 |
| Isopropyl myristate | 2.5 |
| Glyceryl monostearate | 1.8 |
| Polyoxyethylene (POE = 5) glyceryl monostearate | 1.8 |
| Polyoxyethylene (POE = 20) cetyl ether | 1.5 |
| Carboxyvinyl polymer | 0.25 |
| Potassium hydroxide | 0.05 |
| L-Arginine | 0.2 |
| Dipropylene glycol | 5.0 |
| 1,3-Butylene glycol | 3.0 |
| Trisodium edetate | 0.2 |
| Methyl paraben | 0.01 |
| Purified water | balance |

### Example III-12: Cream

| | mass% |
|---|---|
| 3-Phenoxy-1,2-propanediol | 10.0 |
| Stearyl alcohol | 3.5 |
| Stearic acid | 2.0 |
| Squalane | 10.5 |
| Isopropyl myristate | 7.5 |
| Polyoxyethylene (POE = 25) cetyl alcohol ether | 3.0 |
| Glycerol monostearate | 2.0 |
| Tocopherol acetate | 0.2 |
| Monoammonium glycyrrhetinate | 0.05 |
| Glycerol | 3.0 |
| Dipropylene glycol | 5.0 |
| 1,3-Butylene glycol | 3.0 |
| Phenoxyethanol | 0.2 |
| Trisodium edetate | 0.01 |
| Ethyl paraben | 0.1 |
| Purified water | balance |

### Example III-13: Cream

| | mass% |
|---|---|
| 3-Benzyloxy-1,2-propanediol | 10.0 |
| Stearyl alcohol | 3.5 |
| Stearic acid | 2.0 |
| Squalane | 10.5 |
| Isopropyl myristate | 7.5 |
| Polyoxyethylene (POE = 25) cetyl alcohol ether | 3.0 |
| Glyceryl monostearate | 2.0 |
| Tocopherol acetate | 0.2 |
| Monoammonium glycyrrhetinate | 0.05 |
| Glycerol | 3.0 |
| Dipropylene glycol | 5.0 |
| 1,3-Butylene glycol | 3.0 |
| Trisodium edetate | 0.01 |
| Ethyl paraben | 0.1 |
| Purified water | balance |

### Example III-14: Cream

| | mass% |
|---|---|
| 3-Benzyloxy-1,2-propanediol | 4.0 |
| 3-Phenoxy-1,2-propanediol | 5.0 |
| Ethylhexanediol | 3.0 |
| 2,2,4-Trimethyl-1,3-pentanediol | 1.0 |
| 2,2-Diethyl-1,3-propanediol | 0.5 |
| 2,2-Dimethylol pentane | 0.2 |
| Stearyl alcohol | 3.5 |
| Stearic acid | 2.0 |
| Squalane | 10.5 |
| Isopropyl myristate | 7.5 |
| Polyoxyethylene (POE = 25) cetyl alcohol ether | 3.0 |
| Glyceryl monostearate | 2.0 |
| Tocopherol acetate | 0.2 |
| Monoammonium glycyrrhetinate | 0.05 |
| Glycerol | 3.0 |
| Dipropylene glycol | 5.0 |
| 1,3-Butylene glycol | 3.0 |
| Phenoxyethanol | 0.2 |
| Trisodium edetate | 0.01 |
| Ethyl paraben | 0.1 |
| Purified water | balance |

### Example III-15: Cleansing

| | mass% |
|---|---|
| 3-Phenoxy-1,2-propanediol | 0.5 |
| Stearic acid | 8.0 |
| Palmitic acid | 6.0 |
| Myristic acid | 6.0 |
| Lauric acid | 4.0 |
| Potassium hydroxide | 5.2 |
| Glyceryl monostearate | 2.0 |
| Propylene glycol | 1.0 |
| Bees wax | 1.5 |
| Polyethylene glycol 1500 | 5.0 |
| Glycerol | 10.0 |
| Purified water | balance |

### Example III-16: Cleansing

| | mass% |
|---|---|
| 3-Benzyloxy-1,2-propanediol | 0.5 |
| Stearic acid | 8.0 |
| Palmitic acid | 6.0 |
| Myristic acid | 6.0 |
| Lauric acid | 4.0 |
| Potassium hydroxide | 5.2 |
| Glyceryl monostearate | 2.0 |
| Propylene glycol | 1.0 |
| Bees wax | 1.5 |
| Polyethylene glycol 1500 | 5.0 |
| Glycerol | 10.0 |
| Purified water | balance |

### Example III-17: Cleansing

| | mass% |
|---|---|
| 3-Benzyloxy-1,2-propanediol | 0.3 |
| 3-Phenoxy-1,2-propanediol | 0.2 |
| Stearic acid | 8.0 |
| Palmitic acid | 6.0 |
| Myristic acid | 6.0 |
| Lauric acid | 4.0 |
| Ethylhexanediol | 0.5 |
| 2,2,4-Trimethyl-1,3-pentanediol | 0.5 |
| 2,2-Diethyl-1,3-propanediol | 0.5 |
| 2,2-Dimethylol pentane | 0.2 |
| Potassium hydroxide | 5.2 |
| Glyceryl monostearate | 2.0 |
| Propylene glycol | 1.0 |
| Bees wax | 1.5 |
| Polyethylene glycol 1500 | 5.0 |
| Glycerol | 10.0 |
| Purified water | balance |

### Example III-18: Shampoo

| | mass% |
|---|---|
| Lauryl polyoxyethylene (3) sulfate ester sodium salt (30% aqueous solution) | 25.0 |
| Lauryl sulfate ester sodium salt (30% aqueous solution) | 8.0 |
| Coconut oil fatty acid diethanol amide | 4.0 |
| Isoprene glycol | 4.0 |
| Dipropylene glycol | 1.0 |
| 1,3-Butylene glycol | 1.0 |
| Trisodium edetate | 0.01 |
| 2-Butyl-2-methyl-1,3-propanediol | 0.2 |
| 3-Phenoxy-1,2-propanediol | 0.1 |
| Dye | q.s. |
| Fragrance | q.s. |
| Purified water | balance |

### Example III-19: Shampoo

| | mass% |
|---|---|
| Lauryl polyoxyethylene (3) sulfate ester sodium salt (30% aqueous solution) | 25.0 |
| Lauryl sulfate ester sodium salt (30% aqueous solution) | 8.0 |
| Coconut oil fatty acid diethanolamide | 4.0 |
| Isoprene glycol | 3.0 |
| Dipropylene glycol | 1.0 |
| 1,3-Butylene glycol | 1.0 |
| Ethylhexanediol | 0.5 |
| 2,2-Diethyl-1,3-propanediol | 0.4 |
| Trisodium edetate | 0.01 |
| 3-Phenoxy-1,2-propanediol | 0.1 |
| Dye | q.s. |
| Fragrance | q.s. |
| Purified water | balance |

### Example III-20: Shampoo

| | mass% |
|---|---|
| Lauryl polyoxyethylene (3) sulfate ester sodium salt (30% aqueous solution) | 25.0 |
| Lauryl sulfate ester sodium salt (30% aqueous solution) | 8.0 |
| Coconut oil fatty acid diethanolamide | 4.0 |
| Isoprene glycol | 4.0 |
| Dipropylene glycol | 1.0 |
| 2,2-Diethyl-1,3-propanediol | 0.5 |
| 1,3-Butylene glycol | 1.0 |
| Trisodium edetate | 0.01 |
| 3-Benzyloxy-1,2-propanediol | 0.1 |
| Dye | q.s. |
| Fragrance | q.s. |
| Purified water | balance |

### Example III-21: Gelly pack

| | mass% |
|---|---|
| 3-Phenoxy-1,2-propanediol | 0.1 |
| Polyoxyethylene oleyl alcohol ether | 0.5 |
| Monoammonium glycyrrhizinate | 0.05 |
| Carboxymethyl cellulose | 5.0 |
| Ethanol | 12.0 |
| Polyvinyl alcohol | 12.0 |
| 1,3-Butylene glycol | 5.0 |
| Trisodium edetate | 0.01 |
| Purified water | balance |

### Example III-22: Gelly pack

| | mass% |
|---|---|
| 3-Benzyloxy-1,2-propanediol | 0.05 |
| 3-Phenoxy-1,2-propanediol | 0.1 |
| Polyoxyethylene oleyl alcohol ether | 0.5 |
| 2,2-diethyl-1,3-propanediol | 0.1 |
| Ethylhexanediol | 0.1 |
| 2,2,4-Trimethyl-1,3-propanediol | 0.1 |
| 2,2-Dimethylol pentane | 0.1 |
| Monoammonium glycyrrhizinate | 0.05 |
| Carboxymethyl cellulose | 5.0 |
| Ethanol | 12.0 |
| Polyvinyl alcohol | 12.0 |
| 1,3-Butylene glycol | 5.0 |
| Trisodium edetate | 0.01 |
| Purified water | balance |

### Example III-23: Gelly pack

| | mass% |
|---|---|
| 3-Benzyloxy-1,2-propanediol | 0.1 |
| Polyoxyethylene oleyl alcohol ether | 0.5 |
| Monoammonium glycyrrhizinate | 0.05 |
| Carboxymethyl cellulose | 5.0 |
| Ethanol | 12.0 |
| Polyvinyl alcohol | 12.0 |
| 1,3-Butylene glycol | 5.0 |
| Trisodium edetate | 0.01 |
| Purified water | balance |

### Example III-24: Eyeliner

| | mass% |
|---|---|
| 3-Phenoxy-1,2-propanediol | 3.0 |
| Iron oxide (black) | 14.0 |
| Isopropyl myristate | 1.5 |
| Polyoxyethylene sorbitan monooleic ester | 1.0 |
| Vinylacetate resin emulsion | 45.0 |
| Monoammonium glycyrrhizinate | 0.05 |
| Carboxyvinyl polymer | 1.5 |
| Acetyltributyl citrate | 1.0 |
| Dipropylene glycol | 5.0 |
| Ethylhexanediol | 1.0 |
| 2,2,4-Trimethyl-1,3-pentanediol | 1.0 |
| 2,2-Diethyl-1,3-pentanediol | 1.0 |
| 2,2-Dimethylol pentane | 0.5 |
| 1,2-Pentanediol | 2.0 |
| Trisodium edetate | 0.01 |
| Purified water | balance |

### Example III-25: Eyeliner

| | mass% |
|---|---|
| 3-Benzyloxy-1,2-propanediol | 3.0 |
| Iron oxide (black) | 14.0 |
| Isopropyl myristate | 1.5 |

| | |
|---|---|
| Polyoxyethylene sorbitan monooleic ester | 1.0 |
| Vinylacetate resin emulsion | 45.0 |
| Monoammonium glycyrrhizinate | 0.05 |
| Carboxyvinyl polymer | 1.5 |
| Acetyltributyl citrate | 1.0 |
| Dipropylene glycol | 5.0 |
| Ethylhexanediol | 1.0 |
| 2,2-Diethyl-1,3-pentanediol | 1.0 |
| 2,2,4-Trimethyl-1,3-pentanediol | 0.5 |
| 1,2-Pentanediol | 3.0 |
| Trisodium edetate | 0.01 |
| Purified water | balance |

### Example III-26: Eyeliner

| | mass% |
|---|---|
| 3-Benzyloxy-1,2-propanediol | 2.0 |
| 3-Phenoxy-1,2-propanediol | 1.0 |
| Iron oxide (black) | 14.0 |
| Isopropyl myristate | 1.5 |
| Polyoxyethylene sorbitan monooleic ester | 1.0 |
| Vinylacetate resin emulsion | 45.0 |
| Monoammonium glycyrrhizinate | 0.05 |
| Carboxyvinyl polymer | 1.5 |
| Acetyltributyl citrate | 1.0 |
| Dipropylene glycol | 5.0 |
| 2,2-Dimethylol pentane | 1.0 |
| 2,2,4-Trimethyl-1,3-pentanediol | 1.0 |
| 2,2-Diethyl-1,3-propanediol | 0.5 |
| Ethylhexanediol | 1.0 |
| Phenoxyethanol | 0.1 |
| Trisodium edetate | 0.01 |
| Purified water | balance |

### Example III-27: Hair tonic

| | mass% |
|---|---|
| Hydrogenated castor oil ethyleneoxide (40 mol) addition product | 2.0 |
| Ethanol | 60.0 |
| Fragrance | q.s. |
| 3-Phenoxy-1,2-propanediol | 0.01 |
| Purified water | balance |

### Example III-28: Hair tonic

| | mass% |
|---|---|
| Hydrogenated castor oil ethyleneoxide (40 mol) addition product | 2.0 |
| Ethanol | 60.0 |
| Fragrance | q.s. |
| 3-Benzyloxy-1,2-propanediol | 0.01 |
| Purified water | balance |

### Example III-29: Bath agent

| | mass% |
|---|---|
| Sodium bicarbonate | 60.0 |
| Anhydrous sodium sulfate | 35.0 |
| 3-Phenoxy-1,2-propanediol | 5.0 |

### Example III-30: Bath agent

| | mass% |
|---|---|
| Sodium bicarbonate | 60.0 |
| Anhydrous sodium sulfate | 35.0 |
| 3-Benzyloxy-1,2-propanediol | 5.0 |

### Example III-31: Bath agent

| | mass% |
|---|---|
| Sodium bicarbonate | 60.0 |
| Anhydrous sodium sulfate | 35.0 |
| 3-Benzyloxy-1,2-propanediol | 3.0 |
| 3-Phenoxy-1,2-propanediol | 2.0 |

### Example III-32: Chinese noodle

| | mass% |
|---|---|
| Wheat flour | 98.0 |
| Table salt | 1.0 |
| Sweetner | 0.5 |
| 3-Phenoxy-1,2-propanediol | 0.5 |

### Example III-33: Chinese noodle

| | mass% |
|---|---|
| Wheat flour | 98.0 |
| Table salt | 1.0 |
| Sweetner | 0.5 |
| 3-Benzyloxy-1,2-propanediol | 0.5 |

### Example III-34: Chinese noodle

| | mass% |
|---|---|
| Wheat flour | 98.0 |
| Table salt | 1.0 |
| Sweetner | 0.5 |
| 3-Phenoxy-1,2-propanediol | 0.2 |
| 3-Benzyloxy-1,2-propanediol | 0.2 |

### Example III-35: Noodle soup

| | mass% |
|---|---|
| Soysauce | 80.0 |
| Vinegar | 1.0 |
| Glucose | 15.0 |
| Sodium glutamate | 2.0 |
| Sugar | 1.0 |
| 3-Phenoxy-1,2-propanediol | 1.0 |

### Example III-36: Noodle soup

| | mass% |
|---|---|
| Soysauce | 80.0 |
| Vinegar | 1.0 |
| Glucose | 15.0 |
| Sodium glutamate | 2.0 |
| Sugar | 1.0 |
| 3-Benzyloxy-1,2-propanediol | 1.0 |

### Example III-37: Noodle soup

| | mass% |
|---|---|
| Soysauce | 80.0 |
| Vinegar | 1.0 |
| Glucose | 15.0 |
| Sodium glutamate | 2.0 |
| Sugar | 1.0 |
| 3-Benzyloxy-1,2-propanediol | 0.5 |
| 3-Phenoxy-1,2-propanediol | 0.3 |

### Example III-38: Japanese noodle ("Soba")

| | mass% |
|---|---|
| Soba flour | 96.0 |
| Table salt | 0.9 |
| Water | 3.0 |
| 3-Phenoxy-1,2-propanediol | 0.1 |

### Example III-39: Japanese noodle ("Soba")

| | mass% |
|---|---|
| Soba flour | 96.0 |
| Table salt | 0.9 |
| Water | 3.0 |
| 3-Benzyloxy-1,2-propanediol | 0.1 |

### Example III-40: Japanese noodle ("Soba")

| | mass% |
|---|---|
| Soba flour | 96.0 |
| Table salt | 0.9 |
| Water | 3.0 |
| 3-Phenoxy-1,2-propanediol | 0.03 |
| 3-Benzyloxy-1,2-propanediol | 0.06 |

### Example III-41: Bread

| | mass% |
|---|---|
| Wheat flour | 90.0 |
| Table salt | 1.2 |
| Sugar | 2.0 |
| Water | 6.0 |
| 3-Phenoxy-1,2-propanediol | 0.8 |

### Example III-42: Bread

| | mass% |
|---|---|
| Wheat flour | 90.0 |
| Table salt | 1.2 |
| Sugar | 2.0 |
| Water | 6.0 |
| 3-Benzyloxy-1,2-propanediol | 0.8 |

### Example III-43: Bread

| | mass% |
|---|---|
| Wheat flour | 90.0 |
| Table salt | 1.2 |
| Sugar | 2.0 |
| Water | 6.0 |
| 3-Phenoxy-1,2-propanediol | 0.1 |
| 3-Benzyloxy-1,2-propanediol | 0.7 |

### Example III-44: Ham

| | mass% |
|---|---|
| Minced meat | 95.0 |
| Chicken egg | 4.0 |
| Table salt | 0.5 |
| Spice | 0.4 |
| 3-Phenoxy-1,2-propanediol | 0.1 |

### Example III-45: Ham

| | mass% |
|---|---|
| Minced meat | 95.0 |
| Chicken egg | 4.0 |
| Table salt | 0.5 |
| Spice | 0.4 |
| 3-Benzyloxy-1,2-propanediol | 0.1 |

### Example III-46: Ham

| | mass% |
|---|---|
| Minced meat | 95.0 |
| Chicken egg | 4.0 |
| Table salt | 0.5 |
| Spice | 0.4 |
| 3-Phenoxy-1,2-propanediol | 0.3 |
| 3-Benzyloxy-1,2-propanediol | 0.6 |

### Example III-47: Fruitjuice beverage

| | mass% |
|---|---|
| Glucose liquid sugar | 13.0 |
| Orange fruit juice | 85.0 |
| Fragrance | 1.0 |
| 3-Phenoxy-1,2-propanediol | 1.0 |

### Example III-48: Fruitjuice beverage

| | mass% |
|---|---|
| Glucose liquid sugar | 13.0 |
| Orange fruit juice | 85.0 |
| Fragrance | 1.0 |
| 3-Benzyloxy-1,2-propanediol | 1.0 |

### Example III-49: Fruitjuice beverage

| | mass% |
|---|---|
| Glucose liquid sugar | 13.0 |
| Orange fruit juice | 85.0 |
| Fragrance | 1.0 |
| 3-Phenoxy-1,2-propanediol | 0.3 |
| 3-Benzyloxy-1,2-propanediol | 0.6 |

### Usability Test and Preservability Test

The lotion having the formulation shown in Table IV-1 was prepared according to the following preparation method and subjected to the usability and preservability (antisepsis) tests.

### Preparation of Lotion

To the purified water, 3-Phenoxy-1,2-propanediol was added for Examples IV-1, IV-2 and IV-3 and 3-benzyloxy-1,2-propanediol was added for Examples IV-4, IV-5 and IV-6. Citric acid, trisodium citrate, trisodium edetate are dissolved therein (i.e., an aqueous phase). Ethanol, glycerol, 1,3-butylene glycol, POE (60) hydrogenated castor oil, methyl paraben are dissolved (i.e., an alcohol phase). The aqueous phase and the alcohol phase were mixed.

### Test Method for Usability

A panel consisting of 10 women having a sensitive skin, who had an irritation feeling when an external skin treatment composition containing paraben was used. The test was carried out in such a manner that each paneller used the above external skin treatment composition twice a day for 1 week and, based upon the satisfactory degree and the presence or absence of the skin irritation of each paneller, the usability is evaluated based upon the following 4-rank standards. The number of the persons having irritation feeling was also counted.
A: Number of paneller having good feeling is 8 or more
B: Number of paneller having good feeling is 5 to less than 8
C: Number of paneller having good feeling is 3 to less than 5
D: Number of paneller having good feeling is less than 3

### Test Method for Judging Preservability

To 30 ml of the samples of the Examples and Comparative Examples, microbials in a liquid were inoculated and then the change in the number of the microbial was checked after 2 weeks from the inoculation by a smear culture method. The inoculated microbials were as follows.
Mould: Aspergillus niger ATCC16404. Inoculation amount 10⁴ cfu (colony forming unit)/g
Yeast: Candida albicans ATCC10231. Inoculation amount 10⁵ cfu/g
Bacteria: Escherichia coli ATCC8739. Inoculation amount 10⁶ cfu/g; Staphylococcus aureus ATCC6538. Inoculation amount 10⁶ cfu/g; Pseudomonas aeruginosa ATCC15442. Inoculation amount 10⁶ cfu/g

The preservability (or antisepsis) was evaluated used upon the changes in the number of the microbials and the results were classified to the following 4 ranks.
A: All of the mould, yeast and bacteria are decreased to 100 cfu/g or less within 1 week.
B: All of the mould, yeast and bacteria are decreased to 100 cfu/g or less within 2 weeks.
C: Either of the mould, yeast or bacteria remain in an amount of 100 cfu/g or more even after 2 weeks.
D: All of the mould, yeast and bacteria remain in an amount of 100 cfu/g or more even after 2 weeks.

As the preservability of an external skin treatment composition, those judged as "A" or "B" is acceptable. The results of the usability and preservability tests are shown in Table IV-1.

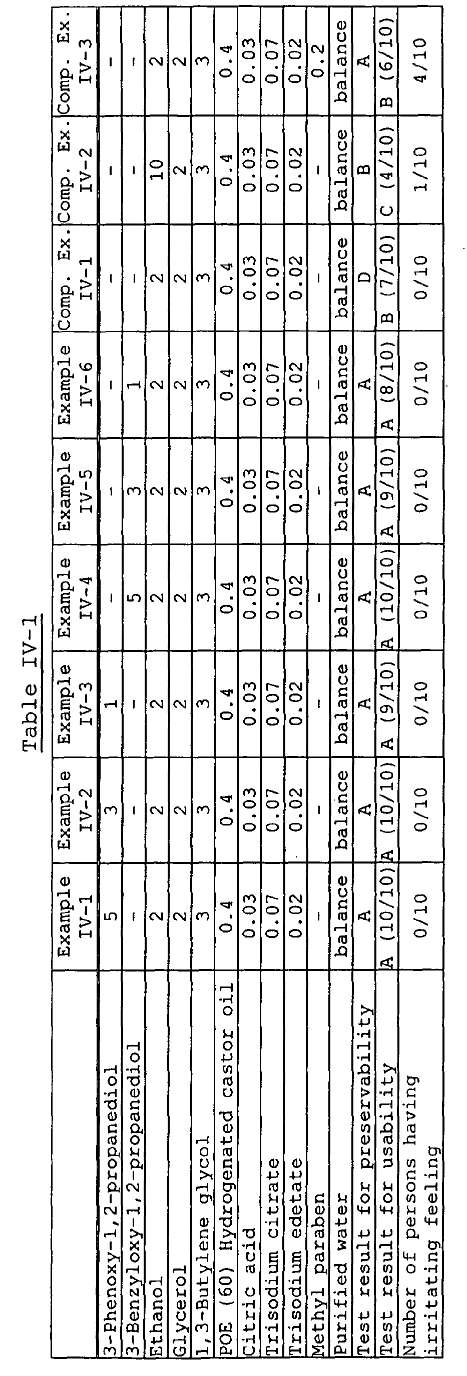

There were no persons having irritating feeling for Examples IV-1 to IV-6 and the ratio of the persons having satisfactory usability are large and thus the preservability was confirmed. This is the effect of the present invention. Also in Comparative Example IV-1, a large number of the panellers are satisfactory in the usability and the skin irritation is small, but the preservability is poor. In Comparative Example IV-2, there are no problems in the preservability, the satisfaction in the usability is low although the preservability is not problem, one paneller felt a skin irritation. Comparative Example IV-3 was problem in the skin irritation because many panellers had skin irritation although the preservability is excellent.

Next, the emulsion having the formulation shown in Table IV-2 was prepared according to the following preparation method and the usability and preservability tests were carried out in the same manner as mentioned above. The results of the usability and preservability tests are shown in Table IV-2.

### Preparation of Emulsion

To the purified water, 3-phenoxy-1,2-propanediol was added for Examples IV-7, IV-8 and IV-9 and 3-benzyloxy-1,2-propanediol for Examples IV-10, IV-11 and IV-12. To each of these, 1,3-butylene glycol, polyethylene glycol 1500, trisodium edetate, triethanol amine were added, followed by heating at 70°C to prepare an aqueous phase. Stearic acid, cetyl alcohol, vaseline, squalane were dissolved and sorbitan monooleic acid ester and methyl paraben were added thereto, followed by heating at 70°C to prepare an oil phase. The oil phase was added to the aqueous phase to the pre-emulsified and then uniformly emulsified in a homomixer followed by cooling.

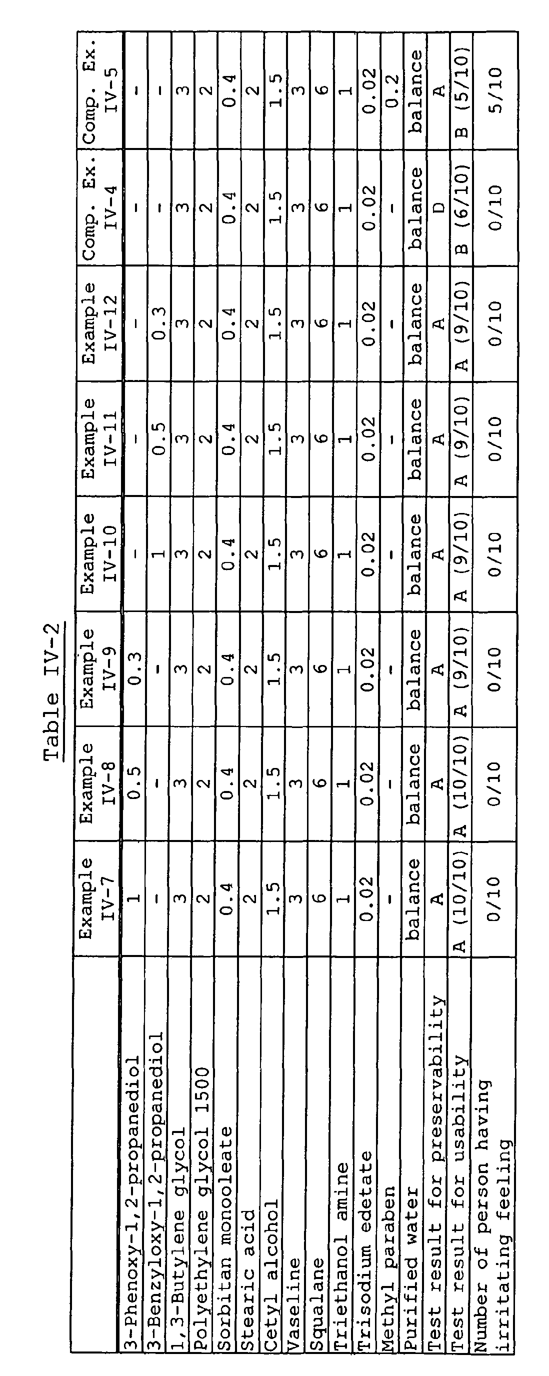

There were no persons having irritating feeling for Examples IV-7 to IV-12 and the ratio of the persons having satisfactory usability are large and thus the preservability was confirmed. This is the effect of the present invention. Also in Comparative Example IV-4, a large number of the panellers are satisfactory in the usability and the skin irritation is small, but the preservability is poor. Comparative Example IV-5 was problem in the skin irritation because many panellers had skin irritation although the usability and preservability are excellent.

Next, the emulsion having the formulation shown in Table IV-3 was prepared according to the following preparation method and the usability and preservability tests were carried out in the same manner as mentioned above. The results of the usability and preservability tests are shown in Table IV-3.

### Preparation of Cream

To the purified water, 3-phenoxy-1,2-propanediol was added for Example IV-13, IV-14 and IV-15 and 3-benzyloxy-1,2-propanediol for Examples IV-16, IV-17 and IV-18. To each of these, 1,3-butylene glycol, propylene glycol were added, followed by heating at 70°C to prepare an aqueous phase. Stearyl alcohol, stearyl alcohol, hydrogenated lanolin, squalane, octyl dodecanol were dissolved and POE (25) cetyl alcohol ether, glycerol monostearate and methyl paraben were added thereto, followed by heating at 70°C to prepare an oil phase. The oil phase was added to the aqueous phase and uniformly emulsified in a homomixer followed by cooling.

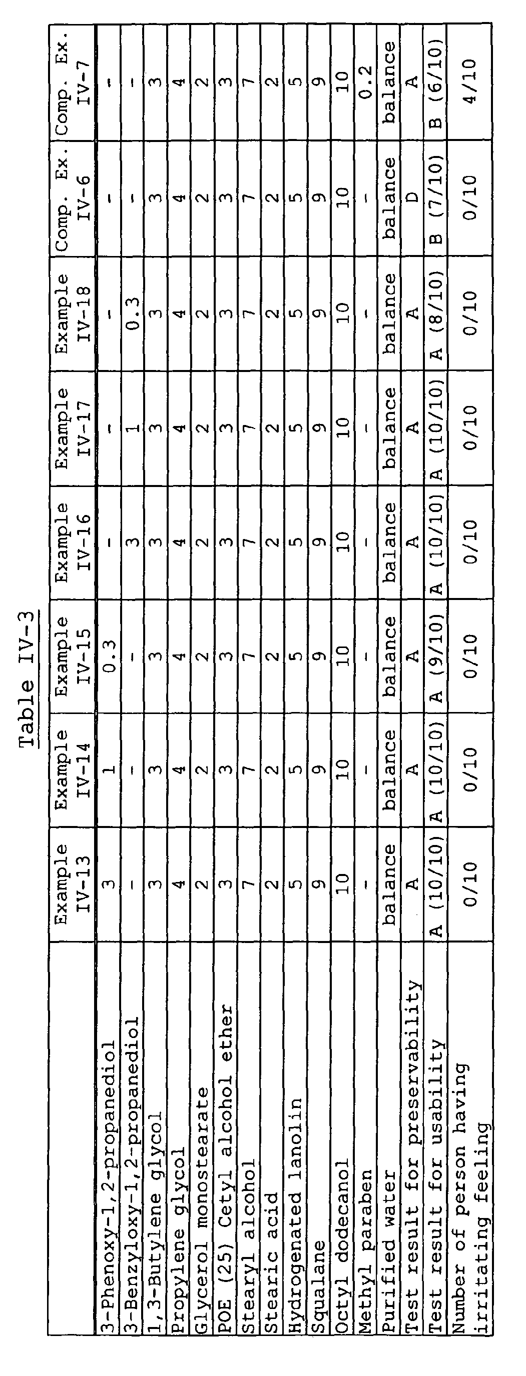

There were no persons having irritating feeling for Examples IV-13 to IV-18 and the ratio of the persons having satisfactory usability are large and thus the preservability was confirmed. This is the effect of the present invention. Also in Comparative Example IV-6, a large number of the panellers are satisfactory in the usability and the skin irritation is small, but the preservability is poor. Comparative Example IV-7 was problem in the skin irritation because many panellers had skin irritation although the usability and preservability are excellent.

Various external skin treatment composition will now be explained and all Examples have no skin irritation and good usability, while maintaining the preservability.

### Example IV-19: Lotion

| (Alcohol phase) | mass% |
|---|---|
| Ethanol | 5.0 |
| Oleyl alcohol | 0.2 |
| 2,2,4-Trimethyl-1,3-pentanediol | 0.5 |
| 2,2-Dimethylol pentane | 0.1 |
| Ethylhexanediol | 0.3 |
| 2,2-Diethyl-1,3-propanediol | 0.1 |
| 2,2-Diethylpropanediol | 0.1 |
| POE (20) Sorbitan monolaurate | 0.5 |
| POE (15) Lauryl ether | 0.5 |
| 4,5-Dimorpholino-3-hydroxypyridazine | 0.1 |
| Phenoxyethanol | 0.2 |
| Methyl paraben | 0.1 |
| Fragrance | q.s. |

| (Aqueous phase) | mass% |
|---|---|
| 1,3-Butylene glycol | 6.0 |
| 1,2-Pentanediol | 1.0 |
| 3-Phenoxy-1,2-propanediol | 1.0 |
| Glycerol | 5.0 |
| Purified water | balance |

### (Preparation method)

The aqueous phase and the alcohol phase were mixed after the preparation of the aqueous phase and the alcohol phase.

### Example IV-20: Lotion

| (Alcohol phase) | mass% |
|---|---|
| Ethanol | 5.0 |
| Oleyl alcohol | 0.2 |
| Ethylhexanediol | 1.0 |
| 2,2,4-Trimethyl-1,3-pentanediol | 0.3 |
| 2,2-Diethyl-1,3-propanediol | 0.5 |
| 2,2-Diethyl-1,3-propanediol | 0.1 |
| 2,2-Dimethylol pentane | 0.1 |
| POE (20) Sorbitan monolaurate | 0.5 |
| POE (15) Lauryl ether | 0.5 |
| 4,5-Dimorpholino-3-hydroxypyridazine | 0.1 |
| Phenoxyethanol | 0.3 |
| Fragrance | q.s. |

| (Aqueous phase) | mass% |
|---|---|
| 1,3-Butylene glycol | 6.0 |
| 1,2-Pentanediol | 1.0 |
| 3-Phenoxy-1,2-propanediol | 0.7 |
| 3-Benzyloxy-1,2-propanediol | 0.5 |
| Glycerol | 5.0 |
| Purified water | balance |

### (Preparation method)

The aqueous phase and the alcohol phase were mixed after the preparation of the aqueous phase and the alcohol phase.

There were no persons having irritating feeling for Examples IV-13 to IV-18 and the ratio of the persons having satisfactory usability are large and thus the preservability was confirmed. This is the effect of the present invention. Also in Comparative Example IV-6, a large number of the panellers are satisfactory in the usability and the skin irritation is small, but the preservability is poor. Comparative Example IV-7 was problem in the skin irritation because many panellers had skin irritation although the usability and preservability are excellent.

Various external skin treatment composition will now be explained and all Examples have no skin irritation and good usability, while maintaining the preservability.

### Example IV-21: Lotion

| (Alcohol phase) | mass% |
|---|---|
| Ethanol | 5.0 |
| Oleyl alcohol | 0.2 |
| Ethylhexanediol | 1.0 |
| 2,2,4-Trimethyl-1,3-pentanediol | 0.3 |
| 2,2-Diethyl-1,3-propanediol | 0.5 |
| 2,2-Dimethyl-1,3-propanediol | 0.1 |
| POE (20) Sorbitan monolaurate | 0.5 |
| POE (15) Lauryl ether | 0.5 |
| 4,5-Dimorpholino-3-hydroxypyridazine | 0.1 |
| Methyl paraben | 0.1 |
| Fragrance | q.s. |

| (Aqueous phase) | mass% |
|---|---|
| 1,3-Butylene glycol | 6.0 |
| 1,2-Pentanediol | 1.0 |
| 3-Phenoxy-1,2-propanediol | 0.7 |
| 3-Benzyloxy-1,2-propanediol | 0.5 |
| Glycerol | 5.0 |
| Purified water | balance |

### (Preparation method)

The aqueous phase and the alcohol phase were mixed after the preparation of the aqueous phase and the alcohol phase.

### Example IV-22: Lotion

| (Alcohol phase) | mass% |
|---|---|
| Ethanol | 5.0 |
| POE (20) Oleyl ether | 0.5 |
| 2,2-Dimethylol pentane | 0.3 |
| Ethylhexanediol | 0.2 |
| 2,2,4-Trimethyl-1,3-propanediol | 0.1 |
| 2,2-Diethylpropanediol | 0.1 |
| Methyl paraben | 0.1 |
| Fragrance | q.s. |

| (Aqueous phase) | mass% |
|---|---|
| Dipropylene glycol | 6.0 |
| 3-Benzyloxy-1,2-propanediol | 0.5 |
| Sorbitol | 4.0 |
| PEG1500 | 5.0 |
| Methyl cellulose | 0.2 |
| Quince Seed | 0.1 |
| Purified water | balance |

### (Preparation method)

To a portion of the purified water, methyl cellulose and Quince Seed were mixed, followed by stirring to prepare a viscous liquid. The remainder of the purified water and the other components of the aqueous phase were mixed and dissolved, and the above viscous liquid were added to obtain a uniform aqueous phase. The alcohol phase was prepared and then added to the aqueous phase, followed by mixing.

### Example IV-23: Lotion

| (Alcohol phase) | mass% |
|---|---|
| Ethanol | 5.0 |
| POE (20) Oleyl ether | 0.5 |
| 1,3-Dimethylol propane | 0.1 |
| Ethylhexanediol | 0.5 |
| 2,2-Diethyl-1,3-propanediol | 0.1 |
| 2,2-Dimethyl-1,3-propanediol | 0.1 |
| 2,2,4-Trimethyl-1,3-propanediol | 0.1 |
| Fragrance | q.s. |

| (Aqueous phase) | mass% |
|---|---|
| Dipropylene glycol | 6.0 |
| Sorbitol | 4.0 |
| PEG1500 | 5.0 |
| 3-Benzyloxy-1,2-propanediol | 0.3 |
| 3-Phenoxy-1,2-propane diol | 0.2 |
| Methyl cellulose | 0.2 |
| Quince Seed | 0.1 |
| Purified water | balance |

### (Preparation method)

To a portion of the purified water, methyl cellulose and Quince Seed were mixed, followed by stirring to prepare a viscous liquid. The remainder of the purified water and the other components of the aqueous phase were mixed and dissolved, and the above viscous liquid were added to obtain a uniform aqueous phase. The alcohol phase was prepared and then added to the aqueous phase, followed by mixing.

### Example IV-24: Lotion

| (Alcohol phase) | mass% |
|---|---|
| Ethanol | 5.0 |
| POE (20) Oleyl ether | 0.5 |
| 3-Phenoxy-1,2-propanediol | 0.5 |
| 2,2,4-Trimethyl-1,3-pentanediol | 0.1 |
| 2,2-Diethylpropanediol | 0.1 |
| Ethylhexanediol | 0.5 |
| Fragrance | q.s. |

| (Aqueous phase) | mass% |
|---|---|
| Dipropylene glycol | 6.0 |
| Sorbitol | 4.0 |
| PEG1500 | 5.0 |
| Methyl cellulose | 0.2 |
| Quince Seed | 0.1 |
| Purified water | balance |

### (Preparation method)

To a portion of the purified water, methyl cellulose and Quince Seed were mixed, followed by stirring to prepare a viscous liquid. The remainder of the purified water and the other components of the aqueous phase were mixed and dissolved, and the above viscous liquid were added to obtain a uniform aqueous phase. The alcohol phase was prepared and then added to the aqueous phase, followed by mixing.

### Example IV-25: Lotion

| (Alcohol phase) | mass% |
|---|---|
| Ethanol | 5.0 |
| Ethylhexanediol | 0.2 |
| POE (20) Oleyl ether | 0.5 |
| 3-Phenoxy-1,2-propanediol | 0.5 |
| 3-Benzyloxy-1,2-propanediol | 0.5 |
| 2,2-Diethyl-1,3-propanediol | 0.1 |
| 2,2,4-Triethyl-1,3-pentanediol | 0.5 |
| Phenoxyethanol | 0.1 |
| Methyl paraben | 0.1 |
| Fragrance | q.s. |

| (Aqueous phase) | mass% |
|---|---|
| Dipropylene glycol | 6.0 |
| Sorbitol | 4.0 |
| PEG1500 | 5.0 |
| Methyl cellulose | 0.2 |
| Quince Seed | 0.1 |
| Purified water | balance |

### (Preparation method)

To a portion of the purified water, methyl cellulose and Quince Seed were mixed, followed by stirring to prepare a viscous liquid. The remainder of the purified water and the other components of the aqueous phase were mixed and dissolved, and the above viscous liquid were added to obtain a uniform aqueous phase. The alcohol phase was prepared and then added to the aqueous phase, followed by mixing.

### Example IV-26: Cream

| | mass% |
|---|---|
| Stearic acid | 5.0 |
| Stearyl alcohol | 4.0 |
| Isopropyl myristate | 18.0 |
| Glycerol monostearate ester | 3.0 |
| Propylene glycol | 10.0 |
| 1,2-Hexane diol | 3.0 |
| 2,2-Diethyl-1,3-propanediol | 0.1 |
| 3-Phenoxy-1,2-propanediol | 3.0 |
| Potassium hydroxide | 0.2 |
| Phenoxyethanol | 0.3 |
| Sodium bisulfite | 0.01 |
| Fragrance | q.s. |
| Purified water | balance |

### (Preparation method)

In the purified water, propylene glycol and potassium hydroxide were added to be dissolved, followed by heating and maintained at 70°C (i.e., an aqueous phase). The other components were mixed, followed by heating and melting and maintained at 70°C (i.e., an oil phase). The oil phase was gradually added to the aqueous phase to be pre-emulsified and uniformly emulsified in a homomixer, followed by cooling to 30°C, with mixing.

### Example IV-27: Cream

| | mass% |
|---|---|
| Stearic acid | 6.0 |
| Sorbitan monostearate | 2.0 |
| POE (20) Sorbitan monostearate | 1.5 |
| Propylene glycol | 10.0 |
| 2,2-Diethyl-1,3-propanediol | 0.1 |
| 3-Benzyloxy-1,2-propanediol | 5.0 |
| Glycerol trioctanoate | 10.0 |
| Squalane | 5.0 |
| Sodium bisulfite | 0.01 |
| Fragrance | q.s. |
| Purified water | balance |

### (Preparation method)

In the purified water, propylene glycol was added to be dissolved, followed by heating and maintained at 70°C (i.e., an aqueous phase). The other components were mixed, followed by heating and melting and maintained at 70°C (i.e., an oil phase). The oil phase was gradually added to the aqueous phase to be pre-emulsified and uniformly emulsified in a homomixer, followed by cooling to 30°C, with mixing.

### Example IV-28: Cream

| | mass% |
|---|---|
| Stearic acid | 6.0 |
| Sorbitan monostearate | 2.0 |
| POE (20) Sorbitan monostearate | 1.5 |
| Propylene glycol | 10.0 |
| 3-Benzyloxy-1,2-propanediol | 3.0 |
| 3-Phenoxy-1,2-propanediol | 2.0 |
| 2,2-Diethyl-1,3-propanediol | 0.1 |
| Glycerol trioctanoate | 10.0 |
| Squalane | 5.0 |
| Sodium bisulfite | 0.01 |
| Phenoxyethanol | 0.1 |
| Fragrance | q.s. |
| Purified water | balance |

### (Preparation method)

In the purified water, propylene glycol was added to be dissolved, followed by heating and maintained at 70°C (i.e., an aqueous phase). The other components were mixed, followed by heating and melting and maintained at 70°C (i.e., an oil phase). The oil phase was gradually added to the aqueous phase to be pre-emulsified and uniformly emulsified in a homomixer, followed by cooling to 30°C, with mixing.

### Example IV-29: Cream

| | mass% |
|---|---|
| Stearic acid | 6.0 |
| Sorbitan monostearate | 2.0 |
| POE (20) Sorbitan monostearate | 1.5 |
| Propylene glycol | 10.0 |
| 3-Benzyloxy-1,2-propanediol | 2.0 |
| 3-Phenoxy-1,2-propanediol | 3.0 |
| 2,2-Diethyl-1,3-propanediol | 0.1 |
| Glycerol trioctanoate | 10.0 |
| Squalane | 5.0 |
| Sodium bisulfite | 0.01 |
| Methyl paraben | 0.05 |
| Phenoxyethanol | 0.1 |
| Fragrance | q.s. |
| Purified water | balance |

### (Preparation method)

In the purified water, propylene glycol was added to be dissolved, followed by heating and maintained at 70°C (i.e., an aqueous phase). The other components were mixed, followed by heating and melting and maintained at 70°C (i.e., an oil phase). The oil phase was gradually added to the aqueous phase to be pre-emulsified and uniformly emulsified in a homomixer, followed by cooling to 30°C, with mixing.

### Example IV-30: Emulsion

| | mass% |
|---|---|
| Stearic acid | 2.5 |
| Cetyl alcohol | 1.5 |
| Vaseline | 5.0 |
| Liquid paraffin | 10.0 |
| POE (10) Monooleate | 2.0 |
| PEG1500 | 3.0 |
| Triethanolamine | 1.0 |
| 3-Phenoxy-1,2-propanediol | 5.0 |
| Sodium bisulfite | 0.01 |
| Carboxyvinyl polymer | 0.05 |
| Fragrance | q.s. |
| Purified water | balance |

### (Preparation method)

In a small amount of the purified water, carboxyvinyl polymer was dissolved (i.e., phase A). To the remainder of the purified water, PEG 1500 and triethanolamine were added and dissolved upon heating at 70°C (i.e., an aqueous phase). The other components were mixed, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example IV-31: Emulsion

| | mass% |
|---|---|
| Stearic acid | 2.5 |
| Cetyl alcohol | 1.5 |
| Vaseline | 5.0 |
| Liquid paraffin | 10.0 |
| POE (10) Monooleate | 2.0 |
| PEG1500 | 3.0 |
| Triethanolamine | 1.0 |
| 3-Benzyloxy-1,2-propanediol | 5.0 |
| Sodium bisulfite | 0.01 |
| Carboxyvinyl polymer | 0.05 |
| Fragrance | q.s. |
| Purified water | balance |

### (Preparation method)

In a small amount of the purified water, carboxyvinyl polymer was dissolved (i.e., phase A). To the remainder of the purified water, PEG 1500 and triethanolamine were added and dissolved upon heating at 70°C (i.e., an aqueous phase). The other components were mixed, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example IV-32: Emulsion

| | mass% |
|---|---|
| Stearic acid | 2.5 |
| Cetyl alcohol | 1.5 |
| Ethylhexanediol | 1.0 |
| 2,2,4-Trimethyl-1,3-propanediol | 0.5 |
| 2,2-dimethylolpropane | 0.3 |
| 2,2-diethyl-1,3-propanediol | 0.2 |
| Vaseline | 5.0 |
| Liquid paraffin | 10.0 |
| POE (10) Monooleate | 2.0 |
| PEG1500 | 3.0 |
| Triethanolamine | 1.0 |
| 3-Benzyloxy-1,2-propanediol | 3.0 |
| 3-Phenoxy-1,2-propanediol | 2.0 |
| Sodium bisulfite | 0.01 |
| Carboxyvinyl polymer | 0.05 |
| Phenoxyethanol | 0.1 |
| Methyl paraben | 0.1 |
| Fragrance | q.s. |
| Purified water | balance |

### (Preparation method)

In a small amount of the purified water, carboxyvinyl polymer was dissolved (i.e., phase A). To the remainder of the purified water, PEG 1500 and triethanolamine were added and dissolved upon heating at 70°C (i.e., an aqueous phase). The other components were mixed, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example IV-33: Emulsion

| | mass% |
|---|---|
| Stearic acid | 2.5 |
| Cetyl alcohol | 1.5 |
| Ethylhexanediol | 1.0 |
| 2,2,4-Trimethyl-1,3-propanediol | 0.5 |
| 2,2-Dimethylol propane | 0.3 |
| 2,2-Diethyl-1,3-propanediol | 0.2 |
| Vaseline | 5.0 |
| Liquid paraffin | 10.0 |
| POE (10) Monooleate | 2.0 |
| PEG1500 | 3.0 |
| Triethanolamine | 1.0 |
| 3-Benzyloxy-1,2-propanediol | 3.0 |
| 3-Phenoxy-1,2-propanediol | 2.0 |
| Sodium bisulfite | 0.01 |
| Carboxyvinyl polymer | 0.05 |
| Methyl paraben | 0.2 |
| Fragrance | q.s. |
| Purified water | balance |

### (Preparation method)

In a small amount of the purified water, carboxyvinyl polymer was dissolved (i.e., phase A). To the remainder of the purified water, PEG 1500 and triethanolamine were added and dissolved upon heating at 70°C (i.e., an aqueous phase). The other components were mixed, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example IV-34: Gel

| | mass% |
|---|---|
| 95% Ethanol | 5.0 |
| Dipropylene glycol | 15.0 |
| 1,2-Octane diol | 2.0 |
| Ethylhexanediol | 1.0 |
| 2,2-Dimethylol pentane | 0.1 |
| POE (50) Oleyl ether | 2.0 |
| Carboxyvinyl polymer | 1.0 |
| Sodium hydroxide | 0.15 |
| 3-Phenoxy-1,2-propanediol | 0.1 |
| Fragrance | q.s. |
| Purified water | balance |

### (Preparation method)

To the purified water, the carboxyvinyl polymer was uniformly dissolved (i.e., phase A). The POE (50) oleyl ether was dissolved in the 95% ethanol and then added to the phase A. After the components other than the sodium hydroxide was added, the sodium hydroxide was added thereto to be neutralized and thickened.

### Example IV-35: Gel

| | mass% |
|---|---|
| 95% Ethanol | 5.0 |
| Dipropylene glycol | 15.0 |
| 1,2-Octanediol | 2.0 |
| Ethylhexane diol | 1.0 |
| 2,2-Dimethylol pentane | 0.1 |
| POE (50) Oleyl ether | 2.0 |
| Carboxyvinyl polymer | 1.0 |
| Sodium hydroxide | 0.15 |
| 3-Benzyloxy-1,2-propanediol | 0.2 |
| Fragrance | q.s. |
| Purified water | balance |

### (Preparation method)

To the purified water, the carboxyvinyl polymer was uniformly dissolved (i.e., phase A). The POE (50) oleyl ether was dissolved in the 95% ethanol and then added to the phase A. After the components other than the sodium hydroxide was added, the sodium hydroxide was added thereto to be neutralized and thickened.

### Example IV-36: Gel

| | mass% |
|---|---|
| 95% Ethanol | 5.0 |
| Dipropylene glycol | 15.0 |
| 1,2-Octanediol | 2.0 |
| Ethylhexanediol | 1.0 |
| 2,2-Dimethylol pentane | 0.1 |
| 2,2-Dimethyl-1,3-propanediol | 0.1 |
| 2,2-Diethyl-1,3-propanediol | 0.1 |
| 2,2,4-Trimethyl-1,3-propanediol | 0.05 |
| POE (50) Oleyl ether | 2.0 |
| Carboxyvinyl polymer | 1.0 |
| Sodium hydroxide | 0.15 |
| 3-Benzyloxy-1,2-propanediol | 0.05 |
| 3-Phenoxy-1,2-propanediol | 0.05 |
| Phenoxyethanol | 0.1 |
| Methyl paraben | 0.1 |
| Fragrance | q.s. |
| Purified water | balance |

### (Preparation method)

To the purified water, the carboxyvinyl polymer was uniformly dissolved (i.e., phase A). The POE (50) oleyl ether was dissolved in the 95% ethanol and then added to the phase A. After the components other than the sodium hydroxide was added, the sodium hydroxide was added thereto to be neutralized and thickened.

### Example IV-37: Gel

| | mass% |
|---|---|
| 95% Ethanol | 5.0 |
| Dipropylene glycol | 15.0 |
| 1,2-Octanediol | 2.0 |
| Ethylhexanediol | 1.0 |
| 2,2-Dimethylol pentane | 0.1 |
| 2,2-Dimethyl-1,3-propanediol | 0.1 |
| 2,2-Diethyl-1,3-propanediol | 0.1 |
| 2,2,4-Trimethyl-1,3-propanediol | 0.05 |
| POE (50) Oleyl ether | 2.0 |
| Carboxyvinyl polymer | 1.0 |
| Sodium hydroxide | 0.15 |
| 3-Benzyloxy-1,2-propanediol | 0.05 |
| 3-Phenoxy-1,2-propanediol | 0.05 |
| Methyl paraben | 0.08 |
| Fragrance | q.s. |
| Purified water | balance |

### (Preparation method)

To the purified water, the carboxyvinyl polymer was uniformly dissolved (i.e., phase A). The POE (50) oleyl ether was dissolved in the 95% ethanol and then added to the phase A. After the components other than the sodium hydroxide was added, the sodium hydroxide was added thereto to be neutralized and thickened.

### Example IV-38: Beauty liquid

| | mass% |
|---|---|
| 95% Ethanol | 5.0 |
| POE (20) Octyldodecanol | 1.0 |
| Pantonyl ethyl ether | 0.1 |
| Potassium hydroxide | 0.1 |
| Glycerol | 5.0 |
| Dipropylene glycol | 10.0 |
| Sodium bisulfite | 0.03 |
| Carboxyvinyl polymer | 0.2 |
| 3-Phenoxy-1,2-propanediol | 0.3 |
| Phenoxyethanol | 0.3 |
| Purified water | balance |

### (Preparation method)

In a portion of the purified water, the carboxyvinyl polymer was dissolved (i.e., phase A). Similarly, a portion of the purified water, the potassium hydroxide was dissolved (i.e., phase B). In the remainder of the purified water, the water-soluble components were dissolved (i.e., phase C). To the ethanol, POE (20) octyl dodecanol and the pantotenyl ethyl ether were dissolved and then the above phase C was added thereto and mixed with stirring, then the above phase A was mixed therewith with mixing and thereafter the above phase B was added thereto and mixed with stirring in a homomixer.

### Example IV-39: Beauty liquid

| | mass% |
|---|---|
| 95% Ethanol | 5.0 |
| POE (20) Octyldodecanol | 1.0 |
| Pantonyl ethyl ether | 0.1 |
| Potassium hydroxide | 0.1 |
| Glycerol | 5.0 |
| Dipropylene glycol | 10.0 |
| Sodium bisulfite | 0.03 |
| Carboxyvinyl polymer | 0.2 |
| 3-Benzyloxy-1,2-propanediol | 0.4 |
| Phenoxyethanol | 0.3 |
| Purified water | balance |

### (Preparation method)

In a portion of the purified water, the carboxyvinyl polymer was dissolved (i.e., phase A). Similarly, a portion of the purified water, the potassium hydroxide was dissolved (i.e., phase B). In the remainder of the purified water, the water-soluble components were dissolved (i.e., phase C). To the ethanol, POE (20) octyl dodecanol and the pantotenyl ethyl ether were dissolved and then the above phase C was added thereto and mixed with stirring, then the above phase A was mixed therewith with mixing and thereafter the above phase B was added thereto and mixed with stirring in a homomixer.

### Example IV-40: Beauty liquid

| | mass% |
|---|---|
| 95% Ethanol | 5.0 |
| POE (20) Octyldodecanol | 1.0 |
| Ethylhexane diol | 0.1 |
| Pantonyl ethyl ether | 0.1 |
| Potassium hydroxide | 0.1 |
| Glycerol | 5.0 |
| Dipropylene glycol | 10.0 |
| Sodium bisulfite | 0.03 |
| Carboxyvinyl polymer | 0.2 |
| 3-Benzyloxy-1,2-propanediol | 0.3 |
| 3-Phenoxy-1,2-propanediol | 0.2 |
| Methyl paraben | 0.1 |
| Phenoxyethanol | 0.1 |
| Purified water | balance |

### (Preparation method)

In a portion of the purified water, the carboxyvinyl polymer was dissolved (i.e., phase A). Similarly, a portion of the purified water, the potassium hydroxide was dissolved (i.e., phase B). In the remainder of the purified water, the water-soluble components were dissolved (i.e., phase C). To the ethanol, POE (20) octyl dodecanol and the pantotenyl ethyl ether were dissolved and then the above phase C was added thereto and mixed with stirring, then the above phase A was mixed therewith with mixing and thereafter the above phase B was added thereto and mixed with stirring in a homomixer.

### Example IV-41: Pack

| (A phase) | mass% |
|---|---|
| Dipropylene glycol | 5.0 |
| POE (60) Hydrogenated castor oil | 5.0 |

| (B phase) | mass% |
|---|---|
| Olive oil | 5.0 |
| Tocopherol acetate | 0.2 |
| Fragrance | 0.2 |

| (C phase) | mass% |
|---|---|
| Sodium bisulfite | 0.03 |
| Polyvinyl alcohol (Degree of saponification 90, Degree of polymerization 2000) | 13.0 |

| | |
|---|---|
| Ethanol | 5.0 |
| 3-Phenoxy-1,2-propanediol | 0.5 |
| 3-Benzyloxy-1,2-propanediol | 0.6 |
| Methyl paraben | 0.1 |
| Purified water | balance |

### (Preparation method)

The phase A, phase B and phase C were uniformly mixed respectively and then the phase B was added to the phase A to be solubilized. Thereafter, the phase C was added thereto, followed by mixing.

### Example IV-42: Pack

| (A phase) | mass% |
|---|---|
| Dipropylene glycol | 5.0 |
| POE (60) Hydrogenated castor oil | 5.0 |

| (B phase) | mass% |
|---|---|
| Olive oil | 5.0 |
| Tocopherol acetate | 0.2 |
| Fragrance | 0.2 |

| (C phase) | mass% |
|---|---|
| Sodium bisulfite | 0.03 |
| Polyvinyl alcohol | |
| (Degree of saponification 90, Degree of polymerization 2000) | 13.0 |
| Ethanol | 5.0 |
| 3-Benzyloxy-1,2-propanediol | 0.6 |
| 3-Phenoxy-1,2-propanediol | 0.3 |
| Phenoxyethanol | 0.1 |
| Methyl paraben | 0.1 |
| Purified water | balance |

### (Preparation method)

The phase A, phase B and phase C were uniformly mixed respectively and then the phase B was added to the phase A to be solubilized. Thereafter, the phase C was added thereto, followed by mixing.

### Example IV-43: Solid powdery foundation

| | mass% |
|---|---|
| (1) Talc | 15.0 |
| (2) Cericite | 10.0 |
| (3) Spherical nylon powder | 10.0 |
| (4) Porous anhydrous silicic acid powder | 15.0 |
| (5) Boron nitride | 5.0 |
| (6) Titanium dioxide | 5.0 |
| (7) Iron oxide | 3.0 |
| (8) Zinc stearate | 5.0 |
| (9) Liquid paraffin | balance |
| (10) Glycerol triisooctanoate | 15.0 |
| (11) Sorbitan sesquioleate | 1.5 |
| (12) 3-Phenoxy-1,2-propanediol | 1.0 |
| (13) 3-Benzyloxy-1,2-propanediol | 1.0 |
| (14) Methyl paraben | 0.2 |
| (15) Fragrance | q.s. |

### (Preparation method)

The components (1)-(8) were mixed and ground and a mixture of the components (9)-(15) was added thereto, followed by mixing with stirring, and the resultant mixture was molded in a container to obtain a solid foundation.

### Example IV-44: Oil-in-water type emulsified foundation

| | mass% |
|---|---|
| (1) Spherical nylon | 10.0 |
| (2) Porous anhydrous silicic acid powder | 8.0 |
| (3) Mica titanium | 2.0 |
| (4) Silicone-treated cericite | 2.0 |
| (5) Silicone-treated mica | 12.0 |
| (6) Silicone-treated titanium dioxide | 5.0 |
| (7) Silicone-treated iron oxide | 2.0 |
| (8) Purified water | balance |
| (9) 3-Phenoxy-1,2-propanediol | 3.0 |
| (10) 3-Benzyloxy-1,2-propanediol | 3.0 |
| (11) Decamethylcyclopentane siloxane | 18.0 |
| (12) Dimethyl polysiloxane | 5.0 |
| (13) Squalane | 1.0 |
| (14) Polyoxyethylene modified dimethylpolysiloxane | 2.0 |
| (15) Methyl paraben | 0.2 |
| (16) Phenoxyethanol | 0.1 |
| (17) Fragrance | q.s. |

### (Preparation method)

The components (9)-(17) were uniformly mixed and dissolved and, then, the mixed and ground components (1)-(7) were added and dispersed therein. To the dispersion thus obtained, the component (8) was added and emulsified, followed by filling in a container to obtain the oil-in-water type emulsified foundation.

### Example IV-45: Oil-in-water type emulsified foundation

| | mass% |
|---|---|
| (1) Spherical nylon | 10.0 |
| (2) Porous anhydrous silicic acid powder | 8.0 |
| (3) Mica titanium | 2.0 |
| (4) Silicone-treated cericite | 2.0 |
| (5) Silicone-treated mica | 12.0 |
| (6) Silicone-treated titanium dioxide | 5.0 |
| (7) Silicone-treated iron oxide | 2.0 |
| (8) Purified water | balance |
| (9) 3-Phenoxy-1,2-propanediol | 3.0 |
| (10) 3-Benzyloxy-1,2-propanediol | 3.0 |
| (11) Ethylhexanediol | 1.0 |
| (12) 2,2-Dimethylol pentane | 1.0 |
| (13) 2,2-Diethyl-1,3-propanediol | 1.0 |
| (14) 2,2-Dimethyl-1,3-propanediol | 0.2 |

| | |
|---|---|
| (15) 2,2,4-Trimethyl-1,3-pentanediol | 1.0 |
| (16) Decamethylcyclopentane siloxane | 18.0 |
| (17) Dimethyl polysiloxane | 5.0 |
| (18) Squalane | 1.0 |
| (19) Polyoxyethylene modified dimethylpolysiloxane | 2.0 |
| (20) Phenoxyethanol | 0.1 |
| (21) Methyl paraben | 0.1 |
| (22) Fragrance | q.s. |

### (Preparation method)

The components (9)-(22) were uniformly mixed and dissolved and, then, the mixed and ground components (1)-(7) were added and dispersed therein. To the dispersion thus obtained, the component (8) was added and emulsified, followed by filling in a container to obtain the oil-in-water type emulsified foundation.

### Example IV-46: Face powder

| | mass% |
|---|---|
| (1) Talc | balance |
| (2) Cericite | 10.0 |
| (3) Spherical nylon powder | 10.0 |
| (4) Boron nitride | 5.0 |
| (5) Iron oxide | 3.0 |
| (6) Magnesium carbonate | 5.0 |
| (7) Squalane | 3.0 |
| (8) Glycerol triisooctanoate | 2.0 |
| (9) Sorbitan sesquioleate | 2.0 |
| (10) 3-Phenoxy-1,2-propanediol | 3.0 |
| (11) 3-Benzyloxy-1,2-propanediol | 2.0 |
| (12) Fragrance | q.s. |

### (Preparation method)

The components (1)-(6) were mixed and ground and the previously mixed components (7)-(12) were added thereto, followed by mixing with stirring, to obtain the face powder.

### Example IV-47: Face powder

| | mass% |
|---|---|
| (1) Talc | balance |
| (2) Cericite | 10.0 |
| (3) Spherical nylon powder | 10.0 |
| (4) Boron nitride | 5.0 |
| (5) Iron oxide | 3.0 |
| (6) Magnesium carbonate | 5.0 |
| (7) Squalane | 3.0 |
| (8) Glycerol triisooctanoate | 2.0 |
| (9) Sorbitan sesquioleate | 2.0 |
| (10) Ethylhexanediol | 0.5 |
| (11) 3-Phenoxy-1,2-propanediol | 1.0 |
| (12) 3-Benzyloxy-1,2-propanediol | 2.0 |
| (13) Methyl paraben | 0.1 |
| (14) Fragrance | q.s. |

### (Preparation method)

The components (1)-(6) were mixed and ground and the previously mixed components (7)-(14) were added thereto, followed by mixing with stirring, to obtain the face powder.

### Example IV-48: Eye shadow

| | mass% |
|---|---|
| (1) Talc | balance |
| (2) Mica | 15.0 |
| (3) Spherical nylon powder | 10.0 |
| (4) Boron nitride | 5.0 |
| (5) Iron oxide | 3.0 |
| (6) Titanium oxide-coated mica | 5.0 |
| (7) Squalane | 3.0 |
| (8) Glycerol triisooctanoate | 2.0 |
| (9) Sorbitan sesquioleate | 2.0 |
| (10) Ethylhexanediol | 1.0 |
| (11) 2,2-Dimethylol-1,3-pentanediol | 0.1 |
| (12) 2,2-Diethyl-1,3-pentanediol | 0.1 |
| (13) 2,2-Dimethyl-1,3-pentanediol | 0.1 |
| (14) 2,2,4-Trimethyl-1,3-propanediol | 0.1 |
| (15) 3-Phenoxy-1,2-propanediol | 0.5 |
| (16) 3-Benzyloxy-1,2-propanediol | 0.5 |
| (17) Methyl paraben | 0.1 |
| (18) Fragrance | q.s. |

### (Preparation method)

The components (1)-(6) were mixed and ground and the components (7)-(18) were added thereto, followed by mixing with stirring, to obtain the eyeshadow.

### Example IV-49: Lipstick

| | mass% |
|---|---|
| (1) Carnauba wax | 0.5 |
| (2) Candelilla wax | 5.0 |
| (3) Ceresine | 10.0 |
| (4) Squalane | balance |
| (5) Glycerol triisostearate | 10.0 |
| (6) Glycerol diisostearate | 20.0 |
| (7) 3-Phenoxy-1,2-propanediol | 0.2 |
| (8) 3-Benzyloxy-1,2-propanediol | 0.1 |
| (9) Ethylhexanediol | 0.2 |
| (10) Cholesteryl Macademia nut oil fatty acid | 4.0 |
| (11) Synthetic sodium-magnesium silicate | 0.5 |
| (12) Hydrophobic silica | 0.5 |
| (13) Purified water | 2.0 |
| (14) Coloring agent | q.s. |
| (15) Fragrance | q.s. |

### (Preparation method)

In the component (10) heated at 60°C, the components (11) and (12) were dispersed, and the component (11) was added thereto, followed by sufficiently stirring. The resultant mixture was added to a mixture of the components (1)-(9), which were separately dissolved by heating at 70°C, followed by sufficiently mixing, and then, the components (14) and (15) were added thereto, followed by dispersing with stirring. Thereafter, the dispersion was flown into a container, followed by cooling and molding to obtain the lipstick.

### Example IV-50: Hair foam

| (Stock solution formulation) | mass% |
|---|---|
| (1) Acrylic resin alkanol amine solution (50%) | 8.0 |
| (2) Polyoxyethylene hydrogenated castor oil | 1.0 |
| (3) Liquid paraffin | 5.0 |
| (4) Glycerol | 3.0 |
| (5) Fragrance | q.s. |
| (6) 3-Phenoxy-1,2-propanediol | 0.01 |
| (7) 3-Benzyloxy-1,2-propanediol | 0.01 |
| (8) Ethanol | 5.0 |
| (9) Purified water | balance |

| (Filling formulation) | mass% |
|---|---|
| (1) Stock solution | 90.0 |
| (2) Liquefied petroleum gas | 10.0 |

### (Preparation method)

The liquid paraffin was added to a dissolved mixture of the glycerol and the polyoxyethylene hydrogenated castor oil and uniformly emulsified in a homomixer. The emulsified product was added to a solution of the other components. The stock solution was filled in a can and, after a valve is attached, a gas is filled.

### Example IV-51: Shampoo

| | mass% |
|---|---|
| Sodium lauryl polyoxyethylene (3) sulfate ester (30% aqueous solution) | 30.0 |
| Sodium lauryl sulfate ester (30% aqueous solution) | 10.0 |
| Coconut oil fatty acid diethanol amide | 4.0 |
| Glycerol | 1.0 |
| 3-Phenoxy-1,2-propanediol | 3.0 |
| 3-Benzyloxy-1,2-propanediol | 3.0 |
| Sodium benzoate | 0.5 |
| Dye | q.s. |
| Fragrance | q.s. |
| Metal sequestering agent | q.s. |
| Purified water | balance |

### (Preparation method)

The purified water was heated to 70°C and the other components were added thereto, followed by uniformly dissolving and then cooling.

### Example IV-52: Rinse

| | mass% |
|---|---|
| Silicone oil | 3.0 |
| Liquid paraffin | 1.0 |
| Cetyl alcohol | 1.5 |
| Stearyl alcohol | 1.0 |
| Stearyl trimethylammonium chloride | 0.7 |
| 3-Phenoxy-1,2-propanediol | 5.0 |
| 3-Benzyloxy-1,2-propanediol | 5.0 |
| Glycerol | 3.0 |
| Dye | q.s. |
| Fragrance | q.s. |
| Purified water | balance |

### (Preparation method)

To the purified water, the stearyl trimethylammonium chloride, glycerol and dye were added and maintained at 70°C (i.e., an aqueous phase). The other components were mixed, followed by heating and dissolving and maintained at 70°C (i.e., an oil phase). The oil phase was added to the aqueous phase and emulsified in a homomixer, followed by cooling, with mixing.

### Example IV-53: Cream

| | mass% |
|---|---|
| Liquid paraffin | 10.0 |

| | |
|---|---|
| Dimethyl polysiloxane | 2.0 |
| Glycerol | 10.0 |
| 1,3-Butylene glycol | 2.0 |
| Erythritol | 1.0 |
| Ethylhexanediol | 0.1 |
| 2,2-Dimethylol propane | 1.0 |
| 2,2,4-Trimethyl-1,3-pentanediol | 0.5 |
| 2,2-Diethyl-1,3-propanediol | 0.1 |
| Polyethylene glycol 1500 | 5.0 |
| Squalane | 15.0 |
| Tetra 2-ethyl hexanoic acid pentaerythritol | 5.0 |
| Potassium hydroxide | 0.1 |
| Sodium hexametaphosphate | 0.05 |
| Tocopherol acetate | 0.05 |
| 3-Phenoxy-1,2-propanediol | 3.0 |
| 3-Benzyloxy-1,2-propanediol | 2.0 |
| Phenoxyethanol | 0.1 |
| Hydroxypropyl- methylcellulose | 0.3 |
| Polyvinyl alcohol | 0.1 |
| Carboxydivinyl polymer | 0.2 |
| Acrylic acid·methacrylic acid alkyl copolymer (Pemulene TR-2) | 0.1 |
| Purified water | balance |

### (Preparation method)

In a small amount of the purified water, carboxyvinyl polymer and acrylic acid alkyl methacrylate copolymer were dissolved (i.e., phase A). To the remainder of the purified water, the water-soluble components were added and dissolved upon heating at 70°C (i.e., an aqueous phase). The oil components were mixed to the liquid oil, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example IV-54: Cream

| | mass% |
|---|---|
| Vaseline | 2.0 |
| Dimethylpolysiloxane (6 mPa.s) | 2.0 |
| Ethanol | 5.0 |
| Behenyl alcohol | 0.5 |
| Batyl alcohol | 0.2 |
| Glycerol | 7.0 |
| 1,3-Butylene glycol | 5.0 |
| Ethylhexanediol | 1.0 |
| Polyethylene glycol 20000 | 0.5 |
| Jojoba oil | 3.0 |
| Squalane | 2.0 |
| Phytosteryl hydroxystearate | 0.5 |
| Pentaerythitol Tetra 2-ethyl hexanoate | 1.0 |
| Polyoxyethylene (60) hydrogenated castor oil | 1.0 |
| Potassium hydroxide | 0.1 |
| Sodium pyrosulfite | 0.01 |
| Sodium hexametaphosphate | 0.05 |
| Stearyl glycyrrhetiate | 0.1 |
| Pantothenyl ethyl ether | 0.1 |
| Albutin | 7.0 |
| Tranexamic acid | 1.0 |
| Tocopherol acetate | 0.1 |
| Sodium hyaluronate | 0.05 |
| 3-Phenoxy-1,2-propanediol | 3.0 |
| 3-Benzyloxy-1,2-propanediol | 3.0 |
| Trisodium edetate | 0.05 |
| 4-t-Butyl-4'-methoxydibenzoyl methane | 0.1 |
| Diparamethoxycinnamic acid mono-2-ethylhexane glyceryl | 0.1 |
| Yellow iron oxide | q.s. |
| Xanthan gum | 0.1 |
| Carboxyvinyl polymer | 0.2 |
| Purified water | balance |

### (Preparation method)

In a small amount of the purified water, carboxyvinyl polymer and xanthan gum were dissolved to form a solution (i.e., phase A). In the remainder of the purified water, the water-soluble components were dissolved upon heating at 70°C (i.e., an aqueous phase). In the liquid oil, the oily components were mixed followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example IV-55: Cream

| | mass% |
|---|---|
| Decamethylcyclopenta siloxane | 30.0 |
| Polyoxyethylene-methylpolysiloxane copolymer (M.W. 6000) | 1.5 |
| Trimethylsiloxy silicic acid | 0.5 |
| Glycerol | 2.0 |
| Dipropylene glycol | 5.0 |
| 3-Phenoxy-1,2-propanediol | 2.0 |
| 3-Benzyloxy-1,2-propanediol | 2.0 |
| Talc | 5.0 |
| Spherical anhydrous silicic acid | 0.5 |
| Dextrin palmitate-coaded titanium oxide fine powder (30 nm) | 7.0 |
| Spheric polyethylene powder | 2.0 |
| Poly(oxyethylene·oxypropylene)·methyl polysiloxane copolymer (M.W. 55000) | 1.0 |
| Phenoxyethanol | 0.2 |
| Methyl paraben | 0.1 |
| Trisodium edetate | 0.02 |
| Dimethyl distearyl ammonium hectolite | 0.5 |
| Purified water | balance |

### (Preparation method)

After dissolving the oily components upon heating, the polyoxyethylene·methyl polysiloxane copolymer, poly(oxyethylene·oxypropylene)·methyl polysiloxane copolymer, dimethyl distearyl ammonium hectrite and the other oily components were added thereto. The temperature of the mixture was adjusted to 70°C and uniformly dispersed and dissolved to obtain an oily gel. To the purified water, the glycerol, dipropylene glycol, 3-phenoxy-1,2-propanediol were added and the temperature was adjusted to 70°C. The resultant mixture was gradually added to the oily gel with stirring and, after uniformly mixing in a homomixer, the mixture was cooled to 30°C.

### Example IV-56: Emulsion

| | mass% |
|---|---|
| Liquid paraffin | 7.0 |
| Vaseline | 3.0 |
| Decamethylcyclopentane siloxane | 2.0 |
| Behenyl alcohol | 1.0 |
| Glycerol | 5.0 |
| Dipropylene glycol | 7.0 |
| Polyethylene glycol 1500 | 2.0 |
| 3-Phenoxy-1,2-propanediol | 2.0 |
| 3-Benzyloxy-1,2-propanediol | 2.0 |
| Jojoba oil | 1.0 |
| Isostearic acid | 0.5 |
| Stearic acid | 0.5 |
| Behenic acid | 0.5 |
| Tetra 2-ethyl hexanoic acid pentaerythitol | 3.0 |
| Cetyl 2-ethylhexanoate | 3.0 |
| Glyceryl monostearate | 1.0 |
| Polyoxyethylene glyceryl monostearate | 1.0 |
| Potassium hydroxide | 0.1 |
| Sodium hexametaphosphate | 0.05 |
| Stearyl glycyrrhetinate | 0.05 |
| L-Arginine | 0.1 |
| Royal gelly extract | 0.1 |
| Tocopherol acetate | 0.1 |
| Sodium acetylated hyaluronate | 0.1 |
| Trisodium edetate | 0.05 |
| 4-t-Butyl-4'-methoxydibenzoyl methane | 0.1 |
| 2-Ethylhexyl p-methoxycinnamate | 0.1 |
| Carboxyvinyl polymer | 0.15 |
| Purified water | balance |

### (Preparation method)

In a small amount of the purified water, carboxyvinyl polymer was dissolved (i.e., phase A). To the remainder of the purified water, the water-soluble components was dissolved upon heating and maintained at 70°C (i.e., an aqueous phase). The oily components were mixed to the liquid oil, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Usability Test and Preservability Test

The lotion having the formulation shown in Table V-1 was prepared according to the following preparation method and subjected to the usability and preservability (antisepsis) tests.

### Preparation of Lotion

To the purified water, 2,2-dimethyl-1-phenyl-1,3-propanediol was added for Examples V-1, V-2 and V-3, and, citric acid, trisodium citrate, trisodium edetate are dissolved thereto (i.e., an aqueous phase). Ethanol, glycerol, 1,3-butylene glycol, POE (60) hydrogenated castor oil, methyl paraben (only for Comparative Example V-3) are dissolved (i.e., an alcohol phase). The aqueous phase and the alcohol phase were mixed.

### Test Method for Usability

A panel consisting of 10 women having a sensitive skin, who had an irritation feeling when an external skin treatment composition containing paraben was used. The test was carried out in such a manner that each paneller used the above external skin treatment composition twice a day for 1 week and, based upon the satisfactory degree and the presence or absence of the skin irritation of each paneller, the usability is evaluated based upon the following 4-rank standards. The number of the persons having irritation feeling was also counted.
A: Number of paneller having good feeling is 8 or more
B: Number of paneller having good feeling is 5 to less than 8
C: Number of paneller having good feeling is 3 to less than 5
D: Number of paneller having good feeling is less than 3

### Test Method for Judging Preservability

To 30 ml of the samples of the Examples and Comparative Examples, microbials in a liquid were inoculated and then the change in the number of the microbial was checked after 2 weeks from the inoculation by a smear culture method. The inoculated microbials were as follows.
Mould: Aspergillus niger ATCC16404. Inoculation amount 10⁴ cfu (colony forming unit)/g
Yeast: Candida albicans ATCC10231. Inoculation amount 10⁵ cfu/g
Bacteria: Escherichia coli ATCC8739. Inoculation amount 10⁶ cfu/g; Staphylococcus aureus ATCC6538. Inoculation amount 10⁶ cfu/g; Pseudomonas aeruginosa ATCC15442. Inoculation amount 10⁶ cfu/g

The preservability (or antisepsis) was evaluated used upon the changes in the number of the microbials and the results were classified to the fallowing 4 ranks.
A: All of the mould, yeast and bacteria are decreased to 100 cfu/g or less within 1 week.
B: All of the mould, yeast and bacteria are decreased to 100 cfu/g or less within 2 weeks.
C: Either of the mould, yeast or bacteria remain in an amount of 100 cfu/g or more even after 2 weeks.
D: All of the mould, yeast and bacteria remain in an amount of 100 cfu/g or more even after 2 weeks.

As the preservability of an external skin treatment composition, those judged as "A" or "B" is acceptable. The results of the usability and preservability tests are shown in Table V-1.

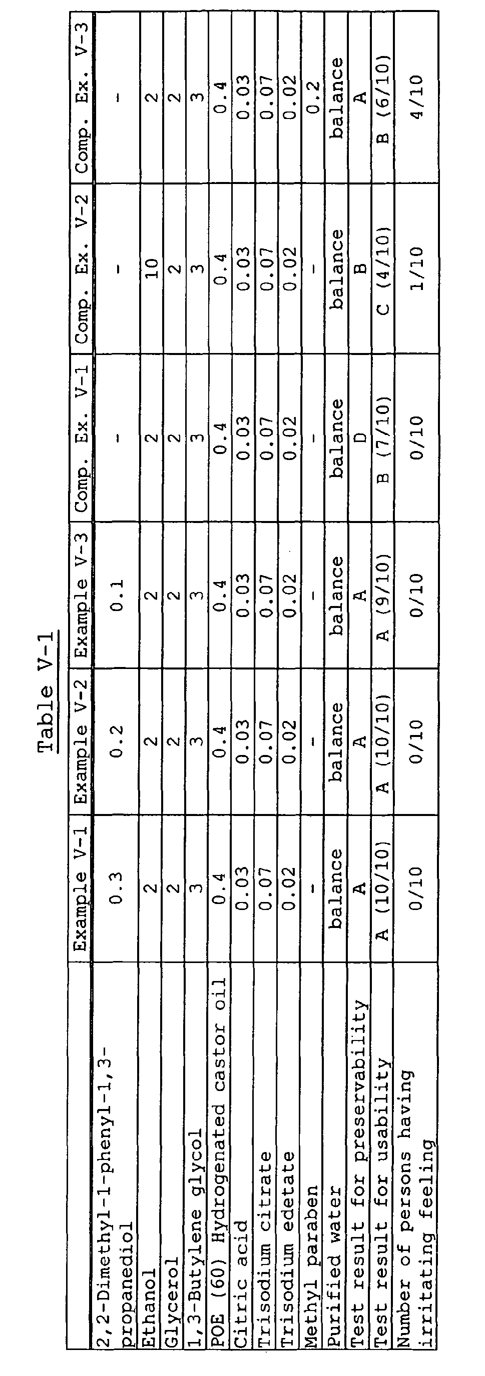

There were no persons having irritating feeling for Examples V-1 to V-3 and the ratio of the persons having satisfactory usability are large and thus the preservability was confirmed. This is the effect of the present invention. Also in Comparative Example V-1, a large number of the panellers are satisfactory in the usability and the skin irritation is small, but the preservability is poor. In Comparative Example V-2, there are no problems in the preservability, the satisfaction in the usability is low although the preservability is not problem, one paneller felt a skin irritation. Comparative Example V-3 was problem in the skin irritation because many panellers had skin irritation although the preservability is excellent.

Next, the emulsion having the formulation shown in Table V-2 was prepared according to the following preparation method and the usability and preservability tests were carried out in the same manner as mentioned above. The results of the usability and preservability tests are shown in Table V-2.

### Preparation of Emulsion

To the purified water, 2,2-dimethyl-1-phenyl-1,3-propanediol was added for Examples V-4, V-5 and V-6. To these, 1,3-butylene glycol, polyethylene glycol 1500, trisodium edetate, triethanol amine were added, followed by heating at 70°C to prepare an aqueous phase. Stearic acid cetyl alcohol, vaseline, squalane were dissolved and sorbitan monooleic acid ester and methyl paraben (only for Comparative Example V-5) were added thereto, followed by heating at 70°C to prepare an oil phase. The oil phase was added to the aqueous phase to the pre-emulsified and then uniformly emulsified in a homomixer followed by cooling.

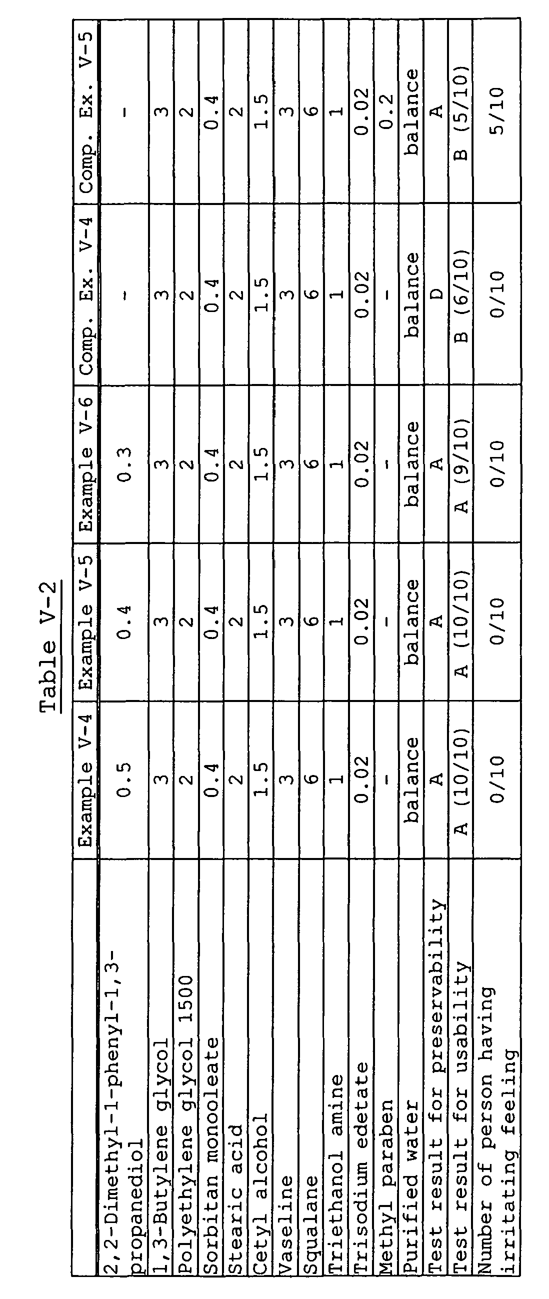

There were no persons having irritating feeling for Examples V-4 to V-6 and the ratio of the persons having satisfactory usability are large and thus the preservability was confirmed. This is the effect of the present invention. Also in Comparative Example V-4, a large number of the panellers are satisfactory in the usability and the skin irritation is small, but the preservability is poor. Comparative Example V-5 was problem in the skin irritation because many panellers had skin irritation although the usability and preservability are excellent.

Next, the emulsion having the formulation shown in Table V-3 was prepared according to the following preparation method and the usability and preservability tests were carried out in the same manner as mentioned above. The results of the usability and preservability tests are shown in Table V-3.

### Preparation of Cream

To the purified water, 2,2-dimethyl-1-phenyl-1,3-propanediol was added for Examples V-7, V-8 and V-9. To these, 1,3-butylene glycol, propylene glycol were added, followed by heating at 70°C to prepare an aqueous phase. Stearyl alcohol, stearyl alcohol, hydrogenated lanolin, squalane, octyl dodecanol were dissolved and POE (25) cetyl alcohol ether, glycerol monostearate and methyl paraben (only for Comparative Example V-7) were added thereto, followed by heating at 70°C to prepare an oil phase. The oil phase was added to the aqueous phase and uniformly emulsified in a homomixer followed by cooling.

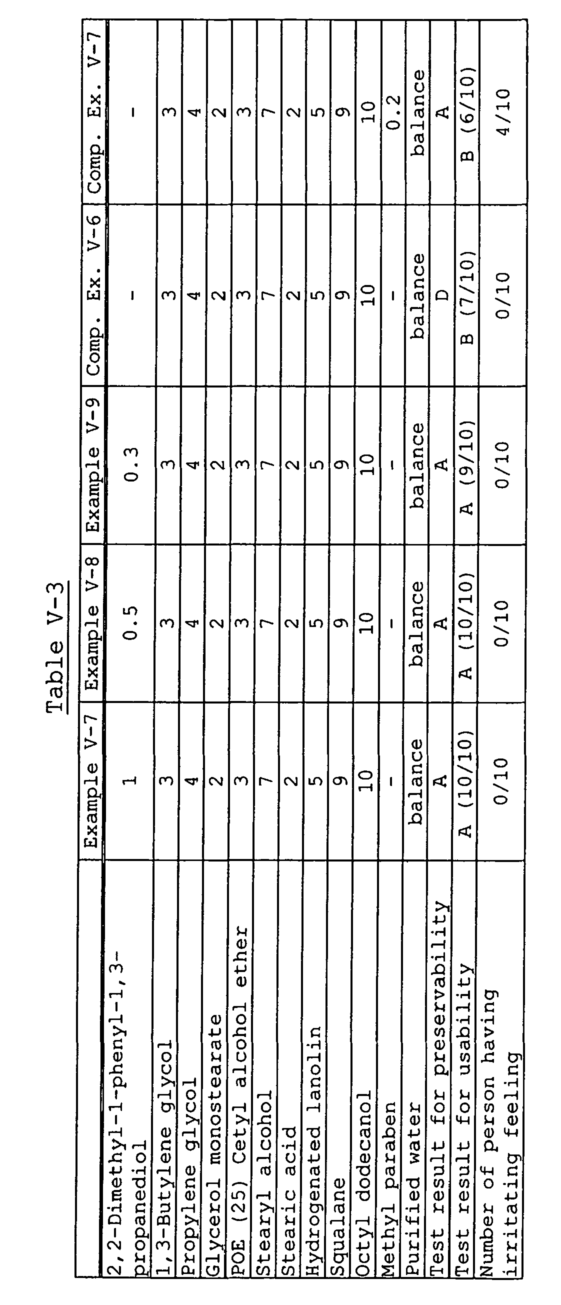

There were no persons having irritating feeling for Examples V-7 to V-9 and the ratio of the persons having satisfactory usability are large and thus the preservability was confirmed. This is the effect of the present invention. Also in Comparative Example V-6, a large number of the panellers are satisfactory in the usability and the skin irritation is small, but the preservability is poor. Comparative Example V-7 was problem in the skin irritation because many panellers had skin irritation although the usability and preservability are excellent.

Various external skin treatment composition will now be explained and all Examples have no skin irritation and good usability, while maintaining the preservability.

### Example V-10: Lotion

| (Alcohol phase) | mass% |
|---|---|
| Ethanol | 5.0 |
| Oleyl alcohol | 0.2 |
| 2,2,4-Trimethyl-1,3-pentanediol | 0.5 |
| 2,2-Dimethylol pentane | 0.1 |
| Ethylhexanediol | 0.3 |
| 2,2-Diethyl-1,3-propanediol | 0.1 |
| 2,2-Dimethyl-1,3-propanediol | 0.1 |
| 2,2-Dimethyl-1-phenyl-1,3-propanediol | 0.3 |
| 3-Hexyne-2,5-diol | 0.1 |
| 4,5-Dimorpholino-3-hydroxypyridazine | 0.1 |
| Phenoxyethanol | 0.3 |
| Methyl paraben | 0.1 |
| Fragrance | q.s. |

| (Aqueous phase) | mass% |
|---|---|
| 1,3-Butylene glycol | 6.0 |
| 1,2-Pentanediol | 2.0 |
| 3-Phenoxy-1,2-propanediol | 1.0 |
| 3-Benzyloxy-1,2-propanediol | 1.0 |
| Glycerol | 5.0 |
| Purified water | balance |

### (Preparation method)

The aqueous phase and the alcohol phase were mixed after the preparation of the aqueous phase and the alcohol phase.

### Example V-11: Lotion

| (Alcohol phase) | mass% |
|---|---|
| Ethanol | 5.0 |
| POE (20) Oleyl ether | 0.5 |
| 2,2-Dimethylol pentane | 0.3 |
| Ethylhexanediol | 0.2 |
| 2,2,4-Trimethyl-1,3-pentanediol | 0.1 |
| 2,2-Diethyl-1,3-propanediol | 0.1 |
| 2,2-Dimethyl-1,3-propanediol | 0.1 |
| 2,2-Dimethyl-1-phenyl-1,3-propanediol | 0.3 |
| 3-Hexyne-2,5-diol | 0.1 |
| Phenoxyethanol | 0.1 |
| Methyl paraben | 0.1 |
| Fragrance | q.s. |

| (Aqueous phase) | mass% |
|---|---|
| Dipropylene glycol | 6.0 |
| 3-Benzyloxy-1,2-propanediol | 0.5 |
| 3-Phenoxy-1,2-propanediol | 0.5 |
| Sorbitol | 4.0 |
| PEG1500 | 5.0 |
| Methyl cellulose | 0.2 |
| Quince Seed | 0.1 |
| Purified water | balance |

### (Preparation method)

To a portion of the purified water, methyl cellulose and Quince Seed were mixed, followed by stirring to prepare a viscous liquid. The remainder of the purified water and the other components of the aqueous phase were mixed and dissolved, and the above viscous liquid were added to obtain a uniform aqueous phase. The alcohol phase was prepared and then added to the aqueous phase, followed by mixing.

### Example V-12: Cream

| | mass% |
|---|---|
| Stearic acid | 5.0 |
| Stearyl alcohol | 4.0 |
| Isopropyl myristate | 18.0 |
| Glycerol monostearate | 3.0 |
| Propylene glycol | 10.0 |
| 1,2-Hexane diol | 1.0 |
| Ethylhexanediol | 2.0 |
| 2,2,4-Trimethyl-1,3-pentanediol | 1.0 |
| 2,2-Dimethylol pentane | 1.0 |
| 2,2-Dimethyl-1-phenyl-1,3-propanediol | 2.0 |
| 2,2-Diethyl-1,3-propanediol | 0.1 |
| 3-Phenoxy-1,2-propanediol | 1.0 |
| 3-Benzyloxy-1,2-propanediol | 3.0 |
| 3-Hexyne-2,5-diol | 0.1 |
| Potassium hydroxide | 0.2 |
| Phenoxyethanol | 0.3 |
| Methyl paraben | 0.1 |
| Sodium bisulfite | 0.01 |
| Fragrance | q.s. |
| Purified water | balance |

### (Preparation method)

In the purified water, propylene glycol and potassium hydroxide were added to be dissolved, followed by heating and maintained at 70°C (i.e., an aqueous phase). The other components were mixed, followed by heating and melting and maintained at 70°C (i.e., an oil phase). The oil phase was gradually added to the aqueous phase to be pre-emulsified and uniformly emulsified in a homomixer, followed by cooling to 30°C, with mixing.

### Example V-13: Cream

| | mass% |
|---|---|
| Stearic acid | 6.0 |
| Sorbitan monostearate | 2.0 |
| POE (20) Sorbitan monostearate | 1.5 |
| Ethylhexanediol | 2.0 |
| 2,2-Diethyl-1,3-propanediol | 1.0 |
| 2,2-Dimethylol pentane | 1.0 |
| 2,2,4-Trimethyl-1,3-pentanediol | 1.0 |
| Propylene glycol | 10.0 |
| 2,2-Dimethyl-1-phenyl-1,3-propanediol | 3.0 |
| 3-Benzyloxy-1,2-propanediol | 3.0 |
| 3-Phenoxy-1,2-propanediol | 1.0 |
| 3-Hexyne-2,5-diol | 0.1 |
| Glycerol trioctanoate | 10.0 |
| Squalane | 5.0 |
| Sodium bisulfite | 0.01 |
| Methyl paraben | 0.01 |
| Phenoxyethanol | 0.1 |
| Fragrance | q.s. |
| Purified water | balance |

### (Preparation method)

In the purified water, propylene glycol was added to be dissolved, followed by heating and maintained at 70°C (i.e., an aqueous phase). The other components were mixed, followed by heating and melting and maintained at 70°C (i.e., an oil phase). The oil phase was gradually added to the aqueous phase to be pre-emulsified and uniformly emulsified in a homomixer, followed by cooling to 30°C, with mixing.

### Example V-14: Emulsion

| | mass% |
|---|---|
| Stearic acid | 2.5 |
| Cetyl alcohol | 1.5 |
| Ethylhexanediol | 1.0 |
| 2,2,4-Trimethyl-1,3-propanediol | 0.5 |
| 2,2-Dimethylol pentane | 0.3 |
| 2,2-Diethyl-1,3-propanediol | 0.2 |
| 2,2-Dimethyl-1-phenyl-1,3-propanediol | 1.5 |
| 3-Hexyne-2,5-diol | 0.2 |
| Vaseline | 5.0 |
| Liquid paraffin | 10.0 |
| POE (10) Monooleate | 2.0 |
| PEG1500 | 3.0 |
| Triethanolamine | 1.0 |
| 3-Phenoxy-1,2-propanediol | 2.0 |
| 3-Benzyloxy-1,2-propanediol | 1.0 |
| Phenoxyethanol | 0.2 |
| Methyl paraben | 0.1 |
| Sodium bisulfite | 0.01 |
| Carboxyvinyl polymer | 0.05 |
| Fragrance | q.s. |
| Purified water | balance |

### (Preparation method)

In a small amount of the purified water, carboxyvinyl polymer was dissolved (i.e., phase A). To the remainder of the purified water, PEG 1500 and triethanolamine were added and dissolved upon heating at 70°C (i.e., an aqueous phase). The other components were mixed, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example V-15: Gel

| | mass% |
|---|---|
| 95% Ethanol | 5.0 |
| Dipropylene glycol | 15.0 |
| 1,2-Octane diol | 2.0 |
| Ethylhexanediol | 1.0 |
| 2,2-Dimethylol pentane | 0.1 |
| 2,2-Dimethyl-1,3-propanediol | 0.1 |
| 2,2-Diethyl-1,3-propanediol | 0.1 |
| 2,2,4-Trimethyl-1,3-propanediol | 0.05 |
| POE (50) Oleyl ether | 2.0 |
| Carboxyvinyl polymer | 1.0 |
| Sodium hydroxide | 0.15 |
| 3-Benzyloxy-1,2-propanediol | 0.05 |
| 3-Phenoxy-1,2-propanediol | 0.05 |
| 2,2-Dimethyl-1-phenyl-1,3-propanediol | 0.3 |
| 3-Hexyne-2,5-diol | 0.1 |
| Phenoxyethanol | 0.1 |
| Methyl paraben | 0.1 |
| Fragrance | q.s. |
| Purified water | balance |

### (Preparation method)

To the purified water, the carboxyvinyl polymer was uniformly dissolved (i.e., phase A). The POE (50) oleyl ether was dissolved in the 95% ethanol and then added to the phase A. After the components other than the sodium hydroxide was added, the sodium hydroxide was added thereto to be neutralized and thickened.

### Example V-16: Beauty liquid

| | mass% |
|---|---|
| 95% Ethanol | 5.0 |
| POE (20) Octyldodecanol | 1.0 |
| Pantonyl ethyl ether | 0.1 |
| Ethylhexanediol | 0.1 |
| 2,2-Dimethylol pentane | 0.1 |
| 2,2-Dimethyl-1,3-propanediol | 0.1 |
| 2,2-Diethyl-1,3-propanediol | 0.1 |
| 2,2,4-Trimethyl-1,3-propanediol | 0.05 |
| 2,2-Dimethyl-1-phenyl-1,3-propanediol | 0.3 |
| Potassium hydroxide | 0.1 |
| Glycerol | 5.0 |
| Dipropylene glycol | 10.0 |
| Sodium bisulfite | 0.03 |
| Carboxyvinyl polymer | 0.2 |
| 3-Phenoxy-1,2-propanediol | 0.2 |
| 3-Benzyloxy-1,3-propanediol | 0.1 |
| Phenoxyethanol | 0.3 |
| Purified water | balance |

### (Preparation method)

In a portion of the purified water, the carboxyvinyl polymer was dissolved (i.e., phase A). Similarly, a portion of the purified water, the potassium hydroxide was dissolved (i.e., phase B). In the remainder of the purified water, the water-soluble components were dissolved (i.e., phase C). To the ethanol, POE (20) octyl dodecanol and the pantotenyl ethyl ether were dissolved and then the above phase C was added thereto and mixed with stirring, then the above phase A was mixed therewith with mixing and thereafter the above phase B was added thereto and mixed with stirring in a homomixer.

### Example V-17: Pack

| (A phase) | mass% |
|---|---|
| Dipropylene glycol | 5.0 |
| POE (60) Hydrogenated castor oil | 5.0 |

| (B phase) | mass% |
|---|---|
| Olive oil | 5.0 |
| Ethylhexanediol | 0.1 |
| 2,2,4-Trimethyl-1,3-pentanediol | 0.1 |
| 2,2-Diethyl-1,3-propanediol | 0.2 |
| 2,2-Dimethylol pentane | 0.05 |
| Tocopherol acetate | 0.2 |
| Fragrance | 0.2 |

| (C phase) | mass% |
|---|---|
| Sodium bisulfite | 0.03 |
| Polyvinyl alcohol (Degree of saponification 90, Degree of polymerization 2000) | 13.0 |
| Ethanol | 5.0 |
| 2,2-Dimethyl-1-phenyl-1,3-propanediol | 0.3 |
| 3-Phenoxy-1,2-propanediol | 0.5 |
| 3-Benzyloxy-1,2-propanediol | 0.6 |
| 3-Hexyne-2,5-diol | 0.1 |
| Methyl paraben | 0.1 |
| Phenoxyethanol | 0.2 |
| Purified water | balance |

### (Preparation method)

The phase A, phase B and phase C were uniformly mixed respectively and then the phase B was added to the phase A to be solubilized. Thereafter, the phase C was added thereto, followed by mixing.

### Example V-18: Solid powdery foundation

| | mass% |
|---|---|
| (1) Talc | 15.0 |
| (2) Cericite | 10.0 |
| (3) Spherical nylon powder | 10.0 |
| (4) Porous anhydrous silicic acid powder | 15.0 |
| (5) Boron nitride | 5.0 |
| (6) Titanium dioxide | 5.0 |
| (7) Iron oxide | 3.0 |
| (8) Zinc stearate | 5.0 |
| (9). Liquid paraffin | balance |
| (10) Glycerol triisooctanoate | 15.0 |
| (11) Sorbitan sesquioleate | 1.5 |
| (12) 3-Phenoxy-1,2-propanediol | 0.5 |
| (13) 3-Benzyloxy-1,2-propanediol | 0.3 |
| (14) 2,2-Dimethyl-1-phenyl-1,3-propanediol | 0.1 |
| (15) Ethylhexanediol | 0.2 |
| (16) 2,2,4-Trimethyl-1,3-pentanediol | 0.1 |
| (17) 2,2-Diethyl-1,3-propanediol | 0.2 |
| (18) 2,2-Dimethylol pentane | 0.05 |
| (19) 3-Hexyne-2,5-diol | 0.1 |
| (20) Methyl paraben | 0.2 |
| (21) Fragrance | q.s. |

### (Preparation method)

The components (1)-(8) were mixed and ground and a mixture of the components (9)-(21) was added thereto, followed by mixing with stirring, and the resultant mixture was molded in a container to obtain a solid foundation.

### Example V-19: Oil-in-water type emulsified foundation

| | mass% |
|---|---|
| (1) Spherical nylon | 10.0 |
| (2) Porous anhydrous silicic acid powder | 8.0 |
| (3) Mica titanium | 2.0 |
| (4) Silicone-treated cericite | 2.0 |
| (5) Silicone-treated mica | 12.0 |
| (6) Silicone-treated titanium dioxide | 5.0 |
| (7) Silicone-treated iron oxide | 2.0 |
| (8) Purified water | balance |
| (9) 3-Phenoxy-1,2-propanediol | 3.0 |
| (10) 3-Benzyloxy-1,2-propanediol | 3.0 |
| (11) Ethylhexanediol | 1.0 |
| (12) 2,2-Dimethylol pentane | 1.0 |
| (13) 2,2-Diethyl-1,3-propanediol | 1.0 |
| (14) 2,2-Dimethyl-1,3-propanediol | 0.2 |
| (15) 2,2,4-Trimethyl-1,3-pentanediol | 1.0 |
| (16) 2,2-Dimethyl-1-phenyl-1,3-propanediol | 0.7 |
| (17) 3-Hexyne-2,5-diol | 0.1 |
| (18) Decamethylcyclopentane siloxane | 18.0 |
| (19) Dimethyl polysiloxane | 5.0 |
| (20) Squalane | 1.0 |
| (21) Polyoxyethylene modified dimethylpolysiloxane | 2.0 |
| (22) Phenoxyethanol | 0.1 |
| (23) Methyl paraben | 0.1 |
| (24) Fragrance | q.s. |

### (Preparation method)

The components (9)-(24) were uniformly mixed and dissolved and, then, the mixed and ground components (1)-(7) were added and dispersed therein. To the dispersion thus obtained, the component (8) was added and emulsified, followed by filling in a container to obtain the oil-in-water type emulsified foundation.

### Example V-20: Face powder

| | mass% |
|---|---|
| (1) Talc | balance |
| (2) Cericite | 10.0 |
| (3) Spherical nylon powder | 10.0 |
| (4) Boron nitride | 5.0 |
| (5) Iron oxide | 3.0 |
| (6) Magnesium carbonate | 5.0 |
| (7) Squalane | 3.0 |
| (8) Glycerol triisooctanoate | 2.0 |
| (9) Sorbitan sesquioleate | 2.0 |
| (10) Ethylhexanediol | 0.5 |
| (11) 3-Phenoxy-1,2-propanediol | 1.0 |
| (12) 3-Benzyloxy-1,2-propanediol | 2.0 |
| (13) Ethylhexanediol | 1.0 |
| (14) 2,2-Dimethylol pentane | 1.0 |
| (15) 2,2-Diethyl-1,3-propanediol | 1.0 |
| (16) 2,2-Dimethyl-1,3-propanediol | 0.2 |
| (17) 2,2,4-Trimethyl-1,3-pentanediol | 1.0 |
| (18) 2,2-Dimethyl-1-phenyl-1,3-propanediol | 0.7 |
| (19) 3-Hexyne-2,5-diol | 0.1 |
| (20) Methyl paraben | 0.3 |
| (21) Fragrance | q.s. |

### (Preparation method)

The components (1)-(6) were mixed and ground and the previously mixed components (7)-(21) were added thereto, followed by mixing with stirring, to obtain the face powder.

### Example V-21: Eye shadow

| | mass% |
|---|---|
| (1) Talc | balance |
| (2) Mica | 15.0 |
| (3) Spherical nylon powder | 10.0 |
| (4) Boron nitride | 5.0 |
| (5) Iron oxide | 3.0 |
| (6) Titanium oxide-coated mica | 5.0 |
| (7) Squalane | 3.0 |
| (8) Glycerol triisooctanoate | 2.0 |
| (9) Sorbitan sesquioleate | 2.0 |
| (10) Ethylhexanediol | 1.0 |
| (11) 2,2-Dimethylol pentane | 0.3 |
| (12) 2,2-Diethyl-1,3-pentanediol | 0.1 |
| (13) 2,2-Dimethyl-1,3-pentanediol | 0.1 |
| (14) 2,2,4-Trimethyl-1,3-propanediol | 0.1 |
| (15) 3-Phenoxy-1,2-propanediol | 0.5 |
| (16) 3-Benzyloxy-1,2-propanediol | 0.5 |
| (17) 2,2-Dimethyl-1-phenyl-1,3-propanediol | 0.7 |
| (18) 3-Hexyne-2,5-diol | 0.3 |
| (19) Methyl paraben | 0.1 |
| (20) Fragrance | q.s. |

### (Preparation method)

The components (1)-(6) were mixed and ground and the components (7)-(20) were added thereto, followed by mixing with stirring, to obtain the eyeshadow.

### Example V-22: Lipstick

| | mass% |
|---|---|
| (1) Carnauba wax | 0.5 |
| (2) Candelilla wax | 5.0 |
| (3) Ceresine | 10.0 |
| (4) Squalane | balance |
| (5) Glycerol triisostearate | 10.0 |
| (6) Glycerol diisostearate | 20.0 |
| (7) 3-Phenoxy-1,2-propanediol | 0.2 |
| (8) 3-Benzyloxy-1,2-propanediol | 0.1 |
| (9) 2,2-Dimethyl-1-phenyl-1,3-propanediol | 0.3 |
| (10) Ethylhexanediol | 1.0 |
| (11) 2,2-Dimethylol pentane | 0.1 |
| (12) 2,2-Diethyl-1,3-pentanediol | 0.1 |
| (13) 2,2-Dimethyl-1,3-pentanediol | 0.1 |
| (14) 2,2,4-Trimethyl-1,3-propanediol | 0.1 |
| (15) 3-Hexyne-2,5-diol | 0.7 |
| (16) Cholesteryl Macademia nut oil fatty acid | 4.0 |
| (17) Synthetic sodium-magnesium silicate | 0.5 |
| (18) Hydrophobic silica | 0.5 |
| (19) Purified water | 2.0 |
| (20) Coloring agent | q.s. |
| (21) Fragrance | q.s. |

### (Preparation method)

In the component (16) heated at 60°C, the components (17) and (18) were dispersed, and the component (19) was added thereto, followed by sufficiently stirring. The resultant mixture was added to a mixture of the components (1)-(15), which were separately dissolved by heating at 70°C, followed by sufficiently mixing, and then, the components (20) and (21) were added thereto, followed by dispersing with stirring. Thereafter, the dispersion was flown into a container, followed by cooling and molding to obtain the lipstick.

### Example V-23: Hair foam

| (Stock solution formulation) | mass% |
|---|---|
| (1) Acrylic resin alkanol amine solution (50%) | 8.0 |
| (2) Polyoxyethylene hydrogenated castor oil | 1.0 |
| (3) Liquid paraffin | 5.0 |
| (4) Glycerol | 3.0 |
| (5) Fragrance | q.s. |
| (6) 3-Phenoxy-1,2-propanediol | 0.01 |
| (7) 3-Benzyloxy-1,2-propanediol | 0.01 |
| (9) 2,2-Dimethyl-1-phenyl-1,3-propanediol | 0.3 |
| (10) Ethylhexanediol | 0.2 |
| (11) 2,2-Dimethylol pentane | 0.1 |
| (12) 2,2-Diethyl-1,3-pentanediol | 0.1 |
| (13) 2,2-Dimethyl-1,3-pentanediol | 0.1 |
| (14) 2,2,4-Trimethyl-1,3-propanediol | 0.1 |
| (15) 3-Hexyne-2,5-diol | 0.3 |
| (16) Ethanol | 5.0 |
| (17) Purified water | balance |

| (Filling formulation) | mass% |
|---|---|
| (1) Stock solution | 90.0 |
| (2) Liquefied petroleum gas | 10.0 |

### (Preparation method)

The liquid paraffin was added to a dissolved mixture of the glycerol and the polyoxyethylene hydrogenated castor oil and uniformly emulsified in a homomixer. The emulsified product was added to a solution of the other components. The stock solution was filled in a can and, after a valve is attached, a gas is filled.

### Example V-24: Shampoo

| | mass% |
|---|---|
| Sodium lauryl polyoxyethylene (3) sulfate ester (30% aqueous solution) | 30.0 |
| Sodium lauryl sulfate ester (30% aqueous solution) | 10.0 |
| Coconut oil fatty acid diethanol amide | 4.0 |
| Glycerol | 1.0 |
| Ethylhexanediol | 0.2 |
| 2,2,4-Trimethyl-1,3-pentanediol | 0.2 |
| 2,2-Diethyl-1,3-propanediol | 0.1 |
| 2,2-Dimethylol pentane | 0.05 |
| 2,2-Dimethyl-1-phenyl-1,3-propanediol | 0.1 |
| 3-Phenoxy-1,2-propanediol | 1.0 |
| 3-Benzyloxy-1,2-propanediol | 1.0 |
| 3-Hexyne-2,5-diol | 0.5 |
| Sodium benzoate | 0.5 |
| Dye | q.s. |
| Fragrance | q.s. |
| Metal sequestering agent | q.s. |
| Purified water | balance |

### (Preparation method)

The purified water was heated to 70°C and the other components were added thereto, followed by uniformly dissolving and then cooling.

### Example V-25: Rinse

| | mass% |
|---|---|
| Silicone oil | 3.0 |
| Liquid paraffin | 1.0 |
| Cetyl alcohol | 1.5 |
| Stearyl alcohol | 1.0 |
| Stearyl trimethylammonium chloride | 0.7 |
| Ethylhexanediol | 0.3 |
| 2,2,4-Trimethyl-1,3-pentanediol | 0.1 |
| 2,2-Diethyl-1,3-propanediol | 0.1 |
| 2,2-Dimethylol pentane | 0.07 |
| 2,2-Dimethyl-1-phenyl-1,3-propanediol | 0.3 |
| 3-Phenoxy-1,2-propanediol | 1.0 |
| 3-Benzyloxy-1,2-propanediol | 2.0 |
| 3-Hexyne-2,5-diol | 0.7 |
| Glycerol | 3.0 |
| Dye | q.s. |
| Fragrance | q.s. |
| Purified water | balance |

### (Preparation method)

To the purified water, the stearyl trimethylammonium chloride, glycerol and dye were added and maintained at 70°C (i.e., an aqueous phase). The other components were mixed, followed by heating and dissolving and maintained at 70°C (i.e., an oil phase). The oil phase was added to the aqueous phase and emulsified in a homomixer, followed by cooling, with mixing.

### Example V-26: Cream

| | mass% |
|---|---|
| Liquid paraffin | 10.0 |
| Dimethyl polysiloxane | 2.0 |
| Glycerol | 10.0 |
| 1,3-Butylene glycol | 2.0 |
| Erythritol | 1.0 |
| Ethylhexanediol | 0.1 |
| 2,2-Dimethylol pentane | 1.0 |
| 2,2,4-Trimethyl-1,3-pentanediol | 0.5 |
| 2,2-Diethyl-1,3-propanediol | 0.1 |
| 2,2-Dimethyl-1-phenyl-1,3-propanediol | 2.0 |
| Polyethylene glycol 1500 | 5.0 |
| Squalane | 15.0 |
| Tetra 2-ethyl hexanoic acid pentaerythritol | 5.0 |
| Potassium hydroxide | 0.1 |
| Sodium hexametaphosphate | 0.05 |
| Tocopherol acetate | 0.05 |
| 3-Phenoxy-1,2-propanediol | 1.0 |
| 3-Benzyloxy-1,2-propanediol | 1.0 |
| 3-Hexyne-2,5-diol | 0.2 |
| Phenoxyethanol | 0.1 |
| Methyl paraben | 0.1 |
| Hydroxydipropyl methylcellulose | 0.3 |
| Polyvinyl alcohol | 0.1 |
| Carboxydivinyl polymer | 0.2 |
| Acrylic acid·methacrylic acid alkyl copolymer (Pemulene TR-2) | 0.1 |
| Purified water | balance |

### (Preparation method)

In a small amount of the purified water, carboxyvinyl polymer and acrylic acid alkyl methacrylate copolymer were dissolved (i.e., phase A). To the remainder of the purified water, water-soluble components were added and dissolved upon heating at 70°C (i.e., an aqueous phase). The oily components were mixed, in liquid oil, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example V-27: Cream

| | mass% |
|---|---|
| Vaseline | 2.0 |
| Dimethylpolysiloxane (6 mPa.s) | 2.0 |
| Ethanol | 5.0 |
| Behenyl alcohol | 0.5 |
| Batyl alcohol | 0.2 |
| Glycerol | 7.0 |
| 1,3-Butylene glycol | 5.0 |
| Polyethylene glycol 20000 | 0.5 |
| Jojoba oil | 3.0 |
| Squalane | 2.0 |
| Phytosteryl hydroxystearate | 0.5 |
| Pentaerythitol Tetra 2-ethyl hexanoate | 1.0 |
| Polyoxyethylene (60) hydrogenated castor oil | 1.0 |
| Potassium hydroxide | 0.1 |
| Sodium pyrosulfite | 0.01 |
| Sodium hexametaphosphate | 0.05 |
| Stearyl glycyrrhetiate | 0.1 |
| Pantothenyl ethyl ether | 0.1 |
| Albutin | 7.0 |
| Tranexamic acid | 1.0 |
| Tocopherol acetate | 0.1 |
| Sodium hyaluronate | 0.05 |
| 3-Phenoxy-1,2-propanediol | 1.0 |
| 3-Benzyloxy-1,2-propanediol | 1.0 |
| Trisodium edetate | 0.05 |
| Ethylhexanediol | 1.0 |
| 2,2-Dimethyl-1-phenyl-1,3-propanediol | 0.6 |
| 2,2-Dimethylol pentane | 0.2 |
| 2,2,4-Trimethyl-1,3-pentanediol | 0.1 |
| 2,2-Diethyl-1,3-propanediol | 0.2 |
| 3-Hexyne-2,5-diol | 0.2 |
| 4-t-Butyl-4'-methoxydibenzoyl methane | 0.1 |
| Diparamethoxycinnamic acid mono-2-ethylhexane glyceryl | 0.1 |
| Phenoxyethanol | 0.1 |
| Methyl paraben | 0.05 |
| Yellow iron oxide | q.s. |
| Xanthan gum | 0.1 |
| Carboxyvinyl polymer | 0.2 |
| Purified water | balance |

### (Preparation method)

In a small amount of the purified water, carboxyvinyl polymer and xanthan gum were dissolved to form a solution (i.e., phase A). In the remainder of the purified water, the water-soluble components were dissolved upon heating at 70°C (i.e., an aqueous phase). In the liquid oil, the oily components were mixed followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example V-28: Cream

| | mass% |
|---|---|
| Decamethylcyclopenta siloxane | 30.0 |
| Polyoxyethylene·methylpolysiloxane copolymer (M.W. 6000) | 1.5 |
| Trimethylsiloxy silicic acid | 0.5 |
| Glycerol | 2.0 |
| Ethylhexanediol | 1.0 |
| 2,2-Dimethyl-1-phenyl-1,3-propanediol | 0.7 |
| 2,2-Dimethylol pentane | 0.2 |
| 2,2,4-Trimethyl-1,3-pentanediol | 0.1 |
| 2,2-Diethyl-1,3-propanediol | 0.3 |
| Dipropylene glycol | 5.0 |
| 3-Phenoxy-1,2-propanediol | 0.5 |
| 3-Benzyloxy-1,2-propanediol | 1.0 |
| 3-Hexyne-2,5-diol | 0.5 |
| Talc | 5.0 |
| Spherical anhydrous silicic acid | 0.5 |
| Dextrin palmitate-coaded titanium oxide fine powder (30 nm) | 7.0 |
| Spheric polyethylene powder | 2.0 |
| Poly(oxyethylene-oxypropylene)-methyl polysiloxane copolymer (M.W. 55000) | 1.0 |
| Phenoxyethanol | 0.2 |
| Methyl paraben | 0.1 |
| Trisodium edetate | 0.02 |
| Dimethyl distearyl ammonium hectolite | 0.5 |
| Purified water | balance |

### (Preparation method)

After dissolving the oily components upon heating, the polyoxyethylene·methyl polysiloxane copolymer, poly(oxyethylene·oxypropylene)·methyl polysiloxane copolymer, dimethyl distearyl ammonium hectrite and the other oily components were added thereto. The temperature of the mixture was adjusted to 70°C and uniformly dispersed and dissolved to obtain an oily gel. To the purified water, the glycerol, dipropylene glycol and 3-phenoxy-1,2-propanediol were added and the temperature was adjusted to 70°C. The resultant mixture was gradually added to the oily gel with stirring and, after uniformly mixing in a homomixer, the mixture was cooled to 30°C.

### Example V-29: Emulsion

| | mass% |
|---|---|
| Liquid paraffin | 7.0 |
| Vaseline | 3.0 |
| Decamethylcyclopentane siloxane | 2.0 |
| Behenyl alcohol | 1.0 |
| Glycerol | 5.0 |
| Dipropylene glycol | 7.0 |
| Polyethylene glycol 1500 | 2.0 |
| Ethylhexanediol | 1.0 |
| 3-Hexyne-2,5-diol | 1.0 |
| 2,2-Dimethyl-1-phenyl-1,3-propanediol | 1.5 |
| 2,2-Dimethylol pentane | 0.2 |
| 2,2,4-Trimethyl-1,3-pentanediol | 0.1 |
| 2,2-Diethyl-1,3-propanediol | 0.2 |
| 3-Phenoxy-1,2-propanediol | 2.0 |
| 3-Benzyloxy-1,2-propanediol | 2.0 |
| Jojoba oil | 1.0 |
| Isostearic acid | 0.5 |
| Stearic acid | 0.5 |
| Behenic acid | 0.5 |
| Tetra 2-ethyl hexanoic acid pentaerythitol | 3.0 |
| Cetyl 2-ethylhexanoate | 3.0 |
| Glyceryl monostearate | 1.0 |
| Polyoxyethylene glyceryl monostearate | 1.0 |
| Potassium hydroxide | 0.1 |
| Sodium hexametaphosphate | 0.05 |
| Stearyl glycyrrhetinate | 0.05 |
| L-Arginine | 0.1 |
| Royal gelly extract | 0.1 |
| Tocopherol acetate | 0.1 |
| Sodium acetylated hyaluronate | 0.1 |
| Trisodium edetate | 0.05 |
| 4-t-Butyl-4'-methoxydibenzoyl methane | 0.1 |
| 2-Ethylhexyl p-methoxycinnamate | 0.1 |
| Carboxyvinyl polymer | 0.15 |
| Purified water | balance |

### (Preparation method)

In a small amount of the purified water, carboxyvinyl polymer was dissolved (i.e., phase A). To the remainder of the purified water, the water-soluble components was dissolved upon heating and maintained at 70°C (i.e., an aqueous phase). The oily components were mixed to the liquid oil, followed by heating and melting and maintained at 70°C (i.e., an oil phase). The oil phase was gradually added to the aqueous phase to be pre-emulsified and the phase A was added thereto and uniformly emulsified in a homomixer, followed by cooling to 30°C, with well mixing.

### Example V-1: Antimicrobial Effect

The minimum inhibiting concentrations (MIC) for various types of microbials were carried out for 2,2-dimethyl-1-phenyl-1,3-propanediol, methyl p-oxybenzoate.

Using the agar plate method, for bacteria, the following various bacteria were inoculated in SCD agar media (made by Eiken) containing 3-hexine-2,5-diol in different concentrations and cultured at 30°C for 24 hours. The concentrations of 3-hexine-2,5-diol not forming colonies (minimum inhibiting concentration: MIC) were found. Further, for fungi, the following various bacteria were inoculated in a potato dextrose agar media containing 3-hexine-2,5-diol in different concentrations and cultured at 25°C for 48 hours. The concentrations of 3-hexine-2,5-diol not forming colonies (minimum inhibiting concentration: MIC) were found. The same was conducted for methyl paraoxybenzoate methyl. The results of the judgment are shown in Table III-1 based on the following evaluation criteria:

### (Test Bacteria)

- Ps:: *Pseudomonas aeuginosa* (ATCC1542)
- E:: *Escherichia coli* (ATCC8739)
- S:: *Staphylococcus* aureus (ATCC6538)
- Can:: *Candida albicans* (ATCC10231)
- Asp:: *Aspergillus niger* (ATCC16404)

### (Evaluation Criteria)

A: Minimum inhibiting concentration of less than 1000 ppm
B: Minimum inhibiting concentration of 1000 ppm to less than 5000 ppm
C: Minimum inhibiting concentration of 5000 ppm to less than 10000 ppm
D: Minimum inhibiting concentration of 10000 ppm to less than 30000 ppm
E: Minimum inhibiting concentration or 30000 ppm or more

**Table V-1**

| Test bacteria | Antimicrobial effect | |
|---|---|---|
| | 2,2-Dimethyl-1-phenyl-1,3-propanediol | Methyl p-xybenzoate |
| Pseudomonas aeuginosa (ATCC15442) | A | C |
| Escherichia coli (ATCC8739) | A | B |
| Staphylococcus aureus (ATCC6538) | A | B |
| Candida albicans (ATCC10231) | A | B |
| Aspergillus niger (ATCC16404) | A | B |

### Example VI-2: Safety Test

The 2,2-dimethyl-1-phenyl-1,3-propanediol of the present invention was tested for safety. A single administration toxicity test was conducted, as a result, of which the toxicity was judged to be extremely weak. Further, a primary skin irritation test and a continuous skin irritation test were conducted, as a result, of which the skin irritability was judged to be extremely weak. Further, a skin sensitization test and a genetic toxicity test were conducted, as a result, of which negative results were obtained.

As explained above, the safety of the 2,2-dimethyl-1-phenyl-1,3-propanediol was good.

### Example V-1: Lotion

| | mass% |
|---|---|
| Ethanol | 5.0 |
| 1,3-Butylene glycol | 6.0 |
| Glycerol | 4.0 |
| Oleyl alcohol | 0.1 |
| POE (20) sorbitan monolaurate | 0.5 |
| POE (15) lauryl ether | 0.5 |
| 2,2-Dimethyl-1-phenyl-1,3-propanediol | 0.3 |
| Fragrance | q.s. |
| Purified water | balance |

### Example V-2: Lotion

| | mass% |
|---|---|
| Ethanol | 5.0 |
| 1,3-Butylene glycol | 6.0 |
| Glycerol | 4.0 |
| Oleyl alcohol | 0.1 |
| Ethylhexanediol | 0.3 |
| 2,2-Diethyl-1,3-propanediol | 0.3 |
| 2,2,4-Trimethyl-1,3-pentanediol | 0.5 |
| 2,2-Dimethylol pentane | 0.2 |
| POE (20) sorbitan monolaurate | 0.5 |
| POE (15) lauryl ether | 0.5 |
| 2,2-Dimethyl-1-phenyl-1,3-propanediol | 0.2 |
| 3-Benzyloxy-1,2-propanediol | 0.3 |
| 3-Phenoxy-1,2-propanediol | 0.2 |
| 3-Hexyne-2,5ϕϕεΥ-diol | 0.1 |
| Phenoxyethanol | 0.2 |
| Methyl paraben | 0.1 |
| Fragrance | q.s. |
| Purified water | balance |

### Example VI-3: Lotion

| | mass% |
|---|---|
| Sorbitol | 4.0 |
| 1,3-Butylene glycol | 6.0 |
| Glycerol | 2.0 |
| POE (20) oleyl alcohol ether | 0.5 |
| Methyl cellulose | 0.2 |
| Quince Seed | 0.1 |
| 2,2-Diethyl-1,3-propane diol | 0.1 |
| Ethylhexanediol | 0.3 |
| 2,2,4-Trimethyl-1,3-pentanediol | 0.2 |
| 2,2-Dimethylol pentane | 0.2 |
| 2,2-Dimethyl-1-phenyl-1,3-propanediol | 0.2 |
| 3-Benzyloxy-1,2-propanediol | 0.3 |
| 3-Phenoxy-1,2-propanediol | 0.2 |
| 3-Hexyne-2,5-diol | 0.1 |
| Phenoxyethanol | 0.2 |
| Methyl paraben | 0.2 |
| Fragrance | q.s. |
| Purified water | balance |

### Example VI-4: Emulsion

| | mass% |
|---|---|
| 2,2-Dimethyl-1-phenyl-1,3-propanediol | 2.0 |
| Glycerol | 3.0 |
| 2,2-Diethyl-1,3-propanediol | 0.2 |
| 2,2,4-Trimethyl-1,3-pentanediol | 0.5 |
| 2,2-Dimethylol pentane | 0.5 |
| Ethylhexanediol | 1.0 |
| Cetanol | 1.5 |
| Stearyl alcohol | 1.8 |
| Dimethyl polysiloxane (20 cs) | 1.5 |
| Squalane | 2.0 |
| Vaseline | 2.0 |
| Isopropyl myristate | 2.5 |
| Glyceryl monostearate | 1.8 |
| Polyoxyethylene (POE = 5) glyceryl monostearate | 1.8 |
| Polyoxyethylene (POE = 20) cetyl ether | 1.5 |
| Carboxyvinyl polymer | 0.25 |
| Potassium hydroxide | 0.05 |
| L-Arginine | 0.2 |
| Dipropylene glycol | 5.0 |
| 1,3-Butylene glycol | 3.0 |
| Trisodium edetate | 0.2 |
| 3-Benzyloxy-1,2-propanediol | 0.2 |
| 3-Phenoxy-1,2-propanediol | 0.1 |
| 3-Hexyne-2,5-diol | 0.1 |
| Phenoxyethanol | 0.1 |
| Methyl paraben | 0.01 |
| Purified water | balance |

### Example VI-5: Cream

| | mass% |
|---|---|
| 2,2-Dimethyl-1-phenyl-1,3-propanediol | 1.0 |
| 3-Benzyloxy-1,2-propanediol | 0.2 |
| 3-Phenoxy-1,2-propanediol | 0.1 |
| 2,2-Diethyl-1,3-propanediol | 0.2 |
| 2,2,4-Trimethyl-1,3-pentanediol | 0.5 |
| 2,2-Dimethylol pentane | 0.5 |
| Ethylhexanediol | 1.0 |
| 3-Hexyne-2,5-diol | 0.1 |
| Stearyl alcohol | 3.5 |
| Stearic acid | 2.0 |
| Squalane | 10.5 |
| Isopropyl myristate | 7.5 |
| Polyoxyethylene (POE = 25) cetyl alcohol ether | 3.0 |
| Glyceryl monostearate | 2.0 |
| Tocopherol acetate | 0.2 |
| Monoammonium glycyrrhetinate | 0.05 |
| Glycerol | 3.0 |
| Dipropylene glycol | 5.0 |
| 1,3-Butylene glycol | 3.0 |
| Phenoxyethanol | 0.2 |
| Trisodium edetate | 0.01 |
| Ethyl paraben | 0.1 |
| Purified water | balance |

### Example VI-6: Cleansing

| | mass% |
|---|---|
| 2,2-Dimethyl-1-phenyl-1,3-propanediol | 0.5 |
| 3-Benzyloxy-1,2-propanediol | 0.2 |
| 3-Phenoxy-1,2-propanediol | 0.1 |
| 2,2-Diethyl-1,3-propanediol | 0.2 |
| 2,2,4-Trimethyl-1,3-pentanediol | 0.5 |
| 2,2-Dimethylol pentane | 0.5 |
| Ethylhexanediol | 0.1 |
| 3-Hexyne-2,5-diol | 0.1 |
| Stearic acid | 8.0 |
| Palmitic acid | 6.0 |
| Myristic acid | 6.0 |
| Lauric acid | 4.0 |
| Potassium hydroxide | 5.2 |
| Glyceryl monostearate | 2.0 |
| Propylene glycol | 1.0 |
| Bees wax | 1.5 |
| Polyethylene glycol 1500 | 5.0 |
| Glycerol | 10.0 |
| Methyl paraben | 0.01 |
| Phenoxyethanol | 0.1 |
| Purified water | balance |

### Example VI-7: Shampoo

| | mass% |
|---|---|
| Lauryl polyoxyethylene (3) sulfate ester sodium salt (30% aqueous solution) | 25.0 |
| Lauryl sulfate ester sodium salt (30% aqueous solution) | 8.0 |
| Coconut oil fatty acid diethanolamide | 4.0 |
| Isoprene glycol | 4.0 |
| Sodium benzoate | 0.5 |
| Dipropylene glycol | 1.0 |
| 1,3-Butylene glycol | 1.0 |
| Trisodium edetate | 0.01 |
| 3-Benzyloxy-1,2-propanediol | 0.1 |
| 3-Phenoxy-1,2-propanediol | 0.1 |
| 2,2-Diethyl-1,3-propanediol | 0.2 |
| 2,2,4-Trimethyl-1,3-pentanediol | 0.5 |
| 2,2-Dimethylol pentane | 0.5 |
| Ethylhexanediol | 0.1 |
| 3-Hexyne-2,5-diol | 0.1 |
| 2,2-Dimethyl-1-phenyl-1,3-propanediol | 0.1 |
| Dye | q.s. |
| Fragrance | q.s. |
| Purified water | balance |

### Example VI-8: Gelly pack

| | mass% |
|---|---|
| 3-Benzyloxy-1,2-propanediol | 0.05 |
| 3-Phenoxy-1,2-propanediol | 0.1 |
| 2,2-Dimethyl-1-phenyl-1,3-propanediol | 0.1 |
| Polyoxyethylene oleyl alcohol ether | 0.5 |
| 2,2-diethyl-1,3-propanediol | 0.1 |
| Ethylhexanediol | 0.1 |
| 2,2,4-Trimethyl-1,3-propanediol | 0.1 |
| 2,2-Dimethylol pentane | 0.1 |
| 3-Hexyne-2,5-diol | 0.1 |
| Monoammonium glycyrrhizinate | 0.05 |
| Carboxymethyl cellulose | 5.0 |
| Ethanol | 12.0 |
| Polyvinyl alcohol | 12.0 |
| 1,3-Butylene glycol | 5.0 |
| Trisodium edetate | 0.01 |
| Purified water | balance |

### Example VI-9: Eyeliner

| | mass% |
|---|---|
| 2,2-Dimethyl-1-phenyl-1,3-propanediol | 1.0 |
| 3-Benzyloxy-1,2-propanediol | 0.2 |
| 3-Phenoxy-1,2-propanediol | 0.5 |
| Iron oxide (black) | 14.0 |
| Isopropyl myristate | 1.5 |
| Polyoxyethylene sorbitan monooleic ester | 1.0 |
| Vinylacetate resin emulsion | 45.0 |
| Monoammonium glycyrrhizinate | 0.05 |
| Carboxyvinyl polymer | 1.5 |
| Acetyltributyl citrate | 1.0 |
| Dipropylene glycol | 4.0 |
| Ethylhexanediol | 1.0 |
| 2,2-Diethyl-1,3-pentanediol | 1.0 |
| 2,2-Dimethyl-1,3-pentanediol | 1.0 |
| 2,2,4-Trimethyl-1,3-pentanediol | 0.5 |
| 2,2-Dimethylol pentane | 0.5 |
| 1,2-Pentanediol | 3.0 |
| 3-Hexyne-2,5-diol | 0.1 |
| Phenoxyethanol | 0.1 |
| Methyl paraben | 0.02 |
| Trisodium edetate | 0.01 |
| Purified water | balance |

### Example VI-10: Hair tonic

| | mass% |
|---|---|
| Hydrogenated castor oil ethyleneoxide | |
| (40 mol) addition product | 2.0 |
| Ethanol | 60.0 |
| Fragrance | q.s. |
| 2,2-Dimethyl-1-phenyl-1,3-propanediol | 0.01 |
| Purified water | balance |

### Example VI-11: Bath agent

| | mass% |
|---|---|
| Sodium bicarbonate | 60.0 |
| Anhydrous sodium sulfate | 35.0 |
| 2,2-Dimethyl-1-phenyl-1,3-propanediol | 0.5 |

### Example VI-12: Chinese noodle

| | mass% |
|---|---|
| Wheat flour | 98.0 |
| Table salt | 1.0 |
| Sweetner | 0.5 |
| 2,2-Dimethyl-1-phenyl-1,3-propanediol | 0.5 |

### Example VI-13: Noodle soup

| | mass% |
|---|---|
| Soysauce | 80.7 |
| Vinegar | 1.0 |
| Glucose | 15.0 |
| Sodium glutamate | 2.0 |
| Sugar | 1.0 |
| 2,2-Dimethyl-1-phenyl-1,3-propanediol | 0.3 |

### Example VI-14: Japanese noodle ("Soba")

| | mass% |
|---|---|
| Soba flour | 96.0 |
| Table salt | 0.9 |
| Water | 3.0 |
| 2,2-Dimethyl-1-phenyl-1,3-propanediol | 0.1 |

### Example VI-15: Bread

| | mass% |
|---|---|
| Wheat flour | 90.0 |
| Table salt | 1.2 |
| Sugar | 2.0 |
| Water | 6.5 |
| 2,2-Dimethyl-1-phenyl-1,3-propanediol | 0.3 |

### Example VI-16: Ham

| | mass% |
|---|---|
| Minced meat | 95.0 |
| Chicken egg | 4.0 |
| Table salt | 0.5 |
| Spice | 0.4 |
| 2,2-Dimethyl-1-phenyl-1,3-propanediol | 0.1 |

### Example VI-17: Fruitjuice beverage

| | mass% |
|---|---|
| Glucose liquid sugar | 13.0 |
| Orange fruit juice | 85.0 |
| Fragrance | 1.0 |
| 2,2-Dimethyl-1-phenyl-1,3-propanediol | 1.0 |

All the above Examples show the good preservability.

### Example VII-1: Antimicrobial Effect

The minimum inhibiting concentrations (MIC) for various types of microbials were found.

The following test was carried out for 2,4,7,9-tetramethyl-5-decyn-4,7-diol, methyl p-oxybenzoate.

Using the agar plate method, for bacteria, the following various bacteria were inoculated in SCD agar media (made by Eiken) containing 3-hexine-2,5-diol in different concentrations and cultured at 30°C for 24 hours. The concentrations of 3-hexine-2,5-diol not forming colonies (minimum inhibiting concentration: MIC) were found. Further, for fungi, the following various bacteria were inoculated in a potato dextrose agar media containing the test compound, in different concentrations and cultured at 25°C for 48 hours. The concentrations of the test compound, not forming colonies (minimum inhibiting concentration: MIC) were found. The same was conducted for methyl paraoxybenzoate methyl. The results of the judgment are shown in Table VII-1 based on the following evaluation criteria:

### (Test Bacteria)

- Ps:: *Pseudomonas aeuginosa* (ATCC1542)
- E:: *Escherichia coli* (ATCC8739)
- S:: *Staphylococcus aureus* (ATCC6538)
- Can:: *Candida albicans* (ATCC10231)
- Asp:: *Aspergillus niger* (ATCC16404)

### (Evaluation Criteria)

A: Minimum inhibiting concentration of less than 1000 ppm
B: Minimum inhibiting concentration of 1000 ppm to less than 5000 ppm
C: Minimum inhibiting concentration of 5000 ppm to less than 10000 ppm
D: Minimum inhibiting concentration of 10000 ppm to less than 30000 ppm
E: Minimum inhibiting concentration or 30000 ppm or more

**Table VII-1**

| Test bacteria | Antimicrobial effect | |
|---|---|---|
| | 2,4,7,9-Tetramethyl-5-decine-4,7-diol | Methyl paraoxybenzoate |
| *Pseudomonas aeuginosa* (ATCC15442) | A | C |
| *Escherichia coli* (ATCC8739) | A | B |
| *Staphylococcus aureus* (ATCC6538) | A | B |
| *Candida albicans* (ATCC10231) | A | B |
| *Aspergillus niger* (ATCC16404) | A | B |

### Example VII-2: Safety Test

The 2,4,7,9-tetramethyl-5-decyn-4,7-diol of the present invention was tested for safety. A single administration toxicity test was conducted, as a result, of which the toxicity was judged to be extremely weak. Further, a primary skin irritation test and a continuous skin irritation test were conducted, as a result, of which the skin irritability was judged to be extremely weak. Further, a skin sensitization test and a genetic toxicity test were conducted, as a result, of which negative results were obtained.

Thus, the 2,4,7,9-tetramethyl-5-decyn-4,7-diol of the present invention has a good safety.

### Example VII-1: Lotion

| | mass% |
|---|---|
| Ethanol | 5.0 |
| 1,3-Butylene glycol | 6.0 |
| Glycerol | 4.0 |
| Oleyl alcohol | 0.1 |
| POE (20) sorbitan monolaurate | 0.5 |
| POE (15) lauryl ether | 0.5 |
| 2,4,7,9-Tetramethyl-5-decyn-4,7-diol | 0.3 |
| Fragrance | q.s. |
| Purified water | balance |

### Example VII-2: Lotion

| | mass% |
|---|---|
| Ethanol | 5.0 |
| 1,3-Butylene glycol | 6.0 |
| Glycerol | 5.0 |
| Oleyl alcohol | 0.1 |
| Ethylhexanediol | 0.3 |
| 1,2-Pentanediol | 0.1 |
| 2,2-Diethyl-1,3-propanediol | 0.3 |
| 2,2,4-Trimethyl-1,3-pentanediol | 0.5 |
| 2,2-Dimethylol pentane | 0.2 |
| 2,2-Dimethyl-1-phenyl-1,3-propanediol | 0.3 |
| POE (20) sorbitan monolaurate | 0.5 |
| POE (15) lauryl ether | 0.5 |
| 2,4,7,9-Tetramethyl-5-decyn-4,7-diol | 0.2 |
| 3-Benzyloxy-1,2-propanediol | 0.3 |
| 3-Phenoxy-1,2-propanediol | 0.2 |
| 3-Hexyne-2,5-diol | 0.1 |
| Phenoxyethanol | 0.2 |
| Methyl paraben | 0.1 |
| Fragrance | q.s. |
| Purified water | balance |

### Example VII-3: Lotion

| | mass% |
|---|---|
| Sorbitol | 4.0 |
| 1,3-Butylene glycol | 6.0 |
| Glycerol | 2.0 |
| POE (20) oleyl alcohol ether | 0.5 |
| Methyl cellulose | 0.2 |
| Quince Seed | 0.1 |

| | |
|---|---|
| 2,2-Diethyl-1,3-propane diol | 0.1 |
| Ethylhexanediol | 0.3 |
| 1,2-Pentanediol | 0.1 |
| 2,2,4-Trimethyl-1,3-pentanediol | 0.2 |
| 2,2-Dimethylol pentane | 0.2 |
| 2,4,7,9-Tetramethyl-5-decyn-4,7-diol | 0.2 |
| 2,2-Dimethyl-1-phenyl-1,3-propanediol | 0.1 |
| 3-Benzyloxy-1,2-propanediol | 0.3 |
| 3-Phenoxy-1,2-propanediol | 0.2 |
| 3-Hexyne-2,5-diol | 0.1 |
| Phenoxyethanol | 0.2 |
| Methyl paraben | 0.2 |
| Fragrance | q.s. |
| Purified water | balance |

### Example VII-4: Lotion

| (Alcohol phase) | mass% |
|---|---|
| Ethanol | 5.0 |
| Oleyl alcohol | 0.2 |
| 2,2,4-Trimethyl-1,3-pentanediol | 0.5 |
| 2,2-Dimethylol pentane | 0.1 |
| Ethylhexanediol | 0.3 |
| 2,2-Dimethyl-1-phenyl-1,3-propanediol | 0.05 |
| 2,2-Diethyl-1,3-propanediol | 0.1 |
| 2,2-Dimethyl-1,3-propanediol | 0.1 |
| 2,4,7,9-Tetramethyl-5-decyn-4,7-diol | 0.3 |
| 3-Hexyne-2,5-diol | 0.1 |
| POE (20) Sorbitan monolaurate | 0.5 |
| POE (15) Lauryl ether | 0.5 |
| 4,5-Dimorpholino-3-hydroxypyridazine | 0.1 |
| Phenoxyethanol | 0.2 |
| Methyl paraben | 0.1 |
| Fragrance | q.s. |

| (Aqueous phase) | mass% |
|---|---|
| 1,3-Butylene glycol | 6.0 |
| 1,2-Pentanediol | 1.0 |
| 3-Phenoxy-1,2-propanediol | 1.0 |

| | |
|---|---|
| 3-Benzyloxy-1,2-propanediol | 1.0 |
| Glycerol | 5.0 |
| Purified water | balance |

### Example VII-5: Lotion

| (Alcohol phase) | mass% |
|---|---|
| Ethanol | 5.0 |
| POE (20) Oleyl ether | 0.5 |
| 2,2-Dimethylol pentane | 0.3 |
| Ethylhexanediol | 0.2 |
| 2,2,4-Trimethyl-1,3-pentanediol | 0.1 |
| 2,2-Diethyl-1,3-propanediol | 0.1 |
| 2,2-Dimethyl-1-phenyl-1,3-propanediol | 0.1 |
| 2,2-Dimethyl-1,3-propanediol | 0.1 |
| 2,4,7,9-Tetramethyl-5-decyn-4,7-diol | 0.3 |
| 3-Hexyne-2,5-diol | 0.1 |
| Phenoxyethanol | 0.1 |
| Methyl paraben | 0.1 |
| Fragrance | q.s. |

| (Aqueous phase) | mass% |
|---|---|
| Dipropylene glycol | 6.0 |
| 3-Benzyloxy-1,2-propanediol | 0.5 |
| 3-Phenoxy-1,2-propanediol | 0.5 |
| Sorbitol | 4.0 |
| PEG1500 | 5.0 |
| Methyl cellulose | 0.2 |
| Quince Seed | 0.1 |
| Purified water | balance |

### Example VII-6: Emulsion

| | mass% |
|---|---|
| 2,4,7,9-Tetramethyl-5-decyn-4,7-diol | 1.0 |
| Glycerol | 3.0 |
| 2,2-Diethyl-1,3-propanediol | 0.2 |
| 2,2,4-Trimethyl-1,3-propanediol | 0.5 |
| 2,2-Dimethylol pentane | 0.5 |
| 2,2-Dimethyl-1-phenyl-1,3-propanediol | 0.1 |

| | |
|---|---|
| Ethylhexanediol | 1.0 |
| Cetanol | 1.5 |
| Stearyl alcohol | 1.8 |
| Dimethyl polysiloxane (20 cs) | 1.5 |
| Squalane | 2.0 |
| Vaseline | 2.0 |
| Isopropyl myristate | 2.5 |
| Glyceryl monostearate | 1.8 |
| Polyoxyethylene (POE = 5) glyceryl monostearate | 1.8 |
| Polyoxyethylene (POE = 20) cetyl ether | 1.5 |
| Carboxyvinyl polymer | 0.25 |
| Potassium hydroxide | 0.05 |
| L-Arginine | 0.2 |
| Dipropylene glycol | 5.0 |
| 1,3-Butylene glycol | 3.0 |
| 1,2-Pentanediol | 0.1 |
| Trisodium edetate | 0.2 |
| 3-Benzyloxy-1,2-propanediol | 0.2 |
| 3-Phenoxy-1,2-propanediol | 0.1 |
| 3-Hexyne-2,5-diol | 0.1 |
| Phenoxyethanol | 0.1 |
| Methyl paraben | 0.01 |
| Ethyl paraben | 0.01 |
| Butyl paraben | 0.01 |
| Purified water | balance |

### Example VII-7: Emulsion

| | mass% |
|---|---|
| Stearic acid | 2.5 |
| Cetyl alcohol | 1.5 |
| Ethylhexanediol | 1.0 |
| 2,2,4-Trimethyl-1,3-propanediol | 0.5 |
| 2,2-Dimethylol pentane | 0.3 |
| 2,2-Diethyl-1,3-propanediol | 0.2 |
| 2,2-Dimethyl-1-phenyl-1,3-propanediol | 0.3 |
| 2,4,7,9-Tetramethyl-5-decyn-4,7-diol | 1.5 |
| 3-Hexyne-2,5-diol | 0.2 |
| Vaseline | 5.0 |
| Liquid paraffin | 10.0 |
| POE (10) monoleic acid ester | 2.0 |
| PEG1500 | 3.0 |
| Triethanolamine | 1.0 |
| 3-Phenoxy-1,2-propanediol | 2.0 |
| 3-Benzyloxy-1,2-propanediol | 1.0 |
| Phenoxyethanol | 0.2 |
| Methyl paraben | 0.1 |
| Ethyl paraben | 0.02 |
| Butyl paraben | 0.01 |
| Sodium bisulfite | 0.01 |
| Carboxyvinyl polymer | 0.05 |
| Fragrance | q.s. |
| Purified water | balance |

### Example VII-8: Emulsion

| | mass% |
|---|---|
| Liquid paraffin | 7.0 |
| Vaseline | 3.0 |
| Decamethylcyclopentansiloxane | 2.0 |
| Behenyl alcohol | 1.0 |
| Glycerol | 5.0 |
| Dipropylene glycol | 7.0 |
| Polyethylene glycol 1500 | 2.0 |
| Ethylhexanediol | 1.0 |
| 3-Hexyne-2,5-diol | 1.0 |
| 2,4,7,9-Tetramethyl-5-decyn-4,7-diol | 1.5 |
| 2,2-Dimethylol pentane | 0.2 |
| 2,2,4-Trimethyl-1,3-pentanediol | 0.1 |
| 2,2-Dimethyl-1-phenyl-1,3-propanediol | 0.2 |
| 2,2-Diethyl-1,3-propanediol | 0.2 |
| 3-Phenoxy-1,2-propanediol | 2.0 |
| 3-Benzyloxy-1,2-propanediol | 2.0 |
| Jojoba oil | 1.0 |
| Isostearic acid | 0.5 |

| | |
|---|---|
| Stearic acid | 0.5 |
| Behenic acid | 0.5 |
| Tetra 2-ethylhexanoic acid pentaerythritol | 3.0 |
| Cetyl 2-Ethylhexanoate | 3.0 |
| Glyceryl monostearate | 1.0 |
| Monostearic acid polyoxyethylene glycerol | 1.0 |
| Potassium hydroxide | 0.1 |
| Sodium hexametaphosphate | 0.05 |
| Stearyl glycyrrhizinate | 0.05 |
| L-Arginine | 0.1 |
| Royal gelly extract | 0.1 |
| Tocopherol acetate | 0.1 |
| Sodium acetylated hyaluronate | 0.1 |
| Trisodium edetate | 0.05 |
| 4-t-butyl-4'-methoxydibenzoyl methane | 0.1 |
| 2-Ethylhexyl p-methoxycinnamate | 0.1 |
| Carboxyvinyl polymer | 0.15 |
| Purified water | balance |

### Example VII-9: Gel

| | mass% |
|---|---|
| 95% Ethanol | 5.0 |
| Dipropylene glycol | 15.0 |
| 1,2-Octane diol | 2.0 |
| Ethylhexanediol | 1.0 |
| 2,2-Dimethylol pentane | 0.1 |
| 2,2-Dimethyl-1,3-propanediol | 0.1 |
| 2,2-Diethyl-1,3-propanediol | 0.1 |
| 2,2,4-Trimethyl-1,3-propanediol | 0.05 |
| POE. (50) Oleyl ether | 2.0 |
| Carboxyvinyl polymer | 1.0 |
| Sodium hydroxide | 0.15 |
| 3-Benzyloxy-1,2-propanediol | 0.05 |
| 3-Phenoxy-1,2-propanediol | 0.05 |
| 2,2-Dimethyl-1-phenyl-1,3-propanediol | 0.1 |

| | |
|---|---|
| 2,4,7,9-Tetramethyl-5-decyn-4,7-diol | 0.3 |
| 3-Hexyne-2,5-diol | 0.1 |
| Phenoxyethanol | 0.1 |
| Methyl paraben | 0.1 |
| Ethyl paraben | 0.03 |
| Butyl paraben | 0.01 |
| Fragrance | q.s. |
| Purified water | balance |

### Example VII-10: Beauty liquid

| | mass% |
|---|---|
| 95% Ethanol | 5.0 |
| POE (20) Octyldodecanol | 1.0 |
| Pantonyl ethyl ether | 0.1 |
| Ethylhexanediol | 0.1 |
| 2,2-Dimethylol pentane | 0.1 |
| 2,2-Dimethyl-1,3-propanediol | 0.1 |
| 2,2-Diethyl-1,3-propanediol | 0.1 |
| 2,2-Dimethyl-1-phenyl-1,3-propanediol | 0.1 |
| 2,2,4-Trimethyl-1,3-propanediol | 0.05 |
| 2,4,7,9-Tetramethyl-5-decyn-4,7-diol | 0.3 |
| Potassium hydroxide | 0.1 |
| Glycerol | 5.0 |
| Dipropylene glycol | 10.0 |
| Sodium bisulfite | 0.03 |
| Carboxyvinyl polymer | 0.2 |
| 3-Phenoxy-1,2-propanediol | 0.2 |
| 3-Benzyloxy-1,2-propanediol | 0.1 |
| Phenoxyethanol | 0.3 |
| Methyl paraben | 0.1 |
| Purified water | balance |

### Example VII-11: Pack

| (A phase) | mass% |
|---|---|
| Dipropylene glycol | 5.0 |
| POE (60) Hydrogenated castor oil | 5.0 |

| (B phase) | mass% |
|---|---|
| Olive oil | 5.0 |
| Ethylhexanediol | 0.1 |
| 2,2,4-Trimethyl-1,3-pentanediol | 0.1 |
| 2,2-Diethyl-1,3-propanediol | 0.2 |
| 2,2-Dimethylol pentane | 0.05 |
| Tocopherol acetate | 0.2 |
| Fragrance | 0.2 |

| (C phase) | mass% |
|---|---|
| Sodium bisulfite | 0.03 |
| Polyvinyl alcohol (Degree of saponification 90, Degree of polymerization 2000) | 13.0 |
| Ethanol | 5.0 |
| 2,4,7,9-Tetramethyl-5-decyn-4,7-diol | 0.3 |
| 2,2-Dimethyl-1-phenyl-1,3-propanediol | 0.2 |
| 3-Phenoxy-1,2-propanediol | 0.5 |
| 3-Benzyloxy-1,2-propanediol | 0.6 |
| 3-Hexyne-2,5-diol | 0.1 |
| Methyl paraben | 0.1 |
| Phenoxyethanol | 0.2 |
| Purified water | balance |

### Example VII-12: Cream

| | mass% |
|---|---|
| 2,4,7,9-Tetramethyl-5-decyn-4,7-diol | 1.0 |
| 2,2-Dimethyl-1-phenyl-1,3-propanediol | 0.5 |
| 3-Benzyloxy-1,2-propanediol | 0.2 |
| 3-Phenoxy-1,2-propanediol | 0.1 |
| 2,2-Diethyl-1,3-propanediol | 0.2 |
| 2,2,4-Trimethyl-1,3-pentanediol | 0.5 |
| 2,2-Dimethylol pentane | 0.5 |
| Ethylhexanediol | 1.0 |
| 3-Hexyne-2,5-diol | 0.1 |
| Stearyl alcohol | 3.5 |
| Stearic acid | 2.0 |
| Squalane | 10.5 |
| Isopropyl myristate | 7.5 |
| Polyoxyethylene (POE = 25) Cetyl alcohol ether | 3.0 |
| Glycerol monostearate | 2.0 |
| Tocopherol acetate | 0.2 |
| Monoammonium glycyrrhizinate | 0.05 |
| Glycerol | 3.0 |
| Dipropylene glycol | 5.0 |
| 1,2-Pentanediol | 0.1 |
| 1,3-Butylene glycol | 3.0 |
| Phenoxyethanol | 0.2 |
| Trisodium edetate | 0.01 |
| Methyl paraben | 0.1 |
| Ethyl paraben | 0.1 |
| Butyl paraben | 0.05 |
| Purified water | balance |

### Example VII-13: Cream

| | mass% |
|---|---|
| Stearic acid | 6.0 |
| Sorbitan monostearate | 2.0 |
| POE (20) Sorbitan monostearate | 1.5 |
| Ethylhexanediol | 2.0 |
| 2,2-Diethyl-1,3-propanediol | 1.0 |
| 2,2-Dimethylol pentane | 1.0 |
| 2,2,4-Trimethyl-1,3-pentanediol | 1.0 |
| Propylene glycol | 10.0 |
| 2,4,7,9-Tetramethyl-5-decyn-4,7-diol | 3.0 |
| 2,2-Dimethyl-1-phenyl-1,3-propanediol | 0.5 |
| 3-Benzyloxy-1,2-propanediol | 3.0 |
| 3-Phenoxy-1,2-propanediol | 1.0 |
| 3-Hexyne-2,5-diol | 0.1 |
| Glycerol trioctanoate | 10.0 |
| Squalane | 5.0 |
| Sodium bisulfite | 0.01 |
| Methyl paraben | 0.01 |
| Ethyl paraben | 0.01 |
| Butyl paraben | 0.01 |
| Phenoxyethanol | 0.1 |
| Fragrance | q.s. |
| Purified water | balance |

### Example VII-14: Cream

| | mass% |
|---|---|
| Liquid paraffin | 10.0 |
| Dimethyl polysiloxane | 2.0 |
| Glycerol | 10.0 |
| 1,3-Butylene glycol | 2.0 |
| Erythritol | 1.0 |
| Ethylhexanediol | 0.1 |
| 2,2-Dimethylol pentane | 1.0 |
| 2,2,4-Trimethyl-1,3-pentanediol | 0.5 |
| 2,2-Dimethyl-1-phenyl-1,3-propanediol | 0.2 |
| 2,2-Diethyl-1,3-propanediol | 0.1 |
| 2,4,7,9-Tetramethyl-5-decyn-4,7-diol | 2.0 |
| Polyethylene glycol 1500 | 5.0 |
| Squalane | 15.0 |
| Tetra 2-ethyl hexanoic acid pentaerythritol | 5.0 |
| Potassium hydroxide | 0.1 |
| Sodium hexametaphosphate | 0.05 |
| Tocopherol acetate | 0.05 |
| 3-Phenoxy-1,2-propanediol | 1.0 |
| 3-Benzyloxy-1,2-propanediol | 1.0 |
| 3-Hexyne-2,5-diol | 0.2 |
| Phenoxyethanol | 0.1 |
| Methyl paraben | 0.2 |
| Ethyl paraben | 0.1 |
| Butyl paraben | 0.05 |
| Hydroxypropyl methylcellulose | 0.3 |
| Polyvinyl alcohol | 0.1 |
| Carboxydivinyl polymer | 0.2 |
| Acrylic acid·methacrylic acid alkyl copolymer (Pemulene TR-2) | 0.1 |

### Example VII-15: Cream

| | mass% |
|---|---|
| Vaseline | 2.0 |
| Dimethylpolysiloxane (6 mPa.s) | 2.0 |
| Ethanol | 5.0 |
| Behenyl alcohol | 0.5 |
| Batyl alcohol | 0.2 |
| Glycerol | 7.0 |
| 1,3-Butylene glycol | 5.0 |
| Polyethylene glycol 20000 | 0.5 |
| Jojoba oil | 3.0 |
| Squalane | 2.0 |
| Phytosteryl hydroxystearate | 0.5 |
| Pentaerythitol Tetra 2-ethyl hexanoate | 1.0 |
| Polyoxyethylene (60) hydrogenated castor oil | 1.0 |
| Potassium hydroxide | 0.1 |
| Sodium pyrosulfite | 0.01 |
| Sodium hexametaphosphate | 0.05 |
| Stearyl glycyrrhetiate | 0.1 |
| Pantothenyl ethyl ether | 0.1 |
| Albutin | 7.0 |
| Tranexamic acid | 1.0 |
| Tocopherol acetate | 0.1 |
| Sodium hyaluronate | 0.05 |
| 3-Phenoxy-1,2-propanediol | 1.0 |
| 3-Benzyloxy-1,2-propanediol | 1.0 |
| Trisodium edetate | 0.05 |
| Ethylhexanediol | 1.0 |
| 2,4,7,9-Tetramethyl-5-decyn-4,7-diol | 0.6 |
| 2,2-Dimethylol pentane | 0.2 |
| 2,2-Dimethyl-1-phenyl-1,3-propanediol | 0.2 |
| 2,2,4-Trimethyl-1,3-pentanediol | 0.1 |
| 2,2-Diethyl-1,3-propanediol | 0.2 |
| 3-Hexyne-2,5-diol | 0.2 |
| 4-t-Butyl-4'-methoxydibenzoyl methane | 0.1 |
| Diparamethoxycinnamic acid mono-2-ethylhexane glyceryl | 0.1 |
| Phenoxyethanol | 0.1 |
| Methyl paraben | 0.05 |
| Ethyl paraben | 0.02 |
| Butyl paraben | 0.03 |
| Yellow iron oxide | q.s. |
| Xanthan gum | 0.1 |
| Carboxyvinyl polymer | 0.2 |
| Purified water | balance |

### Example VII-16: Cream

| | mass% |
|---|---|
| Decamethylcyclopenta siloxane | 30.0 |
| Polyoxyethylene·methylpolysiloxane copolymer (M.W. 6000) | 1.5 |
| Trimethylsiloxy silicic acid | 0.5 |
| Glycerol | 2.0 |
| Ethylhexanediol | 1.0 |
| 2,4,7,9-Tetramethyl-5-decyn-4,7-diol | 0.8 |
| 2,2-Dimethylol pentane | 0.2 |
| 2,2,4-Trimethyl-1,3-pentanediol | 0.1 |
| 2,2-Diethyl-1,3-propanediol | 0.3 |
| Dipropylene glycol | 5.0 |
| 3-Phenoxy-1,2-propanediol | 0.5 |
| 3-Benzyloxy-1,2-propanediol | 1.0 |
| 2,2-Dimethyl-1-phenyl-1,3-propanediol | 0.2 |
| 3-Hexyne-2,5-diol | 0.5 |
| Talc | 5.0 |
| Spherical anhydrous silicic acid | 0.5 |
| Dextrin palmitate-coaded titanium oxide fine powder (30 nm) | 7.0 |
| Spheric polyethylene powder | 2.0 |
| Poly(oxyethylene·oxypropylene)·methyl | |
| polysiloxane copolymer (M.W. 55000) | 1.0 |
| Phenoxyethanol | 0.2 |
| Methyl paraben | 0.1 |
| Ethyl paraben | 0.05 |
| Butyl paraben | 0.05 |
| Trisodium edetate | 0.02 |
| Dimethyl distearyl ammonium hectolite | 0.5 |
| Purified water | balance |

### Example VII-17: Cleansing

| | mass% |
|---|---|
| 2,4,7,9-Tetramethyl-5-decyn-4,7-diol | 0.5 |
| 2,2-Dimethyl-1-phenyl-1,3-propanediol | 0.3 |
| 3-Benzyloxy-1,2-propanediol | 0.2 |
| 3-Phenoxy-1,2-propanediol | 0.1 |
| 2,2-Diethyl-1,3-propanediol | 0.2 |
| 2,2,4-Trimethyl-1,3-pentanediol | 0.5 |
| 2,2-Dimethylol pentane | 0.5 |
| Ethylhexanediol | 0.1 |
| 1,2-Pentanediol | 0.1 |
| 3-Hexyne-2,5-diol | 0.1 |
| Stearic acid | 8.0 |
| Palmitic acid | 6.0 |
| Myristic acid | 6.0 |
| Lauric acid | 4.0 |
| Potassium hydroxide | 5.2 |
| Glyceryl monostearate | 2.0 |
| Propylene glycol | 1.0 |
| Bees wax | 1.5 |
| Polyethylene glycol 1500 | 5.0 |
| Glycerol | 10.0 |
| Methyl paraben | 0.01 |
| Phenoxyethanol | 0.1 |
| Purified water | balance |

### Example VII-18: Shampoo

| | mass% |
|---|---|
| Lauryl polyoxyethylene (3) sulfate ester sodium salt (30% aqueous solution) | 25.0 |
| Lauryl sulfate ester sodium salt (30% aqueous solution) | 8.0 |
| Coconut oil fatty acid diethanol amide | 4.0 |
| Isoprene glycol | 4.0 |
| Sodium benzoate | 0.5 |
| Dipropylene glycol | 1.0 |
| 1,3-Butylene glycol | 1.0 |
| 1,2-Pentanediol | 0.1 |
| Trisodium edetate | 0.01 |
| 2,2-Dimethyl-1-phenyl-1,3-propanediol | 0.1 |
| 3-Benzyloxy-1,2-propanediol | 0.1 |
| 3-Phenoxy-1,2-propanediol | 0.1 |
| 2,2-Diethyl-1,3-propanediol | 0.2 |
| 2,2,4-Trimethyl-1,3-pentanediol | 0.5 |
| 2,2-Dimethylol pentane | 0.5 |
| Ethylhexanediol | 0.1 |
| 3-Hexyne-2,5-diol | 0.1 |
| 2,4,7,9-Tetramethyl-5-decyn-4,7-diol | 0.1 |
| Dye | q.s. |
| Fragrance | q.s. |
| Purified water | balance |

### Example VII-19: Rinse

| | mass% |
|---|---|
| Silicone oil | 3.0 |
| Liquid paraffin | 1.0 |
| Cetyl alcohol | 1.5 |
| Stearyl alcohol | 1.0 |
| Stearyl trimethylammonium chloride | 0.7 |
| Ethylhexanediol | 0.5 |
| 2,2,4-Trimethyl-1,3-pentanediol | 0.3 |
| 2,2-Dimethyl-1-phenyl-1,3-propanediol | 0.2 |
| 2,2-Diethyl-1,3-propanediol | 0.1 |
| 2,2-Dimethylol pentane | 0.07 |
| 2,4,7,9-Tetramethyl-5-decyn-4,7-diol | 0.4 |
| 3-Phenoxy-1,2-propanediol | 1.0 |
| 3-Benzyloxy-1,2-propanediol | 1.0 |
| 3-Hexyne-2,5-diol | 0.7 |
| Glycerol | 3.0 |
| Dye | q.s. |
| Fragrance | q.s. |
| Purified water | balance |

### Example II-20: Gelly pack

| | mass% |
|---|---|
| 3-Benzyloxy-1,2-propanediol | 0.05 |
| 3-Phenoxy-1,2-propanediol | 0.1 |
| 2,4,7,9-Tetramethyl-5-decyn-4,7-diol | 0.1 |
| Polyoxyethylene oleyl alcohol ether | 0.5 |
| 2,2-Diethyl-1,3-propanediol | 0.1 |
| Ethylhexanediol | 0.1 |
| 2,2-Dimethyl-1-phenyl-1,3-propanediol | 0.2 |
| 2,2,4-Trimethyl-1,3-propanediol | 0.1 |
| 2,2-Dimethylol pentane | 0.1 |
| 3-Hexyne-2,5-diol | 0.1 |
| Monoammonium glycyrrhizinate | 0.05 |
| Carboxymethyl cellulose | 5.0 |
| Ethanol | 12.0 |
| Polyvinyl alcohol | 12.0 |
| 1,3-Butylene glycol | 5.0 |
| 1,2-Pentanediol | 0.3 |
| Trisodium edetate | 0.01 |
| Purified water | balance |

### Example VII-21: Solid powdery foundation

| | mass% |
|---|---|
| Talc | 15.0 |
| Cericite | 10.0 |
| Spherical nylon powder | 10.0 |
| Porous anhydrous silicic acid powder | |
| | 15.0 |
| Boron nitride | 5.0 |
| Titanium dioxide | 5.0 |
| Iron oxide | 3.0 |
| Zinc stearate | 5.0 |
| Liquid paraffin | balance |
| Glycerol triisooctanoate | 15.0 |
| Sorbitan sesquioleate | 1.5 |
| 3-Phenoxy-1,2-propanediol | 0.5 |
| 3-Benzyloxy-1,2-propanediol | 0.3 |
| 2,4,7,9-Tetramethyl-5-decyn-4,7-diol | 0.2 |
| Ethylhexanediol | 0.2 |
| 2,2,4-Trimethyl-1,3-pentanediol | 0.1 |
| 2,2-Dimethyl-1-phenyl-1,3-propanediol | 0.2 |
| 2,2-Diethyl-1,3-propanediol | 0.2 |
| 2,2-Dimethylol pentane | 0.05 |
| 3-Hexyne-2,5-diol | 0.1 |
| Methyl paraben | 0.2 |
| Ethyl paraben | 0.1 |
| Fragrance | q.s. |

### Example VII-22: Oil-in-water type emulsified foundation

| | mass% |
|---|---|
| Spherical nylon | 10.0 |
| Porous anhydrous silicic acid powder | |
| | 8.0 |
| Mica titanium | 2.0 |
| Silicone-treated cericite | 2.0 |
| Silicone-treated mica | 12.0 |
| Silicone-treated titanium dioxide | 5.0 |
| Silicone-treated iron oxide | 2.0 |
| Purified water | balance |
| 3-Phenoxy-1,2-propanediol | 3.0 |
| 3-Benzyloxy-1,2-propanediol | 3.0 |
| Ethylhexanediol | 1.0 |
| 2,2-Dimethylol pentane | 1.0 |
| 2,2-Diethyl-1,3-propanediol | 1.0 |
| 2,2-Dimethyl-1,3-propanediol | 0.2 |
| 2,2,4-Trimethyl-1,3-pentanediol | 1.0 |
| 2,4,7,9-Tetramethyl-5-decyn-4,7-diol | 0.8 |
| 2,2-Dimethyl-1-phenyl-1,3-propanediol | 0.2 |
| 3-Hexyne-2,5-diol | 0.1 |
| Decamethylcyclopentane siloxane | 18.0 |
| Dimethyl polysiloxane | 5.0 |
| Squalane | 1.0 |
| Polyoxyethylene modified dimethylpolysiloxane | 2.0 |
| Phenoxyethanol | 0.1 |
| Methyl paraben | 0.1 |
| Fragrance | q.s. |

### Example VII-23: Face powder

| | mass% |
|---|---|
| Talc | balance |
| Cericite | 10.0 |
| Spherical nylon powder | 10.0 |
| Boron nitride | 5.0 |
| Iron oxide | 3.0 |
| Magnesium carbonate | 5.0 |
| Squalane | 3.0 |
| Glycerol triisooctanoate | 2.0 |
| Sorbitan sesquioleate | 2.0 |
| Ethylhexanediol | 0.5 |
| 3-Phenoxy-1,2-propanediol | 1.0 |
| 3-Benzyloxy-1,2-propanediol | 2.0 |
| Ethylhexanediol | 1.0 |
| 2,2-Dimethylol pentane | 1.0 |
| 2,2-Diethyl-1,3-propanediol | 1.0 |
| 2,2-Dimethyl-1,3-propanediol | 0.2 |
| 2,2,4-Trimethyl-1,3-pentanediol | 1.0 |
| 2,2-Dimethyl-1-phenyl-1,3-propanediol | 0.1 |
| 2,4,7,9-Tetramethyl-5-decyn-4,7-diol | 0.4 |
| 3-Hexyne-2,5-diol | 0.1 |
| Methyl paraben | 0.3 |
| Fragrance | q.s. |

### Example VII-24: Eyeliner

| | mass% |
|---|---|
| 2,4,7,9-Tetramethyl-5-decyn-4,7-diol | 1.0 |
| 2,2-Dimethyl-1-phenyl-1,3-propanediol | 0.3 |
| 3-Benzyloxy-1,2-propanediol | 0.2 |
| 3-Phenoxy-1,2-propanediol | 0.5 |
| Iron oxide (black) | 14.0 |
| Isopropyl myristate | 1.5 |
| Polyoxyethylene sorbitan monooleic ester | 1.0 |
| Vinylacetate resin emulsion | 45.0 |
| Monoammonium glycyrrhizinate | 0.05 |
| Carboxyvinyl polymer | 1.5 |
| Acetyltributyl citrate | 1.0 |
| Dipropylene glycol | 4.0 |
| Isopropyl alcohol | 1.0 |
| Ethylhexane diol | 1.0 |
| 2,2-Diethyl-1,3-pentanediol | 1.0 |
| 2,2-Dimethyl-1,3-pentanediol | 1.0 |
| 2,2,4-Trimethyl-1,3-pentanediol | 0.5 |
| 2,2-Dimethylol pentane | 0.5 |
| 1,2-Pentanediol | 3.0 |
| 3-Hexyne-2,5-diol | 0.1 |
| Phenoxyethanol | 0.1 |
| Methyl paraben | 0.02 |
| Trisodium edetate | 0.01 |
| Purified water | balance |

### Example VII-25: Eye shadow

| | mass% |
|---|---|
| Talc | balance |
| Mica | 15.0 |
| Spherical nylon powder | 10.0 |
| Boron nitride | 5.0 |
| Iron oxide | 3.0 |
| Titanium oxide-coated mica | 5.0 |
| Squalane | 3.0 |
| Glycerol triisooctanoate | 2.0 |
| Sorbitan sesquioleate | 2.0 |
| Ethylhexanediol | 1.0 |
| 2,2-Dimethylol pentane | 0.3 |
| 2,2-Diethyl-1,3-pentanediol | 0.1 |
| 2,2-Dimethyl-1,3-pentanediol | 0.1 |
| 2,2,4-Trimethyl-1,3-propanediol | 0.1 |
| 3-Phenoxy-1,2-propanediol | 0.5 |
| 3-Benzyloxy-1,2-propanediol | 0.5 |
| 2,4,7,9-Tetramethyl-5-decyn-4,7-diol | 0.6 |
| 2,2-Dimethyl-1-phenyl-1,3-propanediol | 0.2 |
| 3-Hexyne-2,5-diol | 0.3 |
| Methyl paraben | 0.1 |
| Fragrance | q.s. |

### Example VII-26: Lipstick

| | mass% |
|---|---|
| Carnauba wax | 0.5 |
| Candelilla wax | 5.0 |
| Ceresine | 10.0 |
| Squalane | balance |
| Glycerol triisostearate | 10.0 |
| Glycerol diisostearate | 20.0 |
| 3-Phenoxy-1,2-propanediol | 0.2 |
| 3-Benzyloxy-1,2-propanediol | 0.1 |
| 2,4,7,9-Tetramethyl-5-decyn-4,7-diol | 0.5 |
| Ethylhexanediol | 1.0 |
| 2,2-Dimethylol pentane | 0.1 |
| 2,2-Diethyl-1,3-pentanediol | 0.1 |
| 2,2-Dimethyl-1,3-pentanediol | 0.1 |
| 2,2,4-Trimethyl-1,3-propanediol | 0.1 |
| 3-Hexyne-2,5-diol | 0.7 |
| 2,2-Dimethyl-1-phenyl-1,3-propanediol | 0.1 |
| Cholesteryl Macademia nut oil fatty acid | 4.0 |
| Synthetic sodium-magnesium silicate | 0.5 |
| Hydrophobic silica | 0.5 |
| Purified water | 2.0 |
| Coloring agent | q.s. |
| Fragrance | q.s. |

### Example VII-27: Hair foam

| (Stock solution formulation) | mass% |
|---|---|
| Acrylic resin alkanol amine solution (50%) | 8.0 |
| Polyoxyethylene hydrogenated castor oil | 1.0 |
| Liquid paraffin | 5.0 |
| Glycerol | 3.0 |
| Fragrance | q.s. |
| 3-Phenoxy-1,2-propanediol | 0.01 |
| 3-Benzyloxy-1,2-propanediol | 0.01 |
| 2,4,7,9-Tetramethyl-5-decyn-4,7-diol | 0.3 |
| Ethylhexanediol | 0.2 |
| 2,2-Dimethylol pentane | 0.1 |
| 2,2-Diethyl-1,3-pentanediol | 0.1 |
| 2,2-Dimethyl-1,3-pentanediol | 0.1 |
| 2,2,4-Trimethyl-1,3-propanediol | 0.1 |
| 3-Hexyne-2,5-diol | 0.3 |
| 2,2-Dimethyl-1-phenyl-1,3-propanediol | 0.1 |
| Ethanol | 5.0 |
| Purified water | balance |

| (Filling formulation) | mass% |
|---|---|
| Stock solution | 90.0 |
| Liquefied petroleum gas | 10.0 |

### Example VII-28: Hair tonic

| | mass% |
|---|---|
| Hydrogenated castor oil ethyleneoxide (40 mol) addition product | 2.0 |
| Ethanol | 60.0 |
| Fragrance | q.s. |
| 2,4,7,9-Tetramethyl-5-decyn-4,7-diol | 0.01 |
| Purified water | balance |

### Example VII-29: Bath agent

| | mass% |
|---|---|
| Sodium bicarbonate | 64.5 |
| Anhydrous sodium sulfate | 35.0 |
| 2,4,7,9-Tetramethyl-5-decyn-4,7-diol | 0.5 |

### Example VII-30: Chinese noodle

| | mass% |
|---|---|
| Wheat flour | 98.0 |
| Table salt | 1.0 |
| Sweetner | 0.5 |
| 2,4,7,9-Tetramethyl-5-decyn-4,7-diol | 0.5 |

### Example VII-31: Noodle soup

| | mass% |
|---|---|
| Soysauce | 80.7 |
| Vinegar | 1.0 |
| Glucose | 15.0 |
| Sodium glutamate | 2.0 |
| Sugar | 1.0 |
| 2,4,7,9-Tetramethyl-5-decyn-4,7-diol | 0.3 |

### Example VII-32: Japanese noodle ("Soba")

| | mass% |
|---|---|
| Soba flour | 96.0 |
| Table salt | 0.9 |
| Water | 3.0 |
| 2,4,7,9-Tetramethyl-5-decyn-4,7-diol | 0.1 |

### Example VII-33: Bread

| | mass% |
|---|---|
| Wheat flour | 90.0 |
| Table salt | 1.2 |
| Sugar | 2.0 |
| Water | 6.5 |
| 2,4,7,9-Tetramethyl-5-decyn-4,7-diol | 0.3 |

### Example VII-34: Ham

| | mass% |
|---|---|
| Minced meat | 95.0 |
| Chicken egg | 4.0 |
| Table salt | 0.5 |
| Spice | 0.4 |
| 2,4,7,9-Tetramethyl-5-decyn-4,7-diol | 0.1 |

### Example VII-35: Fruitjuice beverage

| | mass% |
|---|---|
| Glucose liquid sugar | 13.0 |
| Orange fruit juice | 85.0 |
| Fragrance | 1.0 |
| 2,4,7,9-Tetramethyl-5-decyn-4,7-diol | 0.1 |

All the above Examples exhibited good preservability.

### INDUSTRIAL APPLICABILITY

The antimicrobial agent of the present invention has the action of suppressing proliferation of various microorganisms and further is safe for human or animal use. Therefore, if used as an antimicrobial agent, it is possible to prevent spoilage and contamination of various types of preparations and products, and therefore, the invention can be used for various types of external skin treatment compositions in the field of pharmaceuticals, quasi-drugs and cosmetics (including various preparations used for human and other animal use), medical use cleansers meant for disinfecting and cleaning medical equipment or diseased parts, household cleansers for sterilizing and washing eating utensils, cleansers for the food industry, antimicrobial treatment of textile products (sheets, apparel, etc.), food packaging film, plastic, wood, daily necessities, etc., various types of oral medications, sanitary napkins, wet tissue or paper towels, sterilizing cloth and other nonwoven fabric, or oral compositions (gum, candies, etc.) or Japanese *kamaboko, chikuwa*, or other fishpaste products, sausages, ham, and other animal products, western confectioneries, Japanese confectioneries, raw noodles, boiled noodles, Chinese noodles, Japanese *udon* noodles, Japanese *soba* noodles, spaghetti, and other noodles, soysauce, sauces, gravies, and other seasoning, food dishes, juices, soups, and other general foods and beverages can be mentioned.

The antimicrobial agent of the present invention may be utilized for various types of external skin treatment compositions in the field of pharmaceuticals, quasi-drugs, and cosmetics (including various preparations used for human and other animal use), specifically application to toilet water, milk lotions, creams (including ointments), sun screens, foundations, oils, packs, soaps (including medicated soaps), body soaps, lipstick, manicure preparations, eye cosmetics, perfumes, facial cleansers, oral hygiene products (toothpastes, mouthwashes, etc.), deodorants (underarm odor, foot odor, etc.), bath agents, shampoos, rinses, hair tonics, hair sprays, hair dyes, etc. may be mentioned. Further, application to medical use cleansers meant for disinfecting and cleaning medical equipment or diseased parts, household cleansers for sterilizing and washing eating utensils, cleansers for the food industry, etc. may be mentioned. Further, use for textile products (sheets, apparel, etc.), antimicrobial treatment of food packaging film, plastic, wood, daily necessities, etc., various types of oral medications, sanitary napkins, wet tissue or paper towels, sterilizing cloth and other nonwoven fabric, or oral compositions (gum, candies, etc.) or Japanese *kamaboko, chikuwa,* or other fishpaste products, sausages, ham, and other animal products, western confectioneries, Japanese confectioneries, raw noodles, boiled noodles, Chinese noodles, Japanese *udon* noodles, Japanese *soba* noodles, spaghetti, and other noodles, soysauce, sauces, gravies, and other seasoning, food dishes, juices, soups, and other general foods and beverages can be mentioned.

## Claims

1. An external skin treatment composition comprising, based upon total weight of the composition, 0.001% by mass or more of a diol compound having the formula (I), (II), (III) or (IV) or the derivative thereof and a balance of a carrier: 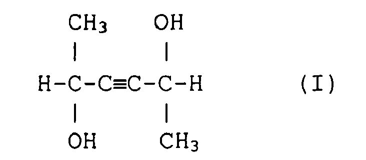 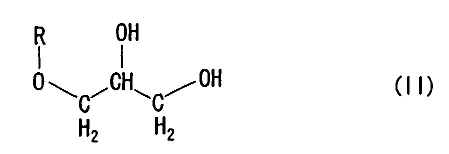 wherein R indicates a benzyl group or phenyl group.

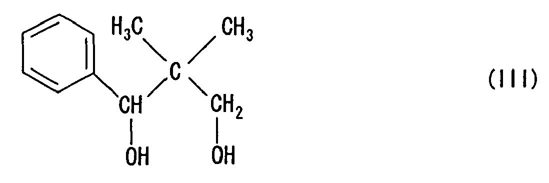

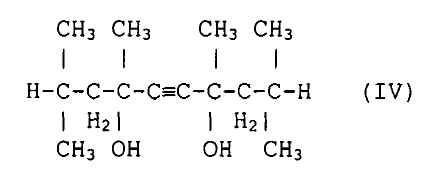

2. An external skin treatment composition as claimed in claim 1, wherein the diol compound is 3-hexine-2,5-diol having the formula (I).

3. An external skin treatment composition as claimed in claim 1, wherein the diol derivative is a glycerin derivative having the formula (II).

4. An external skin treatment composition as claimed in claim 1, wherein the diol compound is 2,2-dimethyl-1-phenyl-1,3-propanediol having the formula (III).

5. An external skin preparation composition as claimed in claim 1, wherein the diol compound is 2,4,7,9-tetramethyl-5-decine-4,7-diol having the formula (IV).

6. An external skin preparation composition as claimed in claim 1, wherein the content of the diol compound or its derivatives is 0.001 to 10% by mass, based upon the total weight of the composition.

7. An antimicrobial agent comprising a diol compound having the formula (I), (II), (III) or (IV) or the derivative thereof. 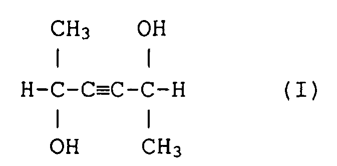 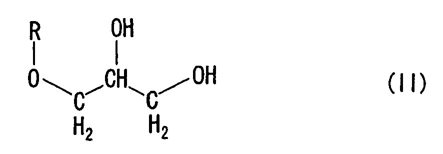 wherein R indicates a benzyl group or a phenyl group.

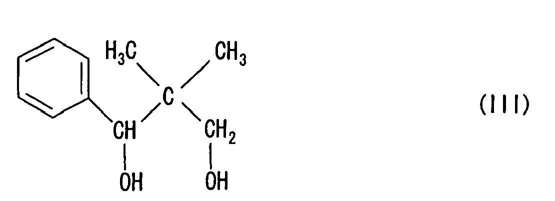

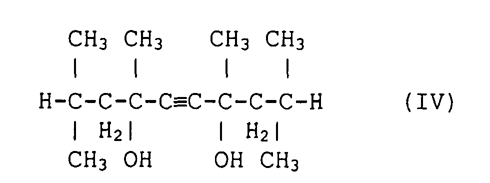

8. An antimicrobial agent as claimed in claim 7, wherein the diol compound is 3-hexine-2,5-diol having the formula (I).

9. An antimicrobial agent as claimed in claim 7, wherein the diol derivative is a glycerin derivative having the formula (II).

10. An antimicrobial agent as claimed in claim 7, wherein the diol compound is 2,2-dimethyl-1-phenyl-1,3-propanediol having the formula (III).

11. An antimicrobial agent as claimed in claim 7, wherein the diol compound is 2,4,7,9-tetramethyl-5-decine-4,7-diol having the formula (IV).
